# EUROPEAN PATENT SPECIFICATION

(11) **EP 3 083 951 B1**
(45) Date of publication and mention of the grant of the patent: **06.10.2021**
(21) Application number: 14830519.6
(22) Date of filing: 12.12.2014
(51) Int. Cl.: C12N 9/10, C12P 19/04

(54) **PROCESS FOR PRODUCING A BETA-GLUCAN POLYMER AND GENETICALLY MODIFIED MICROORGANISMS USEFUL IN THIS PROCESS**
VERFAHREN ZUR HERSTELLUNG EINES BETA-GLUCAN-POLYMERS UND GENETISCH MODIFIZIERTE MIKROORGANISMEN FÜR DIESES VERFAHREN
PROCÉDÉ DE PRODUCTION D'UN POLYMÈRE DE BÊTA-GLUCANE ET MICRO-ORGANISMES GÉNÉTIQUEMENT MODIFIÉS UTILES DANS CE PROCÉDÉ

(30) Priority: 19.12.2013 EP 13198610
(43) Date of publication of application: 26.10.2016
(73) Proprietor: BASF SE, 67056 Ludwigshafen (DE)
(72) Inventor: HLUBEK, Andrea, 67161 Gönnheim (DE); BROCKMANN, Beata, Morristown, NJ 07960 (US); HEROLD, Andrea, 69469 Weinheim (DE); GRANSTRÖM, Mari, FI-00120 Helsinki (FI); FLECK, Christian, 69207 Sandhausen (DE)
(74) Representative: BASF IP Association
(86) International application number: PCT/EP2014/077479
(87) International publication number: WO 2015/091231

(56) References cited:
- KR-B1- 100 892 355
- JOCHEN SCHMID ET AL: "Scleroglucan: biosynthesis, production and application of a versatile hydrocolloid", APPLIED MICROBIOLOGY AND BIOTECHNOLOGY, SPRINGER, BERLIN, DE, vol. 91, no. 4, 6 July 2011 (2011-07-06), pages 937-947, XP019931861, ISSN: 1432-0614, DOI: 10.1007/S00253-011-3438-5
- KUMARI ET AL: "Production of schizophyllan using Schizophyllum commune NRCM", BIORESOURCE TECHNOLOGY, ELSEVIER BV, GB, vol. 99, no. 5, 15 December 2007 (2007-12-15), pages 1036-1043, XP022391646, ISSN: 0960-8524, DOI: 10.1016/J.BIORTECH.2007.02.029
- SZANISZLO ET AL: "Stimulation of beta(1----3)glucan synthetase of various fungi by nucleoside triphosphates: generalized regulatory mechanism for cell wall biosynthesis.", JOURNAL OF BACTERIOLOGY, vol. 161, no. 3, 1 March 1985 (1985-03-01) , pages 1188-1194, XP55078422, ISSN: 0021-9193
- SCHUREN F H J ET AL: "HIGHLY-EFFICIENT TRANSFORMATION OF THE HOMOBASIDIOMYCETE SCHIZOPHYLLUM COMMUNE TO PHLEOMYCIN RESISTANCE", CURRENT GENETICS, NEW YORK, NY, US, vol. 26, no. 2, 1 January 1994 (1994-01-01), pages 179-183, XP008051258, ISSN: 0172-8083, DOI: 10.1007/BF00313808
- None

### SEQUENCE LISTING

<110> Wintershall Holding GmbH
<120> Process for producing a beta-glucan polymer and genetically modified microorganisms useful in this process.
<130> PF 75227
<150> EP 13198610.1
   <151> 2013-12-19
<160> 51
<170> PatentIn version 3.5
<210> 1
   <211> 2133
   <212> DNA
   <213> Schizophyllum commune
<400> 1
   atgcggttgt gctgggctaa cgggctgtgc tgccaggtca tcggcagtgt cgtcgccctc 60
   gtcgtgatca tcgcactggc cgtcggtctt ggtgtcggtc tctcgcgcaa caacaagtcc 120
   tcttcctctg gcagcaaggg cggagcgacc aaccccgagt cgtcagactc ggtcggcagc 180
   gacccgtcca acttcgtcaa ggacgaccgc ttgcaccagt ccttctacgg cattgcttac 240
   acgccggagg gctcgatcat gccgaactgt ggcgctacgc ttggtgagcg cgacgtgtca 300
   gacaggctac ttgcaggcta atgtggcgcg cagagaatgt catcaaggat atccaggtgg 360
   gcagagaaag ccgtcgtttt gagattcccg attgacacgt tgcgcagctt atgtcgcagc 420
   taacgaagcg tgtgcgcttg tatggcgcgg actgcaacca gtcggcgctt gtggtacgtc 480
   ctcgagaccg agttccttgc agcctgatct aagcattgaa ttgcttctca cagctggagg 540
   ccatccagca gaccaaggtc gagtaagtgc ttctgtgccc gctgaaacct aatgagttga 600
   ctcgtggcct gcagtatgca agtgtacctc gccaactacg acgttcctgg cgactcggac 660
   gcgtatacgc gccagcgtga taccctcaag gaggcgatcc agacctacgg cacggaccat 720
   attgcgggca tcaccgtggg caacgagtac atcctcaagt aagcgcgcgt cctgctcgta 780
   ggagggacta caagctgatg agtggtgcag ctacctcaac gacaatggcg gcacggaccc 840
   caatgactct gtcggcaacg cgggcgccaa gcttctcatc gccgacatcg atgacaccaa 900
   gcagatgctg agcgaccttg gcgtcgacct cccggttggc aactctgatg cgggcagcta 960
   cttcaacgac caggtccttg agagcgttga ctacggcgtg cgtattccta ttggtgacgc 1020
   ttagtgggca tcgagctcat tttttccaga tgtcgaacgt tcacgcttgg ttcgcgaaca 1080
   ccacggcgga tgacgcagca tcatgggtgt tctcgttctt cgaggaaacc aacgtcgagc 1140
   ccgcgtcgca gctgagcaac aagccgcaga tgtacattgc ggagactggc tggccgacgg 1200
   tgaggaattt ggcacggtta tactatggac acagattgaa tatacccaca gaaatcgtct 1260
   gacgagggca atgccaacaa cggtgctagc gatgcatccg tggccaacct tcaggtgggc     1320
   ctagaagtaa tgagcctttc gacattctct ctgacctagc ttccagatct tccttgacga     1380
   ctttgtctgc caagcgaacg ccaacaacac tggctacttc tttttcgagg tcagtgtaga     1440
   gtcttactga acacttaatg gtacacacat tgctgacgga gcatatcagt tcttcgatga     1500
   ggagtggaag gatgagatgt acggcggcgt tgagggctgg tggggtctct tcacgaagga     1560
   gtacgttgct tcagtcctgc acgatgcaca cgatgctaaa gctttctagc cgccaactca     1620
   aggacctgac gatccccgac tgcgcggcgc cctaagcgtg ccaacctgct cgttatatat     1680
   tggtctgccg ctccgttcgt cccccctccc ccactttccc tgcctgcccc atcccacccc     1740
   gctttatgtg acagtcatcg caccgctatt ccaccgctaa gcagactttc cgtacttgca     1800
   cacgattaat ttttcgtcct gtctatacgg cctatccgca tgctatcctc ccgcccacct     1860
   taatgactac gtacgcgcgg ttgctggtga cgtgtgtggt tgctttctgc tcctgcagct     1920
   gcatgactgt acttgctgct gcactcgctc gcctgctggc caccgcgttt acgtgctcgc     1980
   ccgctactat acgcgctcga tcacttgcta ttccaacgga catgcttcga gagttacagg     2040
   ttcctactct cagctatcag actcgctttg tacacggact ttcgggcatt gtatgggtac     2100
   cacggtatac atcgaatggg aaccattgaa tgg                                  2133
<210> 2
   <211> 374
   <212> PRT
   <213> Schizophyllum commune
<400> 2
   Met Arg Leu Cys Trp Ala Asn Gly Leu Cys Cys Gln Val Ile Gly Ser
   1               5                   10                  15
   Val Val Ala Leu Val Val Ile Ile Ala Leu Ala Val Gly Leu Gly Val
               20                  25                  30
   Gly Leu Ser Arg Asn Asn Lys Ser Ser Ser Ser Gly Ser Lys Gly Gly
           35                  40                  45
   Ala Thr Asn Pro Glu Ser Ser Asp Ser Val Gly Ser Asp Pro Ser Asn
       50                  55                  60
   Phe Val Lys Asp Asp Arg Leu His Gln Ser Phe Tyr Gly Ile Ala Tyr
   65                  70                  75                  80
   Thr Pro Glu Gly Ser Ile Met Pro Asn Cys Gly Ala Thr Leu Glu Asn
                   85                  90                  95
   Val Ile Lys Asp Ile Gln Leu Met Ser Gln Leu Thr Lys Arg Val Arg
   100 105 110
   Leu Tyr Gly Ala Asp Cys Asn Gln Ser Ala Leu Val Leu Glu Ala Ile
   115 120 125
   Gln Gln Thr Lys Val Asp Met Gln Val Tyr Leu Ala Asn Tyr Asp Val
   130 135 140
   Pro Gly Asp Ser Asp Ala Tyr Thr Arg Gln Arg Asp Thr Leu Lys Glu
   145 150 155 160
   Ala Ile Gln Thr Tyr Gly Thr Asp His Ile Ala Gly Ile Thr Val Gly
   165 170 175
   Asn Glu Tyr Ile Leu Asn Tyr Leu Asn Asp Asn Gly Gly Thr Asp Pro
   180 185 190
   Asn Asp Ser Val Gly Asn Ala Gly Ala Lys Leu Leu Ile Ala Asp Ile
   195 200 205
   Asp Asp Thr Lys Gln Met Leu Ser Asp Leu Gly Val Asp Leu Pro Val
   210 215 220
   Gly Asn Ser Asp Ala Gly Ser Tyr Phe Asn Asp Gln Val Leu Glu Ser
   225 230 235 240
   Val Asp Tyr Gly Met Ser Asn Val His Ala Trp Phe Ala Asn Thr Thr
   245 250 255
   Ala Asp Asp Ala Ala Ser Trp Val Phe Ser Phe Phe Glu Glu Thr Asn
   260 265 270
   Val Glu Pro Ala Ser Gln Leu Ser Asn Lys Pro Gln Met Tyr Ile Ala
   275 280 285
   Glu Thr Gly Trp Pro Thr Lys Ser Ser Asp Glu Gly Asn Ala Asn Asn
   290 295 300
   Gly Ala Ser Asp Ala Ser Val Ala Asn Leu Gln Ile Phe Leu Asp Asp
   305 310 315 320
   Phe Val Cys Gln Ala Asn Ala Asn Asn Thr Gly Tyr Phe Phe Phe Glu
   325 330 335
   Phe Phe Asp Glu Glu Trp Lys Asp Glu Met Tyr Gly Gly Val Glu Gly
   340 345 350
   Trp Trp Gly Leu Phe Thr Lys Asp Arg Gln Leu Lys Asp Leu Thr Ile
   355 360 365
   Pro Asp Cys Ala Ala Pro
   370
<210> 3
   <211> 20
   <212> DNA
   <213> artificial (primer Sto378)
<400> 3
   aaaatcgtca ggcgaacggc 20
<210> 4
   <211> 43
   <212> DNA
   <213> artificial (primer Sto380)
<400> 4
   ccttgccacc ggacttggat ccattcaatg gttcccattc gat 43
<210> 5
   <211> 434
   <212> DNA
   <213> Schizophyllum commune
<400> 5
   caagtccggt ggcaaggtca ccaagtccgc cgagaaggcc gccaagaaga agtaaatgta 60
   gatgtacata tgtattttct cattccgttt ccttcctctt gttgttgttt cactggtcct 120
   ctcgtgctcg ctcgcatcgc atacagccat tgttgtcacc actataactt cacgcattct 180
   gtatttcatg ccaggcgacg gggtcttgtc ctgccaggcc tgtcgcttgt tgtaacgcta 240
   atgaaaagtc acgagtagtg gacgaacgac gatgtatttc tatgtgctgt agcgattatc 300
   catttcgagt tcgccatcga gctctcttca aacctaggtg cgacgttgtg aatgcagtag 360
   caagtgcaga gtattgcaga ctcgtccatt gatgataact tcaagctacg tcagagccag 420
   atgctactga accc 434
<210> 6
   <211> 43
   <212> DNA
   <213> artificial (primer Sto379)
<400> 6
   atcgaatggg aaccattgaa tggatccaag tccggtggca agg 43
<210> 7
   <211> 29
   <212> DNA
   <213> artificial (primer Sto229)
<400> 7
   ccgctcgagg ggttcagtag catctggct 29
<210> 8
   <211> 29
   <212> DNA
   <213> artificial (primer Sto229)
<400> 8
   ccgctcgagg ggttcagtag catctggct 29
<210> 9
   <211> 29
   <212> DNA
   <213> artificial (primer Sto378)
<400> 9
   ccggaattca tgcggttgtg ctgggctaa 29
<210> 10
   <211> 2133
   <212> DNA
   <213> Schizophyllum commune
<400> 10
   atgcggttgt gctgggctaa cgggctgtgc tgccaggtca tcggcagtgt cgtcgccctc      60
   gtcgtgatca tcgcactggc cgtcggtctt ggtgtcggtc tctcgcgcaa caacaagtcc     120
   tcttcctctg gcagcaaggg cggagcgacc aaccccgagt cgtcagactc ggtcggcagc     180
   gacccgtcca acttcgtcaa ggacgaccgc ttgcaccagt ccttctacgg cattgcttac     240
   acgccggagg gctcgatcat gccgaactgt ggcgctacgc ttggtgagcg cgacgtgtca     300
   gacaggctac ttgcaggcta atgtggcgcg cagagaatgt catcaaggat atccaggtgg     360
   gcagagaaag ccgtcgtttt gagattcccg attgacacgt tgcgcagctt atgtcgcagc     420
   taacgaagcg tgtgcgcttg tatggcgcgg actgcaacca gtcggcgctt gtggtacgtc     480
   ctcgagaccg agttccttgc agcctgatct aagcattgaa ttgcttctca cagctggagg     540
   ccatccagca gaccaaggtc gagtaagtgc ttctgtgccc gctgaaacct aatgagttga     600
   ctcgtggcct gcagtatgca agtgtacctc gccaactacg acgttcctgg cgactcggac     660
   gcgtatacgc gccagcgtga taccctcaag gaggcgatcc agacctacgg cacggaccat     720
   attgcgggca tcaccgtggg caacgagtac atcctcaagt aagcgcgcgt cctgctcgta     780
   ggagggacta caagctgatg agtggtgcag ctacctcaac gacaatggcg gcacggaccc     840
   caatgactct gtcggcaacg cgggcgccaa gcttctcatc gccgacatcg atgacaccaa     900
   gcagatgctg agcgaccttg gcgtcgacct cccggttggc aactctgatg cgggcagcta     960
   cttcaacgac caggtccttg agagcgttga ctacggcgtg cgtattccta ttggtgacgc    1020
   ttagtgggca tcgagctcat tttttccaga tgtcgaacgt tcacgcttgg ttcgcgaaca 1080
   ccacggcgga tgacgcagca tcatgggtgt tctcgttctt cgaggaaacc aacgtcgagc 1140
   ccgcgtcgca gctgagcaac aagccgcaga tgtacattgc ggagactggc tggccgacgg 1200
   tgaggaattt ggcacggtta tactatggac acagattgaa tatacccaca gaaatcgtct 1260
   gacgagggca atgccaacaa cggtgctagc gatgcatccg tggccaacct tcaggtgggc 1320
   ctagaagtaa tgagcctttc gacattctct ctgacctagc ttccagatct tccttgacga 1380
   ctttgtctgc caagcgaacg ccaacaacac tggctacttc tttttcgagg tcagtgtaga 1440
   gtcttactga acacttaatg gtacacacat tgctgacgga gcatatcagt tcttcgatga 1500
   ggagtggaag gatgagatgt acggcggcgt tgagggctgg tggggtctct tcacgaagga 1560
   gtacgttgct tcagtcctgc acgatgcaca cgatgctaaa gctttctagc cgccaactca 1620
   aggacctgac gatccccgac tgcgcggcgc cctaagcgtg ccaacctgct cgttatatat 1680
   tggtctgccg ctccgttcgt cccccctccc ccactttccc tgcctgcccc atcccacccc 1740
   gctttatgtg acagtcatcg caccgctatt ccaccgctaa gcagactttc cgtacttgca 1800
   cacgattaat ttttcgtcct gtctatacgg cctatccgca tgctatcctc ccgcccacct 1860
   taatgactac gtacgcgcgg ttgctggtga cgtgtgtggt tgctttctgc tcctgcagct 1920
   gcatgactgt acttgctgct gcactcgctc gcctgctggc caccgcgttt acgtgctcgc 1980
   ccgctactat acgcgctcga tcacttgcta ttccaacgga catgcttcga gagttacagg 2040
   ttcctactct cagctatcag actcgctttg tacacggact ttcgggcatt gtatgggtac 2100
   cacggtatac atcgaatggg aaccattgaa tgg 2133
<210> 11
   <211> 374
   <212> PRT
   <213> Schizophyllum commune
<400> 11
   Met Arg Leu Cys Trp Ala Asn Gly Leu Cys Cys Gln Val Ile Gly Ser
   1               5                   10                  15
   Val Val Ala Leu Val Val Ile Ile Ala Leu Ala Val Gly Leu Gly Val
               20                  25                  30
   Gly Leu Ser Arg Asn Asn Lys Ser Ser Ser Ser Gly Ser Lys Gly Gly
           35                  40                  45
   Ala Thr Asn Pro Glu Ser Ser Asp Ser Val Gly Ser Asp Pro Ser Asn
       50                  55                  60
   Phe Val Lys Asp Asp Arg Leu His Gln Ser Phe Tyr Gly Ile Ala Tyr
   65                  70                  75                  80
   Thr Pro Glu Gly Ser Ile Met Pro Asn Cys Gly Ala Thr Leu Glu Asn
                   85                  90                  95
   Val Ile Lys Asp Ile Gln Leu Met Ser Gln Leu Thr Lys Arg Val Arg
               100                 105                 110
   Leu Tyr Gly Ala Asp Cys Asn Gln Ser Ala Leu Val Leu Glu Ala Ile
           115                 120                 125
   Gln Gln Thr Lys Val Asp Met Gln Val Tyr Leu Ala Asn Tyr Asp Val
       130                 135                 140
   Pro Gly Asp Ser Asp Ala Tyr Thr Arg Gln Arg Asp Thr Leu Lys Glu
   145                 150                 155                 160
   Ala Ile Gln Thr Tyr Gly Thr Asp His Ile Ala Gly Ile Thr Val Gly
                   165                 170                 175
   Asn Glu Tyr Ile Leu Asn Tyr Leu Asn Asp Asn Gly Gly Thr Asp Pro
               180                 185                 190
   Asn Asp Ser Val Gly Asn Ala Gly Ala Lys Leu Leu Ile Ala Asp Ile
           195                 200                 205
   Asp Asp Thr Lys Gln Met Leu Ser Asp Leu Gly Val Asp Leu Pro Val
       210                 215                 220
   Gly Asn Ser Asp Ala Gly Ser Tyr Phe Asn Asp Gln Val Leu Glu Ser
   225                 230                 235                 240
   Val Asp Tyr Gly Met Ser Asn Val His Ala Trp Phe Ala Asn Thr Thr
                   245                 250                 255
   Ala Asp Asp Ala Ala Ser Trp Val Phe Ser Phe Phe Glu Glu Thr Asn
               260                 265                 270
   Val Glu Pro Ala Ser Gln Leu Ser Asn Lys Pro Gln Met Tyr Ile Ala
           275                 280                 285
   Glu Thr Gly Trp Pro Thr Lys Ser Ser Asp Glu Gly Asn Ala Asn Asn
       290                 295                 300
   Gly Ala Ser Asp Ala Ser Val Ala Asn Leu Gln Ile Phe Leu Asp Asp
   305                 310                 315                 320
   Phe Val Cys Gln Ala Asn Ala Asn Asn Thr Gly Tyr Phe Phe Phe Glu
                   325                 330                 335
   Phe Phe Asp Glu Glu Trp Lys Asp Glu Met Tyr Gly Gly Val Glu Gly
               340                 345                 350
   Trp Trp Gly Leu Phe Thr Lys Asp Arg Gln Leu Lys Asp Leu Thr Ile
           355                 360                 365
   Pro Asp Cys Ala Ala Pro
       370
<210> 12
   <211> 29
   <212> DNA
   <213> artificial (primer Sto658)
<400> 12
   ctagactagt atcgccattg taagccgca 29
<210> 13
   <211> 33
   <212> DNA
   <213> artificial (primer Sto659)
<400> 13
   ccggaattct ttgatgtttt ctaggtgaga ttg 33
<210> 14
   <211> 570
   <212> DNA
   <213> Schizophyllum commune
<400> 14
   atgacgacgc ttgacgacac ggcctatagg taccgcacct cggtaccggg cgatgcggaa      60
   gcgatcgagg cgctcgatgg atcttttacg actgacacag tcttccgcgt cacggcaaca     120
   ggcgatgggt ttactttgcg ggaggttccc gttgaccccc cactcaccaa ggtgttcccg     180
   gatgacgaga gcgacgatga atccgacgcc ggggaggacg gggaccctga ctcgcgtacc     240
   ttcgtcgctt acggtgatga cggcgaccta gcaggcttcg tggtggtaag ttattccggc     300
   tggaaccgac gccttactgt cgaggacatt gaggtcgccc ccgagcatcg tggtcacggc     360
   gtgggaaggg ccctcatggg gctcgctacg gagttcgcgc gcgaacgagg agctggccac     420
   ctgtggcttg aggtcacgaa cgtcaatgcc ccagcaatcc atgcgtaccg gcgcatgggc     480
   ttcaccttgt gtggcctcga caccgcgctg tacgatggta ccgcgagcga cggagaacag     540
   gcgctgtaca tgtcaatgcc ttgcccgtga                                      570
<210> 15
   <211> 73
   <212> DNA
   <213> artificial (primer Sto656)
<400> 15
   ataagaatgc ggccgcataa cttcgtatag catacattat acgaagttat tggatcgggc 60
   atgatgtaat cga 73
<210> 16
   <211> 65
   <212> DNA
   <213> artificial (primer Sto671)
<400> 16
   ctagactagt ataacttcgt ataatgtatg ctatacgaag ttatgttcag tagcatctgg 60
   ctctg 65
<210> 17
   <211> 1217
   <212> DNA
   <213> Schizophyllum commune
<400> 17
   ctgctcattg caggagctgt ggcaggtata gtcgctgggg tcttggtgtc aaggaataac 60
   agcagctcat cgaagtcgaa tttggcgtcg aactcgtcct catcgggaga tcccagcgac 120
   ttcgacaagg acgagaatct gcatgtctcg ttctacggta tcgcatgtgc gttcgtgccc 180
   tcttttcgag gtcgatcttt tcagacgacg ttccacagaa tacacccgcc ggttcgcagc 240
   tggacgccaa ttgtggcaac tcgctggacg atgtcatcaa ggacgttcag cttctatccc 300
   agatcacgac tgtatgttct cacatctacc cttcaaaaat cacccttgct cacacaccct 360
   ctagcgcatc cgcctatacg gcgccgattg caatcaatcc gcgctagttc tcgaggcgat 420
   ccaacagacg aaggtcgatc tcacggtctg gctgggtaac tacgtctccg ccaccgacgg 480
   tggagaggcg tacgagcggc agcgcgacac gctcaaggag gtcatccaga catatggcac 540
   agatcatatt ggggtgagca cgcagcgctc acattcttct ctgtatggtc gctcatgccc 600
   gctttgcagc ggtatcactg ttggcaatga gtttatgatg ctcatcacca acatcaccga 660
   cactcgaagc atgctcagtg acataggcgt cgatctatcc gtcggcacgg cggacgcggg 720
   cgcgtacttc aacgagaaac tgttgtcgtc ggtggactat ggggtacgtc ttccgcgtat 780
   aacatgacgc tcggctgtag ccactcttca cgatcgtact aacgaatggt ccacccagat 840
   ggccaacgtg cacccctggt tcggcgacgt cagcatcgac gacgctgcaa catggacctg 900
   gcagttcttc cagaccaacg atgtcagcat ctccgacgag gtcgacaaca agccgcagat 960
   gtacatcgcg gagaccgggt ggccgacggt gcgtgtctgc tctttgcttc cctcgtcgct 1020
   actcttgtta cctacaactg aacccttcca gaaatcctct aacaccagca ccgaaacgaa 1080
   tggggcctca gaggcttccg aggccaacct tcagatcttc ctcgacacct tcgtctgtca 1140
   ggcgaatgcg aatgggacgg agtatttctt ctttgaagtg cgtttccggg agtgttcatc 1200
   gtggggtctg cagctaa 1217
<210> 18
   <211> 405
   <212> PRT
   <213> Schizophyllum commune
<220>
   <221> unsure
   <222> 153 .. 153
   <223> All occurrences of Xaa indicate any amino acid
<220>
   <221> unsure
   <222> 210 .. 210
   <223> All occurrences of Xaa indicate any amino acid
<220>
   <221> unsure
   <222> 227 .. 227
   <223> All occurrences of Xaa indicate any amino acid
<220>
   <221> unsure
   <222> 274 .. 274
   <223> All occurrences of Xaa indicate any amino acid
<220>
   <221> unsure
   <222> 347 .. 347
   <223> All occurrences of Xaa indicate any amino acid
<220>
   <221> unsure
   <222> 354 .. 354
   <223> All occurrences of Xaa indicate any amino acid
<220>
   <221> unsure
   <222> 392 .. 392
   <223> All occurrences of Xaa indicate any amino acid
<400> 18
   Leu Leu Ile Ala Gly Ala Val Ala Gly Ile Val Ala Gly Val Leu Val
   1               5                   10                  15
   Ser Arg Asn Asn Ser Ser Ser Ser Lys Ser Asn Leu Ala Ser Asn Ser
               20                  25                  30
   Ser Ser Ser Gly Asp Pro Ser Asp Phe Asp Lys Asp Glu Asn Leu His
           35                  40                  45
   Val Ser Phe Tyr Gly Ile Ala Cys Ala Phe Val Pro Ser Phe Arg Gly
       50                  55                  60
   Arg Ser Phe Gln Thr Thr Phe His Arg Ile His Pro Pro Val Arg Ser
   65 70 75 80
   Trp Thr Pro Ile Val Ala Thr Arg Trp Thr Met Ser Ser Arg Thr Phe
   85 90 95
   Ser Phe Tyr Pro Arg Ser Arg Leu Tyr Val Leu Thr Ser Thr Leu Gln
   100 105 110
   Lys Ser Pro Leu Leu Thr His Pro Leu Ala His Pro Pro Ile Arg Arg
   115 120 125
   Arg Leu Gln Ser Ile Arg Ala Ser Ser Arg Gly Asp Pro Thr Asp Glu
   130 135 140
   Gly Arg Ser His Gly Leu Ala Gly Xaa Leu Arg Leu Arg His Arg Arg
   145 150 155 160
   Trp Arg Gly Val Arg Ala Ala Ala Arg His Ala Gln Gly Gly His Pro
   165 170 175
   Asp Ile Trp His Arg Ser Tyr Trp Gly Glu His Ala Ala Leu Thr Phe
   180 185 190
   Phe Ser Val Trp Ser Leu Met Pro Ala Leu Gln Arg Tyr His Cys Trp
   195 200 205
   Gln Xaa Val Tyr Asp Ala His His Gln His His Arg His Ser Lys His
   210 215 220
   Ala Gln Xaa His Arg Arg Arg Ser Ile Arg Arg His Gly Gly Arg Gly
   225 230 235 240
   Arg Val Leu Gln Arg Glu Thr Val Val Val Gly Gly Leu Trp Gly Thr
   245 250 255
   Ser Ser Ala Tyr Asn Met Thr Leu Gly Cys Ser His Ser Ser Arg Ser
   260 265 270
   Tyr Xaa Arg Met Val His Pro Asp Gly Gln Arg Ala Pro Leu Val Arg
   275 280 285
   Arg Arg Gln His Arg Arg Arg Cys Asn Met Asp Leu Ala Val Leu Pro
   290 295 300
   Asp Gln Arg Cys Gln His Leu Arg Arg Gly Arg Gln Gln Ala Ala Asp
   305 310 315 320
   Val His Arg Gly Asp Arg Val Ala Asp Gly Ala Cys Leu Leu Phe Ala
   325 330 335
   Ser Leu Val Ala Thr Leu Val Thr Tyr Asn Xaa Thr Leu Pro Glu Ile
   340 345 350
   Leu Xaa His Gln His Arg Asn Glu Trp Gly Leu Arg Gly Phe Arg Gly
   355 360 365
   Gln Pro Ser Asp Leu Pro Arg His Leu Arg Leu Ser Gly Glu Cys Glu
   370 375 380
   Trp Asp Gly Val Phe Leu Leu Xaa Ser Ala Phe Pro Gly Val Phe Ile
   385 390 395 400
   Val Gly Ser Ala Ala
   405
<210> 19
   <211> 30
   <212> DNA
   <213> artificial (primer Sto381)
<400> 19
   ccggaattcc tgctcattgc aggagctgtc 30
<210> 20
   <211> 21
   <212> DNA
   <213> artificial (primer Sto348)
<400> 20
   gacctaaact gcggattctc g 21
<210> 21
   <211> 28
   <212> DNA
   <213> artificial (primer Sto393)
<400> 21
   cgcggatccg ggttcagtag catctggc 28
<210> 22
   <211> 31
   <212> DNA
   <213> artificial (primer Sto282)
<400> 22
   cctgatgtgg ttcctcatct tcgggtgcaa g 31
<210> 23
   <211> 30
   <212> DNA
   <213> artificial (primer Sto381)
<400> 23
   ccggaattcc tgctcattgc aggagctgtc 30
<210> 24
   <211> 30
   <212> DNA
   <213> artificial (primer Sto382)
<400> 24
   cggggtaccg ggttcagtag catctggctc 30
<210> 25
   <211> 1745
   <212> DNA
   <213> Schizophyllum commune
<400> 25
   atgtcggact acaagagcgg aatggctgca gcgcttgaga agagcgaggc gcgctcgcgg 60
   cgggcgaaga agatcgtgcg ttgtgctcgt acgtgggcgg ttgtgctggg ctaacgggct 120
   gtgctgccag gtcatcggca gtgtcgtcgc cctcgtcgtg atcattgcgc tggccgtcgg 180
   tcttggtgtc ggtctctcgc gcaacaagtc ctcttcctct ggcagcaagg gcggagcgac 240
   caaccctgag tcgtcagact cggtcggcag cgacccgtcc aacttcgtca aggacgaccg 300
   cttgcaccag tccttctacg gcattgctta cacgccggag ggctcgatta tgccgaactg 360
   tggcgctacg cttggtgagc gcgacgtgtc agacaggcta cttgcagact aatgtagcgc 420
   gcagagaatg tcatcaagga tatccaggtg ggcagagagc agcgtcctat cgagacgcgc 480
   gactgacacg ccacacagct catgtcgcag ctaacgaagc gtgtgcgctt gtatggcgcg 540
   gactgtaacc agtcggcgct tgtggtacgt cctcgagacc gagttctttg cggcctgatc 600
   taaccatcgc actgcttctc acagctcgag gccatccagc agaccaaggt cgagtgagtg 660
   cttcgaggac ctgtgcccgc tgaaacctca atgagctgac ccgtgaccta cagcatgcaa 720
   gtgtacctcg ccaactacga cgttcctggc gactcggacg cgtatacgcg ccagcgtgat 780
   accctcaagg aggcgatcca gacctacggc acggaccata ttgcgggcat caccgtgggc 840
   aatgagtaca tcctcaagta agcgcccttc ctgctcgtag gatggagtac aagctgatga 900
   gtagcgcagc tacctcaacg acaatggcgg cacggacccc aatgactctg tcggcaacgc 960
   gggcgccaag cttctcattg ccgacatcga tgacaccaag cagatgctga gcgaccttgg 1020
   cgtcgacctc ccggtcggca actctgatgc gggcagctac ttcaacgatc aagtccttga 1080
   gagcgttgac tatggcgtac gtatatttct tggcaaagct tagggaatta tctcaccctc 1140
   aactagatgt cgaacgttca cgcttggttc gcgaacacca cggcggatga cgcagcgtca 1200
   tgggtgttct cgttcttcga agagaccaat gtcgagcccg cgtcgcagtt gagcaataag 1260
   ccgcagatgt acattgcgga gaccggctgg ccgacagtga gaacaaagca tgtatactcc 1320
   tggatagtcg gactgattga atgtctccag aaatcgtctg acgaaggcaa cgccaacaac 1380
   ggtgccagcg acgcatctgt ggctaatctc caggtaagct gaaaatgata cctgaccgac 1440
   aatgtattga cgtggcttgc agatcttcct cgacgacttc gtctgccaag cgaatgccaa 1500
   caacaccggc tacttcttct tcgaggttag tcaggagcct tcagaaagcc aaggtacctt 1560
   gctgacagaa tcatgatagt tcttcgacga ggagtggaag gatgagatgt acggcggtgt 1620
   tgagggctgg tggggtctct tcacgaagga gtatgttgct tccatcctga cacgatgcaa 1680
   acgatgctaa aatgtcctct agccgtcaac tcaaggacct gacgatcccc gactgcgcag 1740
   cgcct 1745
<210> 26
   <211> 386
   <212> PRT
   <213> Schizophyllum commune
<400> 26
   Met Ser Asp Tyr Lys Ser Gly Met Ala Ala Ala Leu Glu Lys Ser Glu
   1               5                   10                  15
   Ala Arg Ser Arg Arg Ala Lys Lys Ile Val Ile Gly Ser Val Val Ala
               20                  25                  30
   Leu Val Val Ile Ile Ala Leu Ala Val Gly Leu Gly Val Gly Leu Ser
           35                  40                  45
   Arg Asn Lys Ser Ser Ser Ser Gly Ser Lys Gly Gly Ala Thr Asn Pro
       50                  55                  60
   Glu Ser Ser Asp Ser Val Gly Ser Asp Pro Ser Asn Phe Val Lys Asp
   65                  70                  75                  80
   Asp Arg Leu His Gln Ser Phe Tyr Gly Ile Ala Tyr Thr Pro Glu Gly
                   85                  90                  95
   Ser Ile Met Pro Asn Cys Gly Ala Thr Leu Glu Asn Val Ile Lys Asp
               100                 105                 110
   Ile Gln Leu Met Ser Gln Leu Thr Lys Arg Val Arg Leu Tyr Gly Ala
           115                 120                 125
   Asp Cys Asn Gln Ser Ala Leu Val Leu Glu Ala Ile Gln Gln Thr Lys
       130                 135                 140
   Val Asp Met Gln Val Tyr Leu Ala Asn Tyr Asp Val Pro Gly Asp Ser
   145                 150                 155                 160
   Asp Ala Tyr Thr Arg Gln Arg Asp Thr Leu Lys Glu Ala Ile Gln Thr
                   165                 170                 175
   Tyr Gly Thr Asp His Ile Ala Gly Ile Thr Val Gly Asn Glu Tyr Ile
               180                 185                 190
   Leu Asn Tyr Leu Asn Asp Asn Gly Gly Thr Asp Pro Asn Asp Ser Val
           195                 200                 205
   Gly Asn Ala Gly Ala Lys Leu Leu Ile Ala Asp Ile Asp Asp Thr Lys
       210                 215                 220
   Gln Met Leu Ser Asp Leu Gly Val Asp Leu Pro Val Gly Asn Ser Asp
   225                 230                 235                 240
   Ala Gly Ser Tyr Phe Asn Asp Gln Val Leu Glu Ser Val Asp Tyr Gly
                   245                 250                 255
   Met Ser Asn Val His Ala Trp Phe Ala Asn Thr Thr Ala Asp Asp Ala
               260                 265                 270
   Ala Ser Trp Val Phe Ser Phe Phe Glu Glu Thr Asn Val Glu Pro Ala
           275                 280                 285
   Ser Gln Leu Ser Asn Lys Pro Gln Met Tyr Ile Ala Glu Thr Gly Trp
       290                 295                 300
   Pro Thr Lys Ser Ser Asp Glu Gly Asn Ala Asn Asn Gly Ala Ser Asp
   305                 310                 315                 320
   Ala Ser Val Ala Asn Leu Gln Ile Phe Leu Asp Asp Phe Val Cys Gln
                   325                 330                 335
   Ala Asn Ala Asn Asn Thr Gly Tyr Phe Phe Phe Glu Phe Phe Asp Glu
               340                 345                 350
   Glu Trp Lys Asp Glu Met Tyr Gly Gly Val Glu Gly Trp Trp Gly Leu
           355                 360                 365
   Phe Thr Lys Asp Arg Gln Leu Lys Asp Leu Thr Ile Pro Asp Cys Ala
       370                 375                 380
   Ala Pro
   385
<210> 27
   <211> 1748
   <212> DNA
   <213> Schizophyllum commune
<220>
   <221> unsure
   <222> 4 .. 4
   <223> All occurrences of n indicate any nucleotide
<220>
   <221> unsure
   <222> 5 .. 5
   <223> All occurrences of n indicate any nucleotide
<220>
   <221> unsure
   <222> 6 .. 6
   <223> All occurrences of n indicate any nucleotide
<400> 27
   atgnnntcgg actacaagag cggaatggct gcagcgcttg agaagagcga ggcgcgctcg 60
   cggcgggcga agaagatcgt gcgttgtgct cgtacgtggg cggttgtgct gggctaacgg 120
   gctgtgctgc caggtcatcg gcagtgtcgt cgccctcgtc gtgatcattg cgctggccgt 180
   cggtcttggt gtcggtctct cgcgcaacaa gtcctcttcc tctggcagca agggcggagc 240
   gaccaaccct gagtcgtcag actcggtcgg cagcgacccg tccaacttcg tcaaggacga 300
   ccgcttgcac cagtccttct acggcattgc ttacacgccg gagggctcga ttatgccgaa 360
   ctgtggcgct acgcttggtg agcgcgacgt gtcagacagg ctacttgcag actaatgtag 420
   cgcgcagaga atgtcatcaa ggatatccag gtgggcagag agcagcgtcc tatcgagacg 480
   cgcgactgac acgccacaca gctcatgtcg cagctaacga agcgtgtgcg cttgtatggc 540
   gcggactgta accagtcggc gcttgtggta cgtcctcgag accgagttct ttgcggcctg 600
   atctaaccat cgcactgctt ctcacagctc gaggccatcc agcagaccaa ggtcgagtga 660
   gtgcttcgag gacctgtgcc cgctgaaacc tcaatgagct gacccgtgac ctacagcatg 720
   caagtgtacc tcgccaacta cgacgttcct ggcgactcgg acgcgtatac gcgccagcgt 780
   gataccctca aggaggcgat ccagacctac ggcacggacc atattgcggg catcaccgtg 840
   ggcaatgagt acatcctcaa gtaagcgccc ttcctgctcg taggatggag tacaagctga 900
   tgagtagcgc agctacctca acgacaatgg cggcacggac cccaatgact ctgtcggcaa 960
   cgcgggcgcc aagcttctca ttgccgacat cgatgacacc aagcagatgc tgagcgacct 1020
   tggcgtcgac ctcccggtcg gcaactctga tgcgggcagc tacttcaacg atcaagtcct 1080
   tgagagcgtt gactatggcg tacgtatatt tcttggcaaa gcttagggaa ttatctcacc 1140
   ctcaactaga tgtcgaacgt tcacgcttgg ttcgcgaaca ccacggcgga tgacgcagcg 1200
   tcatgggtgt tctcgttctt cgaagagacc aatgtcgagc ccgcgtcgca gttgagcaat 1260
   aagccgcaga tgtacattgc ggagaccggc tggccgacag tgagaacaaa gcatgtatac 1320
   tcctggatag tcggactgat tgaatgtctc cagaaatcgt ctgacgaagg caacgccaac 1380
   aacggtgcca gcgacgcatc tgtggctaat ctccaggtaa gctgaaaatg atacctgacc 1440
   gacaatgtat tgacgtggct tgcagatctt cctcgacgac ttcgtctgcc aagcgaatgc 1500
   caacaacacc ggctacttct tcttcgaggt tagtcaggag ccttcagaaa gccaaggtac 1560
   cttgctgaca gaatcatgat agttcttcga cgaggagtgg aaggatgaga tgtacggcgg 1620
   tgttgagggc tggtggggtc tcttcacgaa ggagtatgtt gcttccatcc tgacacgatg 1680
   caaacgatgc taaaatgtcc tctagccgtc aactcaagga cctgacgatc cccgactgcg 1740
   cagcgcct 1748
<210> 28
   <211> 2166
   <212> DNA
   <213> Schizophyllum commune
<400> 28
   atgtcggtca aggagatctc cacgaaacct tttgagggcc agaagcccgg cacctccggc      60
   ctgcgcaaga ggtgtgtagc gcgcgccgct cgtcgcttat cgcgctaaca cgacgcgtcg     120
   cgcctagggt caagatcttc cagcaggagg tgcgcagcgg cgtcctacgt tcatcgatgg     180
   gcgcgcaatg ctcacagttc catagcacta caccgagaac ttcgttcagt ccatcttcga     240
   cgcggttgac ctgaacggcg cgacgctcgt cgtcggcggc gacggccgct tcttctcgcc     300
   cgagacggtg cagacgatcc tcaagatcgg ctccgcgaac ggcgtcgcga agttcatcat     360
   cgggcaggcg ggcatcctgt cgacgcccgc cgcgtcgaac gtgattcgca agtacaaggc     420
   gaacggcggg atcctgctca ccgcgagcca caacccgggc ggcccggaca atgactttgg     480
   gatcaagtac aatgtgtcga atgggggccc ggcgccggag aatgtgacga acaagatcta     540
   tgagaagacg aaggagatct ccaagtacaa ggtcttcgag ctgccgcagg tgtgtgtctg     600
   cgtcgtcgat cactgcccgt caccccggcg cgtctttgtc tagctgcgaa gagcgcagat     660
   gtatgtgcag tctgcttgca gtacgatctc tgtgcttacg ccatgagcgg ccatgggcaa     720
   gcaccctgct gggcggcctt tgcgccagcc gatggtcgtc ctcctagatt gtactgcgtc     780
   agatagtacg cgctgactgc actcgtcgca actggattgg gttgcatcgg ggtcccatgg     840
   ccgtgatcct atattctaga aactgacgac gcgcccccag atcgatctat ccaagatcgg     900
   cgagactaca tacggcccct ccaaagtcca gatcatcgac tccgtctcag actacgtcga     960
   gctcctcaag tcgatcttcg actttgacct catcaaaagc ttcctcctca ccaaccagga    1020
   ctcctttaag gtcctcttcg acggccttca cggcgtcacg ggcccgtacg cccataggat 1080
   cctggtcaag gagctcggcc tgcccgagac ctccgtgcag aactgcaagc cgctgcccga 1140
   ctttggcggt gggcaccccg acccgaacct tacctacgcc cactcgctcg tcgaggccgt 1200
   cgagaagaac aacatcgctt tcggcgctgc cagcgacggc gatggtgacc gtaacatgat 1260
   ctacggcaag ggcgcgttcg tgacaccatc ggactcagtg gcaatcatcg cagactgggc 1320
   cgagtccatc ccatacttca agaagggtgg cgtcaagggt ctcgcgcgga gcatgcctac 1380
   cagcaaggct atcgaccttg tcgcgcaaaa gaagggtctc gagcactttg aggttccgac 1440
   gggtgcgtat ctcttgcatt ctggcccgcg tgtatgttgt cgcgagtgca attggcagcc 1500
   gtgctcacct aaattcttct aggctggaag ttcttcggca acttgatgga cgctggtagg 1560
   ctatccatct gcggcgagga gtccttcggt accggctccg accacatccg tgagaaggac 1620
   ggtatctggg cgatcatcgc ctggctcaac atcctcgccg ccgccaaccg cgagtcgccc 1680
   aacaagctca tcggcatcaa ggagctgctc gagcgccact actccatcta cggccgctcc 1740
   ttcttctcgc gctacgacta cgaggaggtc tcctccgagg gcgcgaacgc gctcgtcaag 1800
   gcgctcgacg accacctcgg cgcgagctcg ctcaccgggc agaccttcgg ccccgggggc 1860
   aagttcaagg tcgcggacct gtacaacttc gcgtacacgg acccgatcga caagtcggtg 1920
   tcgaagaacc aggggcacgt catcacgttc gacgacggct cacgtgtcgt gttccggttg 1980
   agcgggacgg gcagccaggg cgcgacggtg cggatgtaca tcgagcgcta cctaggcccc 2040
   gacgcgagca aggaggcgct cgcggaggac gcggcgaagg ggctgaagga cctgatcgag 2100
   gtcgcgctcg agatcagcaa gctgaaggag ttcatcggcc gtgacaagcc tacggttatc 2160
   acgtga 2166
<210> 29
   <211> 593
   <212> PRT
   <213> Schizophyllum commune
<400> 29
   Met Ser Val Lys Glu Ile Ser Thr Lys Pro Phe Glu Gly Gln Lys Pro
   1               5                   10                  15
   Gly Thr Ser Gly Leu Arg Lys Arg Val Lys Ile Phe Gln Gln Glu His
               20                  25                  30
   Tyr Thr Glu Asn Phe Val Gln Ser Ile Phe Asp Ala Val Asp Leu Asn
           35                  40                  45
   Gly Ala Thr Leu Val Val Gly Gly Asp Gly Arg Phe Phe Ser Pro Glu
       50                  55                  60
   Thr Val Gln Thr Ile Leu Lys Ile Gly Ser Ala Asn Gly Val Ala Lys
   65                  70                  75                  80
   Phe Ile Ile Gly Gln Ala Gly Ile Leu Ser Thr Pro Ala Ala Ser Asn
                   85                  90                  95
   Val Ile Arg Lys Tyr Lys Ala Asn Gly Gly Ile Leu Leu Thr Ala Ser
               100                 105                 110
   His Asn Pro Gly Gly Pro Asp Asn Asp Phe Gly Ile Lys Tyr Asn Val
           115                 120                 125
   Ser Asn Gly Gly Pro Ala Pro Glu Asn Val Thr Asn Lys Ile Tyr Glu
       130                 135                 140
   Lys Thr Lys Glu Ile Ser Lys Tyr Lys Val Phe Glu Leu Pro Gln Ile
   145                 150                 155                 160
   Asp Leu Ser Lys Ile Gly Glu Thr Thr Tyr Gly Pro Ser Lys Val Gln
                   165                 170                 175
   Ile Ile Asp Ser Val Ser Asp Tyr Val Glu Leu Leu Lys Ser Ile Phe
               180                 185                 190
   Asp Phe Asp Leu Ile Lys Ser Phe Leu Leu Thr Asn Gln Asp Ser Phe
           195                 200                 205
   Lys Val Leu Phe Asp Gly Leu His Gly Val Thr Gly Pro Tyr Ala His
       210                 215                 220
   Arg Ile Leu Val Lys Glu Leu Gly Leu Pro Glu Thr Ser Val Gln Asn
   225                 230                 235                 240
   Cys Lys Pro Leu Pro Asp Phe Gly Gly Gly His Pro Asp Pro Asn Leu
                   245                 250                 255
   Thr Tyr Ala His Ser Leu Val Glu Ala Val Glu Lys Asn Asn Ile Ala
               260                 265                 270
   Phe Gly Ala Ala Ser Asp Gly Asp Gly Asp Arg Asn Met Ile Tyr Gly
           275                 280                 285
   Lys Gly Ala Phe Val Thr Pro Ser Asp Ser Val Ala Ile Ile Ala Asp
       290                 295                 300
   Trp Ala Glu Ser Ile Pro Tyr Phe Lys Lys Gly Gly Val Lys Gly Leu
   305                 310                 315                 320
   Ala Arg Ser Met Pro Thr Ser Lys Ala Ile Asp Leu Val Ala Gln Lys
                   325                 330                 335
   Lys Gly Leu Glu His Phe Glu Val Pro Thr Gly Trp Lys Phe Phe Gly
               340                 345                 350
   Asn Leu Met Asp Ala Gly Arg Leu Ser Ile Cys Gly Glu Glu Ser Phe
           355                 360                 365
   Gly Thr Gly Ser Asp His Ile Arg Glu Lys Asp Gly Ile Trp Ala Ile
       370                 375                 380
   Ile Ala Trp Leu Asn Ile Leu Ala Ala Ala Asn Arg Glu Ser Pro Asn
   385                 390                 395                 400
   Lys Leu Ile Gly Ile Lys Glu Leu Leu Glu Arg His Tyr Ser Ile Tyr
                   405                 410                 415
   Gly Arg Ser Phe Phe Ser Arg Tyr Asp Tyr Glu Glu Val Ser Ser Glu
               420                 425                 430
   Gly Ala Asn Ala Leu Val Lys Ala Leu Asp Asp His Leu Gly Ala Ser
           435                 440                 445
   Ser Leu Thr Gly Gln Thr Phe Gly Pro Gly Gly Lys Phe Lys Val Ala
       450                 455                 460
   Asp Leu Tyr Asn Phe Ala Tyr Thr Asp Pro Ile Asp Lys Ser Val Ser
   465                 470                 475                 480
   Lys Asn Gln Gly His Val Ile Thr Phe Asp Asp Gly Ser Arg Val Val
                   485                 490                 495
   Phe Arg Leu Ser Gly Thr Gly Ser Gln Gly Ala Thr Val Arg Met Tyr
               500                 505                 510
   Ile Glu Arg Tyr Leu Gly Pro Asp Ala Ser Lys Glu Ala Leu Ala Glu
           515                 520                 525
   Asp Ala Ala Lys Gly Leu Lys Asp Leu Ile Glu Val Ala Leu Glu Ile
       530                 535                 540
   Ser Lys Leu Lys Glu Phe Ile Gly Arg Asp Lys Pro Thr Val Ile Thr
   545                 550                 555                 560
   Val Arg Cys Ala Ser Phe His Leu Arg Thr Trp Leu Thr Asn Leu Cys
   565 570 575
   Ser Lys Arg Met Glu Asn Gly Arg Asn Trp Lys Trp Leu Leu Tyr His
   580 585 590
   Thr
<210> 30
   <211> 20
   <212> DNA
   <213> artificial (primer Sto687)
<400> 30
   atgtcggtca aggagatctc 20
<210> 31
   <211> 22
   <212> DNA
   <213> artificial (primer Sto688)
<400> 31
   tcacgtgata accgtaggct tg 22
<210> 32
   <211> 2452
   <212> DNA
   <213> Schizophyllum commune
<400> 32
   gctttccctc ccgaccgccg tttcctgcac gttcgtcgcc gtcccgccag cccaagctgc 60
   tctcattcca gctcaaatct cagaagcggc gttcttggtt ttctccaatc cgcgaggaaa 120
   cacatttgga gcagagcgag tcggctgaac accttccccg acgtcataaa accaggtcac 180
   tgctcacgag aaccacttcc gccccggcct ccatcttgtc ccttaccacc accgccccca 240
   tccacatcaa caatccgagg tccgctcacc atcgtcccct tcgtcgcgca gccctgaccc 300
   catctagagc agccatggca agccttatgc ccaaccgcgc agagtcgttc aacacccgca 360
   ctcggggaac gagccatatc gatttcaaga ccgcgaccac cggtgtcgcg gccaaggcca 420
   tgcgcaatga gctgagccat ctcgtctcca cgatctccga cccgcaggag aagaaggtgc 480
   gtccgccgag cgcagcatac gcgtcgcgac tgaccccagt gcgcagaact ttgacatgga 540
   gatgcaatcc ttcttcttcc tcttcacccg ctacctcgcc gagcgcgccc agagccagga 600
   tctgtgcgtg atctgatcct gtctgcccat cgcacgcgct gatcgacctc ctagcgattg 660
   ggaccgtatc aagtcgccct ccgatgacaa ggtcgtcccc tatgcgcagc tctccgagcc 720
   caaggacaag agcgtcctca acaagctcgc cgtcctaaag gtcaacggtg gtctcggcac 780
   gtccatgggc atgaccggcg cgaagagcgc gctcgaggtc aagaacgaca tgaccttcct 840
   cgacctcacc gtgcgacaga tcgagcacct caacacgagc aaccgcgtcg atgttccgct 900
   cctcctcatg acgtccttca acacgcatga cgataccctg cgcatcatca agaagtacgc 960
   gaatcagcag ttgcgcatca ccactttcaa ccagagccgc tacccccgta tcttcaagga 1020
   gaccctcctc ccgtgtccca agcgcgcgga cgatgacaag aagcactggt acccgcccgg 1080
   tcacggtgac gttttcaacg cgctctacca gtctggtgtc ctcgatcagt tgatctcgga 1140
   gggcaaggag tacatcttca tctcgaactc ggacaacttg ggcgctgtgt gcgtatcgtt 1200
   tgccctgtta ctttaccatg cactcactcg tggtacagcg tcgacgagaa gattctccag 1260
   cacatgatcg actctgaggc tgagttcatc atggaggtca ccgacaagac gaaggctgac 1320
   gtcaaggtga atgcagcaaa gcatcatacg gagatgttat actgacgctt ccaccagggt 1380
   ggtaccctca tcgactacga tggcaacatc cgcctcctcg aaattgctca ggtcccatcc 1440
   gagcatgtcg aggacttcaa gtccgtccgc aagttcaaga tcttcaacac caataacctc 1500
   tgggtgaacc tgaagggtaa gctatccctc gtttcggcct ctcctcccgc tcacaatttt 1560
   gatagcgatc aagcgcgtca tggagaacga gggcatggag ctcgagatca tcatcaaccc 1620
   gaaggtgaac gacgacggcc agccggtcat ccaactcgag accgccgcgg gtgccgcgat 1680
   caagcacttc aagaacgggc acggtatcaa cgtgccgcgg tcgcgcttct tgcccgtcaa 1740
   gagctgctcg gacctgctgc tgatcaaatc ggacatttac tcgcttgagc acggccagct 1800
   cgtcatcaac gagcagcgca tgttcgagac gacgccggtg atcaagcttg gggaccactt 1860
   caagaaggtg aggagggtgt gggttgtgaa gagtggggca aggcgctgac gcgggtcgta 1920
   cagatccaac aattccagaa gcgcttcaag aagatcccca agatcctcga gctcgaccac 1980
   ttgacggtta ctggcgacgt ctactttggc cgcaacgtca ctttgcgcgg gacggtcatc 2040
   agtgcgtctt gcctttcgtt cacgctgccg tgttcaagct aactctgctt tagtcgtcgc 2100
   gaacgagggg cagagcatct acatccccga cggctgcata ctggagaacc gcctcctgtc 2160
   gggcaacctg aacatgattg agctgtgatg agcgtcgacg acggactgaa atacacgaaa 2220
   ttacgatggg aatgctatgg acaaaatctt gagtagggta gcttttgtcg tttggggtag 2280
   tgcgcgcgct cggcgagggc tgcgcgagca gggttttacc tcgcgaaata ccaacgaaag 2340
   cgacacttga gtacgagtgc tttgattttc ttgcggactt tagacatgcc ctagaatata 2400
   tggcagagaa tgtatgagga tgggtttcgt agttctttct tgacaacgtg aa 2452
<210> 33
   <211> 504
   <212> PRT
   <213> Schizophyllum commune
<400> 33
   Met Ala Ser Leu Met Pro Asn Arg Ala Glu Ser Phe Asn Thr Arg Thr
   1               5                   10                  15
   Arg Gly Thr Ser His Ile Asp Phe Lys Thr Ala Thr Thr Gly Val Ala
               20                  25                  30
   Ala Lys Ala Met Arg Asn Glu Leu Ser His Leu Val Ser Thr Ile Ser
           35                  40                  45
   Asp Pro Gln Glu Lys Lys Asn Phe Asp Met Glu Met Gln Ser Phe Phe
       50                  55                  60
   Phe Leu Phe Thr Arg Tyr Leu Ala Glu Arg Ala Gln Ser Gln Asp Leu
   65                  70                  75                  80
   Asp Trp Asp Arg Ile Lys Ser Pro Ser Asp Asp Lys Val Val Pro Tyr
                   85                  90                  95
   Ala Gln Leu Ser Glu Pro Lys Asp Lys Ser Val Leu Asn Lys Leu Ala
               100                 105                 110
   Val Leu Lys Val Asn Gly Gly Leu Gly Thr Ser Met Gly Met Thr Gly
           115                 120                 125
   Ala Lys Ser Ala Leu Glu Val Lys Asn Asp Met Thr Phe Leu Asp Leu
       130                 135                 140
   Thr Val Arg Gln Ile Glu His Leu Asn Thr Ser Asn Arg Val Asp Val
   145                 150                 155                 160
   Pro Leu Leu Leu Met Thr Ser Phe Asn Thr His Asp Asp Thr Leu Arg
                   165                 170                 175
   Ile Ile Lys Lys Tyr Ala Asn Gln Gln Leu Arg Ile Thr Thr Phe Asn
               180                 185                 190
   Gln Ser Arg Tyr Pro Arg Ile Phe Lys Glu Thr Leu Leu Pro Cys Pro
           195                 200                 205
   Lys Arg Ala Asp Asp Asp Lys Lys His Trp Tyr Pro Pro Gly His Gly
       210                 215                 220
   Asp Val Phe Asn Ala Leu Tyr Gln Ser Gly Val Leu Asp Gln Leu Ile
   225                 230                 235                 240
   Ser Glu Gly Lys Glu Tyr Ile Phe Ile Ser Asn Ser Asp Asn Leu Gly
                   245                 250                 255
   Ala Val Val Asp Glu Lys Ile Leu Gln His Met Ile Asp Ser Glu Ala
               260                 265                 270
   Glu Phe Ile Met Glu Val Thr Asp Lys Thr Lys Ala Asp Val Lys Gly
           275                 280                 285
   Gly Thr Leu Ile Asp Tyr Asp Gly Asn Ile Arg Leu Leu Glu Ile Ala
       290                 295                 300
   Gln Val Pro Ser Glu His Val Glu Asp Phe Lys Ser Val Arg Lys Phe
   305                 310                 315                 320
   Lys Ile Phe Asn Thr Asn Asn Leu Trp Val Asn Leu Lys Ala Ile Lys
                   325                 330                 335
   Arg Val Met Glu Asn Glu Gly Met Glu Leu Glu Ile Ile Ile Asn Pro
               340                 345                 350
   Lys Val Asn Asp Asp Gly Gln Pro Val Ile Gln Leu Glu Thr Ala Ala
           355                 360                 365
   Gly Ala Ala Ile Lys His Phe Lys Asn Gly His Gly Ile Asn Val Pro
       370                 375                 380
   Arg Ser Arg Phe Leu Pro Val Lys Ser Cys Ser Asp Leu Leu Leu Ile
   385                 390                 395                 400
   Lys Ser Asp Ile Tyr Ser Leu Glu His Gly Gln Leu Val Ile Asn Glu
                   405                 410                 415
   Gln Arg Met Phe Glu Thr Thr Pro Val Ile Lys Leu Gly Asp His Phe
               420                 425                 430
   Lys Lys Ile Gln Gln Phe Gln Lys Arg Phe Lys Lys Ile Pro Lys Ile
           435                 440                 445
   Leu Glu Leu Asp His Leu Thr Val Thr Gly Asp Val Tyr Phe Gly Arg
       450                 455                 460
   Asn Val Thr Leu Arg Gly Thr Val Ile Ile Val Ala Asn Glu Gly Gln
   465                 470                 475                 480
   Ser Ile Tyr Ile Pro Asp Gly Cys Ile Leu Glu Asn Arg Leu Leu Ser
                   485                 490                 495
   Gly Asn Leu Asn Met Ile Glu Leu
               500
<210> 34
   <211> 28
   <212> DNA
   <213> artificial (primer Sto126)
<400> 34
   ctagactagt gctttccctc ccgaccgc 28
<210> 35
   <211> 24
   <212> DNA
   <213> artificial (primer Sto334)
<400> 35
   ccggaattca cgttgtcaag aaag 24
<210> 36
   <211> 2321
   <212> DNA
   <213> Schizophyllum commune
<400> 36
   cttgccccca tctccccatc tcttccccgc tctacttgca ttcagacgcg cctcgacgcc        60
   tcccgcagcg accatgagca cactagcgac cccctcgaac gcgcggcagc cgcgcacccg       120
   cggcgtgtcc gccatggtca gtactgccta aagcctctgc gcctcccgcg agcgttgcta       180
   atgcatatct ctgcctgcat gcaattcttc gcccatatct ttgccgcgcc tgacaaatcg       240
   cctcgccgcc gcctgcgatt gcgccccacg cgcttgtcga catggatccg ccgcctgcag       300
   aacttcaaga gcgcgacgac cggcgtcgcc tcttcggcga tgaagaatga gctcaaccgt       360
   cttgttgcga cggagaagaa cccgcagttg cagcaagcgc gtagcagact actgtccgat       420
   cgcaagtact gacccttcgc aggcgttcaa gcaggagatg gactcgttct tctacctctt       480
   cacgcgctac cttgacgaac gtgccaagag ccacgaactg tgcgtcttct tccgcttccc       540
   tcggacttgc tatctgatat ccctccaggg actgggataa gattcgctcc ccctccaaag       600
   agcaggtcgc atcctatgca gacctttccg ccccggccga caaggctgcc ctcaacaagc       660
   tcgccgtcct caaggtcaat ggtggtctgg gtacctcgat gggtgcgcga taacgttgag       720
   acgctcgaat ccgagatact gaactatgaa tgcaggcatg aagggcgcaa agagcgcact       780
   cgaggtcaag aatggcatga ccttcctgga ccttaccgtg cgccagatcg agcacctcaa       840
   cactagcctc cacgtcgatg tccccctcat cctcatgacc tccttcaaca cgcacgagga       900
   cacgctgcgc atcatcaaga agtaggtctc gtccccatag aatctcgtcc ctatagaagc       960
   ggtccaggaa cttaccgtac ttctcgtaga tacgccaacc agcaggtccg catcaccacg      1020
   ttcaaccagt cgcgctaccc gcgcatcatc gcggagacgc tgctgcccca gccgaagtcg      1080
   gtggacgacg acaagtcgaa gtggtacccg cccggtcacg gtgacctcta caatgcgctt      1140
   gtgcactccg gcgtcctcga ccagttgttg gctgacggga aggaatacct cttcgtctct 1200
   aactcggata acttgggcgc tgtgtaagtc tacaaggggt ggttgctacc atggatggaa 1260
   agctaacacg atacagcgtg gatgagcgca tcctgcagca catgatcgac acccaggcgg 1320
   agttcgtcat ggaggtgacg gacaagacga aggcggacgt gaagggtggt accctcatcg 1380
   actacgaggg ccagatgcgc ctcctcgaaa tcgctcaggt ccccaacgag cacgttgagg 1440
   acttcaagtc tggtaagctc tttactctca ttcgtatccc ttatctcacc tgtcccctaa 1500
   gtccgcaagt tcaagatctt caacacgaac aacctgtgga tcaacctgaa ggccctcaag 1560
   cgcatcatga gcaccgaggg catggagctc gacatcatcg tcaacccgaa ggtcgccgat 1620
   gacggccagg ccgtcctgca gctcgagacc gcgtctggtg cggctgtcaa gcacttcaag 1680
   aacgcccacg gcatcaacgt gccccggacg cgctttttgc ccgtcaagag ctgctcggac 1740
   ctgctgctca tcatgtcgga catctactcg ctcgagcatg ggcagctcgt catcaacgag 1800
   cagcgcatgt tcgagacgac gccggttatc aagttgggcg atcactttaa gaaggtgggt 1860
   tgactgtgat gaggctaaat gactggaagg ttgacgagcg cgggaatctg cagatcgcca 1920
   acttccagaa gcggttcaag aagatcccga acatcgtcga gcttgaccac ttgacggtga 1980
   cgggcgatgt gtacttcggg cgcaatgtga ccttgcgcgg cacagtaatt gtggtcgcca 2040
   acgagggcca gcgcatcgat attccggacg gatgtgtgct tgagaaccgt aagtcatccg 2100
   cattactaca gtggtcagta tgctcacgag tcccgcaggg ctactctccg gcaacctgac 2160
   catgacggta agcggccttc ggcgctcggc aggaggctgg tactgactat acctaatagg 2220
   aactataaag tgatgaggga agctgggcac gagatggact tcaagtagta actgcgtgct 2280
   gtacaaaaat tgaatgggtt ttatgcacgg tcgtatgttt g 2321
<210> 37
   <211> 503
   <212> PRT
   <213> Schizophyllum commune
<400> 37
   Met Ser Thr Leu Ala Thr Pro Ser Asn Ala Arg Gln Pro Arg Thr Arg
   1               5                   10                  15
   Gly Val Ser Ala Met Asn Phe Lys Ser Ala Thr Thr Gly Val Ala Ser
               20                  25                  30
   Ser Ala Met Lys Asn Glu Leu Asn Arg Leu Val Ala Thr Glu Lys Asn
           35                  40                  45
   Pro Gln Leu Gln Gln Ala Phe Lys Gln Glu Met Asp Ser Phe Phe Tyr
       50                  55                  60
   Leu Phe Thr Arg Tyr Leu Asp Glu Arg Ala Lys Ser His Glu Leu Asp
   65 70 75 80
   Trp Asp Lys Ile Arg Ser Pro Ser Lys Glu Gln Val Ala Ser Tyr Ala
   85 90 95
   Asp Leu Ser Ala Pro Ala Asp Lys Ala Ala Leu Asn Lys Leu Ala Val
   100 105 110
   Leu Lys Val Asn Gly Gly Leu Gly Thr Ser Met Gly Met Lys Gly Ala
   115 120 125
   Lys Ser Ala Leu Glu Val Lys Asn Gly Met Thr Phe Leu Asp Leu Thr
   130 135 140
   Val Arg Gln Ile Glu His Leu Asn Thr Ser Leu His Val Asp Val Pro
   145 150 155 160
   Leu Ile Leu Met Thr Ser Phe Asn Thr His Glu Asp Thr Leu Arg Ile
   165 170 175
   Ile Lys Lys Tyr Ala Asn Gln Gln Val Arg Ile Thr Thr Phe Asn Gln
   180 185 190
   Ser Arg Tyr Pro Arg Ile Ile Ala Glu Thr Leu Leu Pro Gln Pro Lys
   195 200 205
   Ser Val Asp Asp Asp Lys Ser Lys Trp Tyr Pro Pro Gly His Gly Asp
   210 215 220
   Leu Tyr Asn Ala Leu Val His Ser Gly Val Leu Asp Gln Leu Leu Ala
   225 230 235 240
   Asp Gly Lys Glu Tyr Leu Phe Val Ser Asn Ser Asp Asn Leu Gly Ala
   245 250 255
   Val Val Asp Glu Arg Ile Leu Gln His Met Ile Asp Thr Gln Ala Glu
   260 265 270
   Phe Val Met Glu Val Thr Asp Lys Thr Lys Ala Asp Val Lys Gly Gly
   275 280 285
   Thr Leu Ile Asp Tyr Glu Gly Gln Met Arg Leu Leu Glu Ile Ala Gln
   290 295 300
   Val Pro Asn Glu His Val Glu Asp Phe Lys Ser Val Arg Lys Phe Lys
   305 310 315 320
   Ile Phe Asn Thr Asn Asn Leu Trp Ile Asn Leu Lys Ala Leu Lys Arg
   325 330 335
   Ile Met Ser Thr Glu Gly Met Glu Leu Asp Ile Ile Val Asn Pro Lys
   340 345 350
   Val Ala Asp Asp Gly Gln Ala Val Leu Gln Leu Glu Thr Ala Ser Gly
   355 360 365
   Ala Ala Val Lys His Phe Lys Asn Ala His Gly Ile Asn Val Pro Arg
   370 375 380
   Thr Arg Phe Leu Pro Val Lys Ser Cys Ser Asp Leu Leu Leu Ile Met
   385 390 395 400
   Ser Asp Ile Tyr Ser Leu Glu His Gly Gln Leu Val Ile Asn Glu Gln
   405 410 415
   Arg Met Phe Glu Thr Thr Pro Val Ile Lys Leu Gly Asp His Phe Lys
   420 425 430
   Lys Ile Ala Asn Phe Gln Lys Arg Phe Lys Lys Ile Pro Asn Ile Val
   435 440 445
   Glu Leu Asp His Leu Thr Val Thr Gly Asp Val Tyr Phe Gly Arg Asn
   450 455 460
   Val Thr Leu Arg Gly Thr Val Ile Val Val Ala Asn Glu Gly Gln Arg
   465 470 475 480
   Ile Asp Ile Pro Asp Gly Cys Val Leu Glu Asn Arg Leu Leu Ser Gly
   485 490 495
   Asn Leu Thr Met Thr Glu Leu
   500
<210> 38
   <211> 29
   <212> DNA
   <213> artificial (primer Sto124)
<400> 38
   ctagactagt cttgccccca tctccccat 29
<210> 39
   <211> 34
   <212> DNA
   <213> artificial (primer Sto201)
<400> 39
   ccggaattcc aaacatacga ccgtgcataa aacc 34
<210> 40
   <211> 1958
   <212> DNA
   <213> Schizophyllum commune
<400> 40
   atggatatca tagacgaccg ctcactggtg tcccgggagt ttctcgccgt cattctttgt      60
   ggctttggca atgagtgagt ggggcgacag accgtagtgc acttgatcgc ccaagccaat     120
   gacgatcgca gattgacgcc gctaacgagt aacaacggcg atgagtcctg cccaaaagca     180
   ctgctccctg tcgcaaacaa ggcgatcctc gactacgttc tcgcttgggt agagcaagcg     240
   cgcatccaag gtgcatatat ctctgcgcgc gcctcaattt cctcgagcac atccctcatc     300
   cccctcagat gtccttgtta tttgccccac cgcgcaccgc actgcaatct cccaccacat     360
   tgactccgac atcagctctt cctccaccgg cctgcgcatc gatgtccaga ccttcgacga     420
   gtcgcaggac agtagcatcg gcaccggcac agtcctccgt cacttcgcca cccgcattca     480
   gtctgacttc atcctcctat catgcgactt catccccccg ccttcactcc ccctttccac     540
   cctgcttgcc aagttccgca cggaggcact cgcggacggc gcgatcgcca ctacgtgctg     600
   gttcgcgggt accaagaaga acgagaagga caagtcaacc acgcccgagg aatggggtcc     660
   cttccccgaa cccaccgtca tcgtgtggga cgagacgaca ggcacgctcc tgcacgtcga     720
   cacccccgac gcgatcgatg agaacgcgga cgagatggag gttcgcatgg ctctgctgga     780
   caggtatgtg gcgcgcgccg cgcaccgatc gtgtctcata attgactaga tacccccaca     840
   caaaactctc tgcatccttc acggactcgc acgtctacgt ctgcaagcgc tccgtcctcg     900
   acgcactagt tgcgaaaccg cttcttgagt ccttccgcga agacttcctt ccctggctat     960
   gcaaggtcca ataccagcgt gccaagcgcc gaaaatgggg ccaaagtgcg tatatcgact    1020
   ctccaacaat cttatccgtc gtattcatct cttcgcacct agctctacac cccagtacca    1080
   gtgcgctcac ccaagatctc gcgctgagcc actctaccct ccatatcaac ccaaatcagg    1140
   gcgacgacga cactgaggat tcggatatgc aagatactcc agccagctta cgtgtaggcg    1200
   ctgtccttta caagtcccca cgccccgaag aatatatcgc gagaattaac aacattcact    1260
   cgttcttcga ggctaatcgc caggtagaat gctcgatctt atatgcgaga ccatctgctg    1320
   attcacactg cgcagtgtct aacgacagcc acctggtcac ttcccaccga gcccaaagcc    1380
   cgctctctca tcgacgcgaa ggcgcaaatt tcgacagact caatcatcgg taagtcactc    1440
   atactgtatc atcccaaata tcccaaacct gatctctaca tcctacccct taggcacctc    1500
   tactcaggtc ggcgagcgtg ccaccataaa acgcagcact atcggaaagc actgcaagat    1560
   cggcaaaatg gccaaaatca ctagctgcgt tctgctcgac cattgttcgg tggatgatgg 1620
   gtatgaatgt ccattatata tgcggccgtc ttctcttatg gtcgccttcc ttcacagtgc 1680
   taagctagaa gggtgcattc tcggcaaaaa cacgaagatc ggcgcaaaag ctgatctctc 1740
   gaaatgtgta acgcaggctg gctacgaagt cgctgcagct ggtcggtcca cgtccctata 1800
   gcataccgta caattgctga tcttgatcag aaaccatcaa aaacgagaag cttgatgttt 1860
   ccgactggac cgcagtgtcc gacgacgaag acgaggggtt cggcggctca ggagacagca 1920
   gctcagacga cgactcgagt ggctcaagca gcgagtaa 1958
<210> 41
   <211> 505
   <212> PRT
   <213> Schizophyllum commune
<400> 41
   Met Asp Ile Ile Asp Asp Arg Ser Leu Val Ser Arg Glu Phe Leu Ala
   1               5                   10                  15
   Val Ile Leu Cys Gly Phe Gly Asn Glu Leu Thr Pro Leu Thr Ser Asn
               20                  25                  30
   Asn Gly Asp Glu Ser Cys Pro Lys Ala Leu Leu Pro Val Ala Asn Lys
           35                  40                  45
   Ala Ile Leu Asp Tyr Val Leu Ala Trp Val Glu Gln Ala Arg Ile Gln
       50                  55                  60
   Asp Val Leu Val Ile Cys Pro Thr Ala His Arg Thr Ala Ile Ser His
   65                  70                  75                  80
   His Ile Asp Ser Asp Ile Ser Ser Ser Ser Thr Gly Leu Arg Ile Asp
                   85                  90                  95
   Val Gln Thr Phe Asp Glu Ser Gln Asp Ser Ser Ile Gly Thr Gly Thr
               100                 105                 110
   Val Leu Arg His Phe Ala Thr Arg Ile Gln Ser Asp Phe Ile Leu Leu
           115                 120                 125
   Ser Cys Asp Phe Ile Pro Pro Pro Ser Leu Pro Leu Ser Thr Leu Leu
       130                 135                 140
   Ala Lys Phe Arg Thr Glu Ala Leu Ala Asp Gly Ala Ile Ala Thr Thr
   145                 150                 155                 160
   Cys Trp Phe Ala Gly Thr Lys Lys Asn Glu Lys Asp Lys Ser Thr Thr
                   165                 170                 175
   Pro Glu Glu Trp Gly Pro Phe Pro Glu Pro Thr Val Ile Val Trp Asp
               180                 185                 190
   Glu Thr Thr Gly Thr Leu Leu His Val Asp Thr Pro Asp Ala Ile Asp
           195                 200                 205
   Glu Asn Ala Asp Glu Met Glu Val Arg Met Ala Leu Leu Asp Arg Tyr
       210                 215                 220
   Pro His Thr Lys Leu Ser Ala Ser Phe Thr Asp Ser His Val Tyr Val
   225                 230                 235                 240
   Cys Lys Arg Ser Val Leu Asp Ala Leu Val Ala Lys Pro Leu Leu Glu
                   245                 250                 255
   Ser Phe Arg Glu Asp Phe Leu Pro Trp Leu Cys Lys Val Gln Tyr Gln
               260                 265                 270
   Arg Ala Lys Arg Arg Lys Trp Gly Gln Thr Leu His Pro Ser Thr Ser
           275                 280                 285
   Ala Leu Thr Gln Asp Leu Ala Leu Ser His Ser Thr Leu His Ile Asn
       290                 295                 300
   Pro Asn Gln Gly Asp Asp Asp Thr Glu Asp Ser Asp Met Gln Asp Thr
   305                 310                 315                 320
   Pro Ala Ser Leu Arg Val Gly Ala Val Leu Tyr Lys Ser Pro Arg Pro
                   325                 330                 335
   Glu Glu Tyr Ile Ala Arg Ile Asn Asn Ile His Ser Phe Phe Glu Ala
               340                 345                 350
   Asn Arg Gln Cys Leu Thr Thr Ala Thr Trp Ser Leu Pro Thr Glu Pro
           355                 360                 365
   Lys Ala Arg Ser Leu Ile Asp Ala Lys Ala Gln Ile Ser Thr Asp Ser
       370                 375                 380
   Ile Ile Gly Thr Ser Thr Gln Val Gly Glu Arg Ala Thr Ile Lys Arg
   385                 390                 395                 400
   Ser Thr Ile Gly Lys His Cys Lys Ile Gly Lys Met Ala Lys Ile Thr
                   405                 410                 415
   Ser Cys Val Leu Leu Asp His Cys Ser Val Asp Asp Gly Ala Lys Leu
               420                 425                 430
   Glu Gly Cys Ile Leu Gly Lys Asn Thr Lys Ile Gly Ala Lys Ala Asp
           435                 440                 445
   Leu Ser Lys Cys Val Thr Gln Ala Gly Tyr Glu Val Ala Ala Ala Glu
       450                 455                 460
   Thr Ile Lys Asn Glu Lys Leu Asp Val Ser Asp Trp Thr Ala Val Ser
   465                 470                 475                 480
   Asp Asp Glu Asp Glu Gly Phe Gly Gly Ser Gly Asp Ser Ser Ser Asp
                   485                 490                 495
   Asp Asp Ser Ser Gly Ser Ser Ser Glu
               500                 505
<210> 42
   <211> 36
   <212> DNA
   <213> artificial (primer Sto781)
<400> 42
   ataagaatgc ggccgcatgg gcatcataga cgaccg 36
<210> 43
   <211> 29
   <212> DNA
   <213> artificial (primer Sto782)
<400> 43
   cgcggatcct tactcgctgc ttgagccac 29
<210> 44
   <211> 2715
   <212> DNA
   <213> Schizophyllum commune
<400> 44
   atgcccgtgt cgcgcagagg atccggctcc gtgacgctcc cttcggggca gagcatcacc      60
   atccaccacc gcactaccga ggagcccggc gaggacctcc cgcacgcgac gaagaagacg     120
   atggcggacc acctcaggaa gttcgagagc ctcttcacgc tgacgccgca gcgcatgcgg     180
   atgatcgtcg atgcattcaa ggtgcggagg gggtagcttt gcttagggga acagcggcgg     240
   actgacgatc gcgcgttagg acaccctcga tcttggattg aagaagcccg ggcaggtcgt     300
   gcccatgttc ccgacctacg tcttcggctg gccgacggga gatgtgcgtg gcgcgatgcg     360
   agcctttgat gactgctgac cctccccagg aaaccggcga cttcctcgcg ctcgatctcg     420
   ggggcaccaa cttgcgcgtg tgccttgtca ccctcaaggg gaacggccag ttcgagatga     480
   cgcagtccaa gtaccgtctc actgaggagc agaagcaagg ggatggacag gcactcttcg 540
   acttctgcgc cgagtgcttg aagacgttca ttgaggggaa cttcgatgag gaagaacgcc 600
   agaagggtct acaccttccc ctgggcttca cgttcagcta tccatgcatc caagtgtgtc 660
   ttccaataca tacgctgctc aagcgctcta ctggaccacg tactaatgtc tcgaatagga 720
   gcgcatcgat cacggagtgt tggtacgatg gacgaaggtg cgttatcatc aggacctttg 780
   aactcaaagc taacggacgt cactcagggc ttcggcgctg ctaacaccga gggcaacgat 840
   gtcgctgcca tgtttaagga gagcttgaag aaatacgtgc gcacccacct ccgttcgcat 900
   tcccgacaat catctcatga accttcagga cctccccatc agcttgaccg ctctcatcaa 960
   cgatacgaca ggaacgctca tcgcgagtca ctacgtcaac ccgtcaaggt ccgcattaac 1020
   ccgctatcgc attcatactt ccctcccttt ctgacgtatc ctcagcaaga tcgccgtcat 1080
   cttcggtact ggttgcaacg cggcgtacat ggagagaatc gggaacattg agaagctcaa 1140
   ggtaggtatc taccttcaag ctcatacgac catcgcagtt cctcctccat acctgaccgg 1200
   cataggctct tccctacgtc atccctcttt gggggtactg cgatggtggg cttactaacg 1260
   ttgcttcgca ggacttgggc ttgcctgccg acgcgcaaat ggcgatcaac tgcgagtggg 1320
   gtgcgttcga cagcttcgtg cacgagcacc taccgaggac aaagtgagta ccgaatttcg 1380
   actctggtgg gttcgtcttg cagggtaaag cactccgtgc acgagcgagg gctggaggcc 1440
   cgctatgacc taagtgctac cgcgaaatcg catgcgcttc gcgctatcgc tttatgtctc 1500
   gggacgtcga tgaacgtctt gcggtagtgc ttcatcacat tcatcgtctg tgccctgaga 1560
   tgcgcgtctg tatgtggcgc tcctcgtatt tcttccacct cagtcatggg ataacctcgc 1620
   atgaccttct atgtcatctc atctcactcg ctatgcatgt ttaggacaac gcactgacct 1680
   cgcccaggta cgacatcatc gtcgacgaga ccagcaacaa gccgcacgag caggcgttcg 1740
   aggtacgcgc atcccatgcc gtgccacaga acgtagattt acgcgtctat ctactgacct 1800
   cctcctttac ttcgcacaga aactcatctc gggccgctac ctcggcgaga tcatgcgcct 1860
   ggttatctgc gagctcattg atgatggcgt attgttcctc gggcaggaga cgtgagtact 1920
   tgtcgagggc gcagttgcgg aatgaggtaa ctgacgacgc acgataggta caagttggag 1980
   tccccgtacg tcttcgagac ggcgttcttg agtttgatgg agaggtgtgt ttcttcttcg 2040
   acatcgcctt tcaaaagtga cttacgctcc gtgcagtgac cctacggacg agctcctgat 2100
   gatcattggc atcttctcgc acttcttcgc cgtcgagacg acactggctg agcgccaatt 2160
   cttccgcgca ttggcgaagc tgattggtaa gttttcgttc tctcgccatc cactcgctgg 2220
   gaccttcact tgtcttacca ttgctgatcc gtccggcagg ccgtcgcgct gcgcgtcttt 2280
   ctgcctgcgg tatcgcctcc atcgtcagca agatgggcta cctggacgag ctcaaggagg 2340
   gcgagatcat cggcgtcggc tcggacggta gcttgtataa cgtgcgttga ggtttcgctg     2400
   cttcagttca tggcctgcat ctcaactatc atctagaaat accctgcgtt cgccgagcgc     2460
   gtccacgaag gcctcgtcga tatcttcggc gagaagggga agtaagtgcc atctttccgc     2520
   tcgggaagta gacgtggatg ctgactagtc ccttaggcag gtcgtcaccc accacgccga     2580
   ggatggaagt ggtgtgggca gcgcgatcat cgctggtgag tctttcgcct tgcgccttgc     2640
   caacatcgta tactgatgct cgcaccagcc atgaccaaga tccgcaagga caagggcatc     2700
   ttcgccgacg tctaa                                                      2715
<210> 45
   <211> 503
   <212> PRT
   <213> Schizophyllum commune
<400> 45
   Met Pro Val Ser Arg Arg Gly Ser Gly Ser Val Thr Leu Pro Ser Gly
   1               5                   10                  15
   Gln Ser Ile Thr Ile His His Arg Thr Thr Glu Glu Pro Gly Glu Asp
               20                  25                  30
   Leu Pro His Ala Thr Lys Lys Thr Met Ala Asp His Leu Arg Lys Phe
           35                  40                  45
   Glu Ser Leu Phe Thr Leu Thr Pro Gln Arg Met Arg Met Ile Val Asp
       50                  55                  60
   Ala Phe Lys Asp Thr Leu Asp Leu Gly Leu Lys Lys Pro Gly Gln Val
   65                  70                  75                  80
   Val Pro Met Phe Pro Thr Tyr Val Phe Gly Trp Pro Thr Gly Asp Glu
                   85                  90                  95
   Thr Gly Asp Phe Leu Ala Leu Asp Leu Gly Gly Thr Asn Leu Arg Val
               100                 105                 110
   Cys Leu Val Thr Leu Lys Gly Asn Gly Gln Phe Glu Met Thr Gln Ser
           115                 120                 125
   Lys Tyr Arg Leu Thr Glu Glu Gln Lys Gln Gly Asp Gly Gln Ala Leu
       130                 135                 140
   Phe Asp Phe Cys Ala Glu Cys Leu Lys Thr Phe Ile Glu Gly Asn Phe
   145                 150                 155                 160
   Asp Glu Glu Glu Arg Gln Lys Gly Leu His Leu Pro Leu Gly Phe Thr
   165 170 175
   Phe Ser Tyr Pro Cys Ile Gln Glu Arg Ile Asp His Gly Val Leu Val
   180 185 190
   Arg Trp Thr Lys Gly Phe Gly Ala Ala Asn Thr Glu Gly Asn Asp Val
   195 200 205
   Ala Ala Met Phe Lys Glu Ser Leu Lys Lys Tyr Asp Leu Pro Ile Ser
   210 215 220
   Leu Thr Ala Leu Ile Asn Asp Thr Thr Gly Thr Leu Ile Ala Ser His
   225 230 235 240
   Tyr Val Asn Pro Ser Ser Lys Ile Ala Val Ile Phe Gly Thr Gly Cys
   245 250 255
   Asn Ala Ala Tyr Met Glu Arg Ile Gly Asn Ile Glu Lys Leu Lys Asp
   260 265 270
   Leu Gly Leu Pro Ala Asp Ala Gln Met Ala Ile Asn Cys Glu Trp Gly
   275 280 285
   Ala Phe Asp Ser Phe Val His Glu His Leu Pro Arg Thr Lys Tyr Asp
   290 295 300
   Ile Ile Val Asp Glu Thr Ser Asn Lys Pro His Glu Gln Ala Phe Glu
   305 310 315 320
   Lys Leu Ile Ser Gly Arg Tyr Leu Gly Glu Ile Met Arg Leu Val Ile
   325 330 335
   Cys Glu Leu Ile Asp Asp Gly Val Leu Phe Leu Gly Gln Glu Thr Tyr
   340 345 350
   Lys Leu Glu Ser Pro Tyr Val Phe Glu Thr Ala Phe Leu Ser Leu Met
   355 360 365
   Glu Ser Asp Pro Thr Asp Glu Leu Leu Met Ile Ile Gly Ile Phe Ser
   370 375 380
   His Phe Phe Ala Val Glu Thr Thr Leu Ala Glu Arg Gln Phe Phe Arg
   385 390 395 400
   Ala Leu Ala Lys Leu Ile Gly Arg Arg Ala Ala Arg Leu Ser Ala Cys
   405 410 415
   Gly Ile Ala Ser Ile Val Ser Lys Met Gly Tyr Leu Asp Glu Leu Lys
   420 425 430
   Glu Gly Glu Ile Ile Gly Val Gly Ser Asp Gly Ser Leu Tyr Asn Lys
   435 440 445
   Tyr Pro Ala Phe Ala Glu Arg Val His Glu Gly Leu Val Asp Ile Phe
   450 455 460
   Gly Glu Lys Gly Lys Gln Val Val Thr His His Ala Glu Asp Gly Ser
   465 470 475 480
   Gly Val Gly Ser Ala Ile Ile Ala Ala Met Thr Lys Ile Arg Lys Asp
   485 490 495
   Lys Gly Ile Phe Ala Asp Val
   500
<210> 46
   <211> 5223
   <212> DNA
   <213> Schizophyllum commune
<400> 46
   atgtccggtc caggatatgg caggaatcca ttcgacaatc ccccgcccaa cagaggtccc 60
   tatggccagc agccaggttt cccggggccc ggccctcggc cttacgactc ggacgcggac 120
   atgagccaga cctatggcag cacaaccagg ctcgccggca gtgccggtta cagcgacaga 180
   aacggcagct tcgacggcga ccgctcctac gcgccctcaa ttgactcgcg cgccagcgtg 240
   cccagcatat cgcccttcgc agacccgggt atcggctcta atgagccgta tcccgcttgg 300
   tcggtcgaac gccagatccc catgtccacg gaggagattg aggatatctt cctcgacctc 360
   acccaaaagt ttggcttcca gcgcgactcc atgcggaata cgttcgactt catgatgcac 420
   ctccttgatt cccgtgcctc gcgcatgacg cccaaccaag ctctgctcac gcttcacgcc 480
   gactacattg gtggccagca cgccaactat aggaagtggt atttcgccgc tcagctcaac 540
   ctcgatgacg cggtcgggca aaccaataac cccggtatcc agcgcttgaa gaccatcaag 600
   ggcgctacga agaccaagtc gctcgacagc gcactcaacc gctggcgcaa tgcgatgaac 660
   aacatgagcc agtacgatcg cctccggcaa attgcgctct atctcctctg ctggggagaa 720
   gcaggcaaca tccgtctggc gcccgagtgc ttgtgcttca tcttcaagtg cgcggacgac 780
   tactacagaa gtcccgagtg tcagaaccgg atggaccccg tgccggaagg gctgtacctc 840
   cagacggtca tcaagccgct ctatcgcttc ctacgtgatc aggcgtacga agtcgttgat 900
   gggaagcaag tgaagcgcga gaaggaccac gaccagatta tcggttatga cgacgtcaac 960
   cagttattct ggtatccgga aggtttggct aagatcgtca tgtcggacaa cacacgactt 1020
   gtagatgtac ctccggcgca gcggttcatg aagttcgcca agatcgagtg gaaccgcgtc 1080
   ttcttcaaga cgtactttga gaagcgctct actgcccatc tcctggtcaa cttcaaccgt 1140
   atatggatcc tccacgtctc gatgtacttc ttctacacgg cattcaactc tccacgagtc 1200
   tacgcgccgc acggcaaact cgacccctcc cctgagatga cctggtccgc gactgccctt 1260
   ggaggcgctg tgtccaccat gatcatgatc cttgccacta tcgcggagta cacctacatc 1320
   cccacgacat ggaacaatgc gtcgcacctc accacgcggc tcattttcct cctggtcatc 1380
   ctcgcgctca ctgctggacc aacattctat atcgccatga tagacggacg cacggacatc 1440
   ggccaagtac cactcatcgt ggccatagtg cagttcttca tctccgtcgt cgccaccctc 1500
   gctttcgcta ccatcccttc tggtcgcatg ttcggcgacc gtgtggctgg caagtcaaga 1560
   aagcacatgg catcgcagac gttcacagcg tcgtacccgt ccatgaagcg gtcatctcgc 1620
   gtagcgagta tcatgctgtg gcttttggtc tttggctgca aatacgtcga gtcttacttc 1680
   ttcttgacgt cctccttctc cagcccgatc gcggtcatgg cgcgtacgaa ggtacagggc 1740
   tgcaacgacc gtatcttcgg cagccagctg tgcacgaatc aggtcccgtt cgcgctggca 1800
   atcatgtacg tgatggacct ggtactgttc ttcctggaca cgtacctgtg gtacatcatc 1860
   tggctggtga tcttctcgat ggtgcgcgcg ttcaagcttg gtatctcgat ctggacgccc 1920
   tggagcgaga tcttcacccg catgccgaag cgtatctacg cgaagctgct ggcgacggcc 1980
   gagatggagg tcaagtataa gcccaaggtg ctcgtctcgc aaatctggaa cgcggtcatc 2040
   atctccatgt accgggagca tctcttgtcc atcgagcacg tccagcgcct gctataccac 2100
   caggttgatg gtccagacgg tcgccgcacc ctcagggcac cgccgttctt caccagccag 2160
   cgaactgcga agccaggcct gttcttccct cctggtggcg aggctgagcg ccgtatctcg 2220
   ttctttgcct catcgctgac gaccgcgctc cctgagcctc tgccgatcga cgccatgccc 2280
   accttcaccg tgctcgttcc ccattactcg gagaagattc tgctcagtct gcgcgagatt 2340
   attcgcgagg aggaccagaa cacccgcgtc accttgctgg agtacctcaa gcagctccac 2400
   cctgtcgaat gggacaactt cgtcaaggac accaagatct tggcggaaga gtcgggcgac 2460
   gtccaggacg agaagcgcgc gcgcacggac gacttgccgt tctactgcat cgggttcaag 2520
   acctcgtcac cagagtacac cctgcgtacg cgtatctggg cttcactgcg cgcacagacg 2580
   ctgtaccgca cggtctccgg tatgatgaac tactccaagg cgatcaagct cctctatcgc 2640
   gtcgagaacc cggatgtcgt tcatgccttc ggtgggaaca cggaacgtct tgaacgcgag 2700
   cttgagcgca tgtctcgccg caagttcaag ttcgtcatct cgatgcagcg gtactctaag 2760
   ttcaacaagg aggagcaaga gaacgccgaa ttccttctgc gcgcgtaccc ggatttgcag 2820
   atcgcgtacc tcgatgaaga gcccggtccc agcaagagcg acgaggttcg gttgttttcg 2880
   acactcatcg atggacactc cgaggtggat gagaagaccg gccgccgcaa gcccaagttc 2940
   cgcattgagc tgcccggtaa ccccatcctc ggtgacggga agtcggataa ccagaaccac 3000
   gccattgtct tctaccgcgg cgagtacatc caggtcatcg acgctaacca ggacaattac 3060
   ctggaagagt gtctcaagat ccgtaacgtc ctgggcgagt ttgaggaata ctccgtgtcg 3120
   agccagagcc cgtacgcaca gtggggccac aaggagttca acaagtgccc cgtcgctatc 3180
   ctgggttctc gcgagtacat cttctcggag aacatcggta tcctcggtga catcgccgcc 3240
   ggcaaggaac agacgttcgg taccattacg gcgcgtgcgc ttgcgtggat cggcggcaag 3300
   ctgcattacg gtcacccgga tttcctcaat gcgacgttca tgacgacgcg tggtggcgtg 3360
   tcaaaagcgc agaagggctt gcatctcaac gaggatatct tcgctggtat gaccgccgtg 3420
   tcccgcggag ggcgcatcaa gcacatggag tactaccagt gcggcaaagg tcgtgatctc 3480
   ggtttcggca cgatcttgaa cttccagacg aagatcggta ctggtatggg cgagcagctc 3540
   ctctcgcgcg agtactacta cctgggcacg caattgccta tcgaccggtt cttgacgttc 3600
   tactacgcgc acgctggttt ccacgtcaac aacatcctgg tcatctactc catccaggtc 3660
   ttcatggtca ccttgctgta cctgggcaca ttgaacaagc agctgttcat ctgcaaggtc 3720
   aactccaatg gccaggttct tagtggacaa gctgggtgct acaacctcat cccggtcttc 3780
   gagtggattc gccggagtat catctccatc ttcttggtgt tcttcatcgc cttcttgcct 3840
   ctattcttgc aagagctgtg cgagcgcgga acgggaaagg cgttgctgcg tctcgggaag 3900
   cacttcttgt cactgtcgcc cattttcgaa gtgttctcca cccagattta ctcgcaggcg 3960
   ctcttgaaca acatgagctt cggtggtgcg cgctacatcg ccacaggtcg tggtttcgcg 4020
   actagtcgca tacccttcaa catcctctac tcgcgtttcg cgccgccaag catctacatg 4080
   ggcatgcgta acctgctgct cctgctgtac gcgacgatgg ccatttggat cccgcacctg 4140
   atctacttct ggttctccgt cctctccctc tgcatcgcgc cattcatgtt caatccgcat 4200
   caattctcgt acgccgactt catcatcgac taccgggagt tcttgcgctg gatgtcgcgc 4260
   ggtaactcgc gaacgaaggc gagcagctgg tacggatact gccgtctgtc gcgtaccgcg 4320
   attactgggt acaagaagaa gaagctggga cacccgtcgg agaagctgtc gggcgacgta 4380
   ccgcgtgcgc cgtggaggaa cgttatcttc tcggagatcc tgtggcccat cggcgcgtgc 4440
   atcatcttca tcgtcgcgta catgttcgtc aagtcgttcc ccgacgagca gggcaacgcg 4500
   ccgccgagcc cgctggtccg gattctgctc atcgcggttg gccctactgt gtggaacgcg 4560
   gcggtgctca taacgctgtt cttcctgtcg ctcttcctgg gcccgatgat ggatggctgg 4620
   gtcaagttcg gctcggtcat ggcggccctt gcgcatggcc tggcgcttat aggcatgctc 4680
   acgttctttg agttcttctg gttccttgag ctctgggatg cctcgcacgc cgtgctcggc 4740
   gtcatcgcta tcattgccgt tcagcgcggg atccagaaga tcctcattgc cgtcttcctg 4800
   acgcgtgagt acaagcacga cgagacgaac cgcgcgtggt ggacaggtaa atggtatgga 4860
   cgcgggctgg gtacctcggc catgtcccag ccggcgcgcg agttcatcgt gaagatcgtg 4920
   gagatgtcgt tgtggacgtc ggacttcctg cttgcgcacc tgttgctcat catcttgacg 4980
   gtgccgctac tgctgccgtt cttcaactca attcattcga cgatgctttt ctggttgcgc 5040
   ccttcgaagc agattaggca acctctgttc tccaccaagc agaagcggca acggcgatgg 5100
   attgtcatga agtataccgt ggtatatctc gtggtggtgg ctttcctcgt cgcgctcatc 5160
   gctctgcccg ccctcttccg cgagagcatc cacttcaact gcgagatctg ccagagtata 5220
   tag 5223
<210> 47
   <211> 1740
   <212> PRT
   <213> Schizophyllum commune
<400> 47
   Met Ser Gly Pro Gly Tyr Gly Arg Asn Pro Phe Asp Asn Pro Pro Pro
   1               5                   10                  15
   Asn Arg Gly Pro Tyr Gly Gln Gln Pro Gly Phe Pro Gly Pro Gly Pro
               20                  25                  30
   Arg Pro Tyr Asp Ser Asp Ala Asp Met Ser Gln Thr Tyr Gly Ser Thr
           35                  40                  45
   Thr Arg Leu Ala Gly Ser Ala Gly Tyr Ser Asp Arg Asn Gly Ser Phe
       50                  55                  60
   Asp Gly Asp Arg Ser Tyr Ala Pro Ser Ile Asp Ser Arg Ala Ser Val
   65                  70                  75                  80
   Pro Ser Ile Ser Pro Phe Ala Asp Pro Gly Ile Gly Ser Asn Glu Pro
                   85                  90                  95
   Tyr Pro Ala Trp Ser Val Glu Arg Gln Ile Pro Met Ser Thr Glu Glu
               100                 105                 110
   Ile Glu Asp Ile Phe Leu Asp Leu Thr Gln Lys Phe Gly Phe Gln Arg
           115                 120                 125
   Asp Ser Met Arg Asn Thr Phe Asp Phe Met Met His Leu Leu Asp Ser
       130                 135                 140
   Arg Ala Ser Arg Met Thr Pro Asn Gln Ala Leu Leu Thr Leu His Ala
   145                 150                 155                 160
   Asp Tyr Ile Gly Gly Gln His Ala Asn Tyr Arg Lys Trp Tyr Phe Ala
                   165                 170                 175
   Ala Gln Leu Asn Leu Asp Asp Ala Val Gly Gln Thr Asn Asn Pro Gly
               180                 185                 190
   Ile Gln Arg Leu Lys Thr Ile Lys Gly Ala Thr Lys Thr Lys Ser Leu
           195                 200                 205
   Asp Ser Ala Leu Asn Arg Trp Arg Asn Ala Met Asn Asn Met Ser Gln
       210                 215                 220
   Tyr Asp Arg Leu Arg Gln Ile Ala Leu Tyr Leu Leu Cys Trp Gly Glu
   225                 230                 235                 240
   Ala Gly Asn Ile Arg Leu Ala Pro Glu Cys Leu Cys Phe Ile Phe Lys
                   245                 250                 255
   Cys Ala Asp Asp Tyr Tyr Arg Ser Pro Glu Cys Gln Asn Arg Met Asp
               260                 265                 270
   Pro Val Pro Glu Gly Leu Tyr Leu Gln Thr Val Ile Lys Pro Leu Tyr
           275                 280                 285
   Arg Phe Leu Arg Asp Gln Ala Tyr Glu Val Val Asp Gly Lys Gln Val
       290                 295                 300
   Lys Arg Glu Lys Asp His Asp Gln Ile Ile Gly Tyr Asp Asp Val Asn
   305                 310                 315                 320
   Gln Leu Phe Trp Tyr Pro Glu Gly Leu Ala Lys Ile Val Met Ser Asp
                   325                 330                 335
   Asn Thr Arg Leu Val Asp Val Pro Pro Ala Gln Arg Phe Met Lys Phe
               340                 345                 350
   Ala Lys Ile Glu Trp Asn Arg Val Phe Phe Lys Thr Tyr Phe Glu Lys
           355                 360                 365
   Arg Ser Thr Ala His Leu Leu Val Asn Phe Asn Arg Ile Trp Ile Leu
       370                 375                 380
   His Val Ser Met Tyr Phe Phe Tyr Thr Ala Phe Asn Ser Pro Arg Val
   385                 390                 395                 400
   Tyr Ala Pro His Gly Lys Leu Asp Pro Ser Pro Glu Met Thr Trp Ser
                   405                 410                 415
   Ala Thr Ala Leu Gly Gly Ala Val Ser Thr Met Ile Met Ile Leu Ala
               420                 425                 430
   Thr Ile Ala Glu Tyr Thr Tyr Ile Pro Thr Thr Trp Asn Asn Ala Ser
           435                 440                 445
   His Leu Thr Thr Arg Leu Ile Phe Leu Leu Val Ile Leu Ala Leu Thr
       450                 455                 460
   Ala Gly Pro Thr Phe Tyr Ile Ala Met Ile Asp Gly Arg Thr Asp Ile
   465                 470                 475                 480
   Gly Gln Val Pro Leu Ile Val Ala Ile Val Gln Phe Phe Ile Ser Val
                   485                 490                 495
   Val Ala Thr Leu Ala Phe Ala Thr Ile Pro Ser Gly Arg Met Phe Gly
               500                 505                 510
   Asp Arg Val Ala Gly Lys Ser Arg Lys His Met Ala Ser Gln Thr Phe
           515                 520                 525
   Thr Ala Ser Tyr Pro Ser Met Lys Arg Ser Ser Arg Val Ala Ser Ile
       530                 535                 540
   Met Leu Trp Leu Leu Val Phe Gly Cys Lys Tyr Val Glu Ser Tyr Phe
   545                 550                 555                 560
   Phe Leu Thr Ser Ser Phe Ser Ser Pro Ile Ala Val Met Ala Arg Thr
                   565                 570                 575
   Lys Val Gln Gly Cys Asn Asp Arg Ile Phe Gly Ser Gln Leu Cys Thr
               580                 585                 590
   Asn Gln Val Pro Phe Ala Leu Ala Ile Met Tyr Val Met Asp Leu Val
           595                 600                 605
   Leu Phe Phe Leu Asp Thr Tyr Leu Trp Tyr Ile Ile Trp Leu Val Ile
       610                 615                 620
   Phe Ser Met Val Arg Ala Phe Lys Leu Gly Ile Ser Ile Trp Thr Pro
   625                 630                 635                 640
   Trp Ser Glu Ile Phe Thr Arg Met Pro Lys Arg Ile Tyr Ala Lys Leu
                   645                 650                 655
   Leu Ala Thr Ala Glu Met Glu Val Lys Tyr Lys Pro Lys Val Leu Val
               660                 665                 670
   Ser Gln Ile Trp Asn Ala Val Ile Ile Ser Met Tyr Arg Glu His Leu
           675                 680                 685
   Leu Ser Ile Glu His Val Gln Arg Leu Leu Tyr His Gln Val Asp Gly
       690                 695                 700
   Pro Asp Gly Arg Arg Thr Leu Arg Ala Pro Pro Phe Phe Thr Ser Gln
   705                 710                 715                 720
   Arg Thr Ala Lys Pro Gly Leu Phe Phe Pro Pro Gly Gly Glu Ala Glu
                   725                 730                 735
   Arg Arg Ile Ser Phe Phe Ala Ser Ser Leu Thr Thr Ala Leu Pro Glu
               740                 745                 750
   Pro Leu Pro Ile Asp Ala Met Pro Thr Phe Thr Val Leu Val Pro His
           755                 760                 765
   Tyr Ser Glu Lys Ile Leu Leu Ser Leu Arg Glu Ile Ile Arg Glu Glu
       770                 775                 780
   Asp Gln Asn Thr Arg Val Thr Leu Leu Glu Tyr Leu Lys Gln Leu His
   785                 790                 795                 800
   Pro Val Glu Trp Asp Asn Phe Val Lys Asp Thr Lys Ile Leu Ala Glu
                   805                 810                 815
   Glu Ser Gly Asp Val Gln Asp Glu Lys Arg Ala Arg Thr Asp Asp Leu
               820                 825                 830
   Pro Phe Tyr Cys Ile Gly Phe Lys Thr Ser Ser Pro Glu Tyr Thr Leu
           835                 840                 845
   Arg Thr Arg Ile Trp Ala Ser Leu Arg Ala Gln Thr Leu Tyr Arg Thr
       850                 855                 860
   Val Ser Gly Met Met Asn Tyr Ser Lys Ala Ile Lys Leu Leu Tyr Arg
   865                 870                 875                 880
   Val Glu Asn Pro Asp Val Val His Ala Phe Gly Gly Asn Thr Glu Arg
                   885                 890                 895
   Leu Glu Arg Glu Leu Glu Arg Met Ser Arg Arg Lys Phe Lys Phe Val
               900                 905                 910
   Ile Ser Met Gln Arg Tyr Ser Lys Phe Asn Lys Glu Glu Gln Glu Asn
   915 920 925
   Ala Glu Phe Leu Leu Arg Ala Tyr Pro Asp Leu Gln Ile Ala Tyr Leu
   930 935 940
   Asp Glu Glu Pro Gly Pro Ser Lys Ser Asp Glu Val Arg Leu Phe Ser
   945 950 955 960
   Thr Leu Ile Asp Gly His Ser Glu Val Asp Glu Lys Thr Gly Arg Arg
   965 970 975
   Lys Pro Lys Phe Arg Ile Glu Leu Pro Gly Asn Pro Ile Leu Gly Asp
   980 985 990
   Gly Lys Ser Asp Asn Gln Asn His Ala Ile Val Phe Tyr Arg Gly Glu
   995 1000 1005
   Tyr Ile Gln Val Ile Asp Ala Asn Gln Asp Asn Tyr Leu Glu Glu
   1010 1015 1020
   Cys Leu Lys Ile Arg Asn Val Leu Gly Glu Phe Glu Glu Tyr Ser
   1025 1030 1035
   Val Ser Ser Gln Ser Pro Tyr Ala Gln Trp Gly His Lys Glu Phe
   1040 1045 1050
   Asn Lys Cys Pro Val Ala Ile Leu Gly Ser Arg Glu Tyr Ile Phe
   1055 1060 1065
   Ser Glu Asn Ile Gly Ile Leu Gly Asp Ile Ala Ala Gly Lys Glu
   1070 1075 1080
   Gln Thr Phe Gly Thr Ile Thr Ala Arg Ala Leu Ala Trp Ile Gly
   1085 1090 1095
   Gly Lys Leu His Tyr Gly His Pro Asp Phe Leu Asn Ala Thr Phe
   1100 1105 1110
   Met Thr Thr Arg Gly Gly Val Ser Lys Ala Gln Lys Gly Leu His
   1115 1120 1125
   Leu Asn Glu Asp Ile Phe Ala Gly Met Thr Ala Val Ser Arg Gly
   1130 1135 1140
   Gly Arg Ile Lys His Met Glu Tyr Tyr Gln Cys Gly Lys Gly Arg
   1145 1150 1155
   Asp Leu Gly Phe Gly Thr Ile Leu Asn Phe Gln Thr Lys Ile Gly
       1160                1165                1170
   Thr Gly Met Gly Glu Gln Leu Leu Ser Arg Glu Tyr Tyr Tyr Leu
       1175                1180                1185
   Gly Thr Gln Leu Pro Ile Asp Arg Phe Leu Thr Phe Tyr Tyr Ala
       1190                1195                1200
   His Ala Gly Phe His Val Asn Asn Ile Leu Val Ile Tyr Ser Ile
       1205                1210                1215
   Gln Val Phe Met Val Thr Leu Leu Tyr Leu Gly Thr Leu Asn Lys
       1220                1225                1230
   Gln Leu Phe Ile Cys Lys Val Asn Ser Asn Gly Gln Val Leu Ser
       1235                1240                1245
   Gly Gln Ala Gly Cys Tyr Asn Leu Ile Pro Val Phe Glu Trp Ile
       1250                1255                1260
   Arg Arg Ser Ile Ile Ser Ile Phe Leu Val Phe Phe Ile Ala Phe
       1265                1270                1275
   Leu Pro Leu Phe Leu Gln Glu Leu Cys Glu Arg Gly Thr Gly Lys
       1280                1285                1290
   Ala Leu Leu Arg Leu Gly Lys His Phe Leu Ser Leu Ser Pro Ile
       1295                1300                1305
   Phe Glu Val Phe Ser Thr Gln Ile Tyr Ser Gln Ala Leu Leu Asn
       1310                1315                1320
   Asn Met Ser Phe Gly Gly Ala Arg Tyr Ile Ala Thr Gly Arg Gly
       1325                1330                1335
   Phe Ala Thr Ser Arg Ile Pro Phe Asn Ile Leu Tyr Ser Arg Phe
       1340                1345                1350
   Ala Pro Pro Ser Ile Tyr Met Gly Met Arg Asn Leu Leu Leu Leu
       1355                1360                1365
   Leu Tyr Ala Thr Met Ala Ile Trp Ile Pro His Leu Ile Tyr Phe
       1370                1375                1380
   Trp Phe Ser Val Leu Ser Leu Cys Ile Ala Pro Phe Met Phe Asn
   1385 1390 1395
   Pro His Gln Phe Ser Tyr Ala Asp Phe Ile Ile Asp Tyr Arg Glu
   1400 1405 1410
   Phe Leu Arg Trp Met Ser Arg Gly Asn Ser Arg Thr Lys Ala Ser
   1415 1420 1425
   Ser Trp Tyr Gly Tyr Cys Arg Leu Ser Arg Thr Ala Ile Thr Gly
   1430 1435 1440
   Tyr Lys Lys Lys Lys Leu Gly His Pro Ser Glu Lys Leu Ser Gly
   1445 1450 1455
   Asp Val Pro Arg Ala Pro Trp Arg Asn Val Ile Phe Ser Glu Ile
   1460 1465 1470
   Leu Trp Pro Ile Gly Ala Cys Ile Ile Phe Ile Val Ala Tyr Met
   1475 1480 1485
   Phe Val Lys Ser Phe Pro Asp Glu Gln Gly Asn Ala Pro Pro Ser
   1490 1495 1500
   Pro Leu Val Arg Ile Leu Leu Ile Ala Val Gly Pro Thr Val Trp
   1505 1510 1515
   Asn Ala Ala Val Leu Ile Thr Leu Phe Phe Leu Ser Leu Phe Leu
   1520 1525 1530
   Gly Pro Met Met Asp Gly Trp Val Lys Phe Gly Ser Val Met Ala
   1535 1540 1545
   Ala Leu Ala His Gly Leu Ala Leu Ile Gly Met Leu Thr Phe Phe
   1550 1555 1560
   Glu Phe Phe Trp Phe Leu Glu Leu Trp Asp Ala Ser His Ala Val
   1565 1570 1575
   Leu Gly Val Ile Ala Ile Ile Ala Val Gln Arg Gly Ile Gln Lys
   1580 1585 1590
   Ile Leu Ile Ala Val Phe Leu Thr Arg Glu Tyr Lys His Asp Glu
   1595 1600 1605
   Thr Asn Arg Ala Trp Trp Thr Gly Lys Trp Tyr Gly Arg Gly Leu
   1610 1615 1620
   Gly Thr Ser Ala Met Ser Gln Pro Ala Arg Glu Phe Ile Val Lys
   1625 1630 1635
   Ile Val Glu Met Ser Leu Trp Thr Ser Asp Phe Leu Leu Ala His
   1640 1645 1650
   Leu Leu Leu Ile Ile Leu Thr Val Pro Leu Leu Leu Pro Phe Phe
   1655 1660 1665
   Asn Ser Ile His Ser Thr Met Leu Phe Trp Leu Arg Pro Ser Lys
   1670 1675 1680
   Gln Ile Arg Gln Pro Leu Phe Ser Thr Lys Gln Lys Arg Gln Arg
   1685 1690 1695
   Arg Trp Ile Val Met Lys Tyr Thr Val Val Tyr Leu Val Val Val
   1700 1705 1710
   Ala Phe Leu Val Ala Leu Ile Ala Leu Pro Ala Leu Phe Arg Glu
   1715 1720 1725
   Ser Ile His Phe Asn Cys Glu Ile Cys Gln Ser Ile
   1730 1735 1740
<210> 48
   <211> 4869
   <212> DNA
   <213> Schizophyllum commune
<400> 48
   atgcggaata tgttcgactt caccatgcag ctgcttgaca gccgagcgtc tcgtatgacc 60
   cccaaccagg cgctcctcac cctccacgcc gactacattg gtggccagca tgcgaactac 120
   cggaagtggt acttcgcggc gcagctcgac cttgacgacg ccgtgggaca aactcagaat 180
   ccgggtctca accgcctcaa gtccactcgc ggatcgggca agcgaccacg ccatgaaaag 240
   tcgctgaaca cggcattgga gcgctggcgg caagccatga acaacatgtc gcagtatgac 300
   cgcttacgcc agatcgcgct ctacctgctc tgctggggcg aagcggcgca agtgcgattc 360
   atgcccgagt gcttgtgctt catcttcaag tgcgccgacg actactatcg ttcgccggag 420
   tgccagaaca ggatggagcc ggtaccggag ggtctctacc tgaggacggt cgtaaagccg 480
   ctctacagat ttgtccggga tcaaggctat gaggtggtgg agggaaaatt cgtacggcgg 540
   gaacgggatc acgaccaaat cattggttac gatgacgtga atcagctgtt ctggtacccg 600
   gagggaattg cccgtatcgt cctgtcggac aagagtcgtc tagtcgacct ccccccagca 660
   cagcgcttca tgaagttcga ccgtatcgag tggaatcgcg tcttcttcaa gacgttttac 720
   gagactcgat ccttcacgca tcttttggtc gacttcaacc gtatctgggt cgtgcacatc 780
   gctctctact tcttctacac tgcatacaac tcccccacga tctacgccat caacggcaac 840
   acaccgacgt ctctggcttg gagcgcgact gcgctcggcg gtgcggtagc gacaggtatc 900
   atgatcctcg ccacgatcgc cgagttctcg cacatcccca cgacatggaa caacacctcg 960
   catctgactc gccgcctcgc cttcctcctc gtcacgctcg gcctcacatg tggtccgacg 1020
   ttctacgtcg cgattgcaga gagcaacggg agcggcggct ctttggcctt gattctcggt 1080
   atcgtccagt tcttcatctc cgtcgtggca actgcgctct tcactatcat gccttctggt 1140
   cgtatgttcg gcgaccgtgt cgcaggcaag agtcgcaagt atctcgccag ccagacgttc 1200
   acggccagct acccgtcgtt gcccaagcac cagcggttcg cctcactcct gatgtggttc 1260
   ctcatcttcg ggtgcaagtt gacggagagt tacttctttc tgacgctgtc cttccgcgac 1320
   cctatccgcg tcatggtcgg catgaagatc cagaactgcg aggacaagat tttcggcagc 1380
   ggcctttgca ggaatcacgc agcattcacc ctcacgatca tgtacatcat ggacctcgtc 1440
   ttgttcttcc tcgacacctt cctttggtat gtcatctgga actcggtttt cagtatcgca 1500
   cgctctttcg tactcggcct ttcgatctgg acaccgtgga gagacatctt ccagcgtctg 1560
   ccgaagcgga tctacgcgaa gcttctggcg actggcgaca tggaggtcaa gtacaagccc 1620
   aaggtcttgg tctcgcaaat ctggaacgcc atcatcatct ccatgtaccg cgagcacttg 1680
   ctctctattg agcacgtcca gaagctcctg taccaccaag tggacactgg cgaagccggc 1740
   aagcggagtc ttcgcgcgcc tccgttcttc gtcgcgcagg gcagcagcgg tggctcgggc 1800
   gagttcttcc cgcctggcag cgaggccgag cgtcgtatct ctttcttcgc gcagtcgctt 1860
   tctacggaga ttcctcagcc catcccggtc gacgccatgc cgacgttcac ggtgcttacg 1920
   cctcactaca gcgagaagat ccttctctct ctccgtgaaa ttatccgcga ggaggaccag 1980
   aacactcgcg ttacgttgct cgagtacctg aagcagctgc atccggtcga gtgggagaat 2040
   ttcgtcaagg acactaaaat tttggccgag gagtccgcta tgtttaacgg tccgagtcct 2100
   ttcggcaacg acgagaaggg tcagtccaag atggacgatc taccgttcta ctgcatcggt 2160
   ttcaagagcg ccgcgcccga gtacaccctc cgcacccgta tctgggcgtc cctgcgcgcg 2220
   cagacgctgt accgcacggt ctccggcatg atgaactatg cgaaggcgat caagctgctc 2280
   taccgcgttg agaacccgga ggtcgtacaa cagttcggcg gcaacacgga caagctcgag 2340
   cgcgagttgg agcggatggc gcgacggaag ttcaagttcc tcgtgtccat gcagcgctac 2400
   tcgaagttca acaaggagga gcacgagaac gccgagttct tgctccgcgc gtacccggac 2460
   ttgcagatcg cgtacctcga ggaagagccc cctcgcaagg agggcggcga tccacgcatc 2520
   ttctctgccc tcgtcgacgg ccacagcgac atcatcccgg agaccggcaa gcggcgcccc 2580
   aagttccgta tcgagctgcc cggtaacccc attctcggtg acggtaaatc cgacaatcag 2640
   aaccacgcta tcgtcttcta ccgcggcgag tacctccagc ttatcgacgc caaccaggac 2700
   aactacctcg aggagtgctt gaagatccgt aacgtgctcg ccgagtttga ggagtacgac 2760
   gtctccagcc agagcccgta cgcgcagtgg agtgtcaagg agttcaagcg ctctccggtc 2820
   gccatcgtcg gtgcacgcga gtacatcttc tcagagcaca tcggtatcct cggtgatctg 2880
   gcggctggca aggaacagac gttcggtacg ctcacggcac gcaacaacgc cttccttggc 2940
   ggcaagctgc actacggtca ccccgatttc ctcaacgccc tctacatgaa cacgcgcggt 3000
   ggtgtctcca aggcgcagaa gggtctccat ctcaacgagg atatctacgc cggtatgaac 3060
   gcggtcggtc gcggtggacg cattaagcac agcgagtact atcagtgcgg caagggtcgt 3120
   gacctcggtt tcggcaccat cttgaacttc cagaccaaga tcggtacggg tatgggcgag 3180
   cagatcctct cgcgcgagta ctactatctc ggaacacaac tgcccatcga tcgcttcctc 3240
   acgttctact acgcgcaccc gggtttccag atcaacaaca tgctggtcat cctctccgtg 3300
   caggtcttca tcgttaccat ggtcttcctc ggtaccttga agtcttcggt cacgatctgc 3360
   aagtacacgt ccagcggtca gtacatcggt ggtcaatccg gttgctacaa cctcgtcccg 3420
   gtcttccagt ggatcgagcg ctgcatcatc agcatcttct tggtgttcat gatcgctttc 3480
   atgccgctct tcctgcaaga actcgtcgag cgcggtacct ggagtgccat ctggcgtctg 3540
   ctcaagcagt ttatgtcgct gtcgcctgtc ttcgaggtgt tctccaccca gattcagacg 3600
   cactccgtgt tgagcaactt gacgttcggt ggtgcgcgtt acatcgctac cggtcgtggg 3660
   ttcgccacca gtcgtatcag cttcagcatc ttgttctcgc gtttcgcagg cccgagtatc 3720
   tacctcggca tgcgcacgct cattatgctg ctctacgtga cgttgacgat ctggacgcca 3780
   tgggtcattt acttctgggt ttccattctc tcgctctgca tcgcgccgtt cttgttcaac 3840
   ccgcatcaat tcgtattctc ggacttcctc atcgactaca gggaatacct gcggtggatg 3900
   tcgcgtggca actcgcgctc gcacaacaac tcctggattg ggtactgccg gttgtcccgc 3960
   acgatgatca ctgggtacaa gaagaagaag ctgggccacc cgtcggagaa gctttccggc 4020
   gacgttcctc gtgcaggctg gcgcgccgtc ttgttctcgg agatcatctt cccggcgtgc 4080
   atggccatcc tcttcatcat cgcgtacatg ttcgtcaagt cgttccctct cgacggcaag 4140
   cagcctccct ccggcctcgt tcgcatcgcc gtcgtgtcta tcggccccat cgtgtggaac 4200
   gccgccatcc tgttgacgct cttccttgtg tcgttgttcc tcggccccat gctcgacccg 4260
   gtcttccccc tcttcggttc cgttatggcc ttcatcgcgc atttccttgg cacaatcgga 4320
   atgattgggt tcttcgagtt cctgtggttc ctcgagtcct gggaggcgtc gcatgccgtg 4380
   ctgggtctca tcgccgtcat ctccatccag cgcgccattc acaagatcct tatcgccgtt 4440
   ttcctcagtc gcgagttcaa gcacgacgag acgaacaggg cctggtggac tggtcgctgg 4500
   tatggccgtg gcctcggcac gcacgccatg tcgcagccgg cgcgtgagtt cgtcgtcaag 4560
   atcatcgagt tgtcgctttg gagctcggat ctcatactcg gccacatcct gctgttcatg 4620
   cttactccgg ccgtcctcat cccgtacttc gaccgtttgc acgccatgat gctcttctgg 4680
   ctgcgtccct cgaagcaaat ccgcgcgcct ctgtactcga tcaagcagaa gaggcaaaga 4740
   cgctggatta tcatgaagta cggtactgta tacgttaccg tcatcgcgat cttcgtcgcg 4800
   ctcatcgcgc ttcccctcgt attccgacac actctaaagg tcgagtgctc cctttgcgac 4860
   agcttgtaa 4869
<210> 49
   <211> 1783
   <212> PRT
   <213> Schizophyllum commune
<400> 49
   Met Pro Arg Pro Gly Gly Thr Ser Ala Glu Gly Gly Tyr Ala Ser Ser
   1               5                   10                  15
   Pro Ser Met Glu Thr Thr Pro Ser Asp Pro Phe Gly Thr Ala Asn Gly
               20                  25                  30
   Ala Pro Arg Arg Tyr Tyr Asp Asn Asp Ser Glu Glu Tyr Gly Pro Gly
           35                  40                  45
   Arg Arg Asp Thr Tyr Ala Ser Asp Ser Ser Asn Gln Gly Leu Thr Asp
       50                  55                  60
   Pro Gly Tyr Tyr Asp Gln Asn Gly Ala Tyr Asp Pro Tyr Pro Thr Gly
   65                  70                  75                  80
   Asp Thr Asp Ser Asp Gly Asp Val Tyr Gly Gln Arg Tyr Gly Pro Ser
                   85                  90                  95
   Ala Glu Ser Leu Gly Thr His Lys Phe Gly His Ser Asp Ser Ser Thr
               100                 105                 110
   Pro Thr Phe Val Asp Tyr Ser Ala Ser Ser Gly Gly Arg Asp Ser Tyr
           115                 120                 125
   Pro Ala Trp Thr Ala Glu Arg Asn Ile Pro Leu Ser Lys Glu Glu Ile
       130                 135                 140
   Glu Asp Ile Phe Leu Asp Leu Thr Gln Lys Phe Gly Phe Gln Arg Asp
   145                 150                 155                 160
   Ser Met Arg Asn Met Phe Asp Phe Thr Met Gln Leu Leu Asp Ser Arg
                   165                 170                 175
   Ala Ser Arg Met Thr Pro Asn Gln Ala Leu Leu Thr Leu His Ala Asp
   180 185 190
   Tyr Ile Gly Gly Gln His Ala Asn Tyr Arg Lys Trp Tyr Phe Ala Ala
   195 200 205
   Gln Leu Asp Leu Asp Asp Ala Val Gly Gln Thr Gln Asn Pro Gly Leu
   210 215 220
   Asn Arg Leu Lys Ser Thr Arg Gly Ser Gly Lys Arg Pro Arg His Glu
   225 230 235 240
   Lys Ser Leu Asn Thr Ala Leu Glu Arg Trp Arg Gln Ala Met Asn Asn
   245 250 255
   Met Ser Gln Tyr Asp Arg Leu Arg Gln Ile Ala Leu Tyr Leu Leu Cys
   260 265 270
   Trp Gly Glu Ala Ala Gln Val Arg Phe Met Pro Glu Cys Leu Cys Phe
   275 280 285
   Ile Phe Lys Cys Ala Asp Asp Tyr Tyr Arg Ser Pro Glu Cys Gln Asn
   290 295 300
   Arg Met Glu Pro Val Pro Glu Gly Leu Tyr Leu Arg Thr Val Val Lys
   305 310 315 320
   Pro Leu Tyr Arg Phe Val Arg Asp Gln Gly Tyr Glu Val Val Glu Gly
   325 330 335
   Lys Phe Val Arg Arg Glu Arg Asp His Asp Gln Ile Ile Gly Tyr Asp
   340 345 350
   Asp Val Asn Gln Leu Phe Trp Tyr Pro Glu Gly Ile Ala Arg Ile Val
   355 360 365
   Leu Ser Asp Lys Ser Arg Leu Val Asp Leu Pro Pro Ala Gln Arg Phe
   370 375 380
   Met Lys Phe Asp Arg Ile Glu Trp Asn Arg Val Phe Phe Lys Thr Phe
   385 390 395 400
   Tyr Glu Thr Arg Ser Phe Thr His Leu Leu Val Asp Phe Asn Arg Ile
   405 410 415
   Trp Val Val His Ile Ala Leu Tyr Phe Phe Tyr Thr Ala Tyr Asn Ser
   420 425 430
   Pro Thr Ile Tyr Ala Ile Asn Gly Asn Thr Pro Thr Ser Leu Ala Trp
   435 440 445
   Ser Ala Thr Ala Leu Gly Gly Ala Val Ala Thr Gly Ile Met Ile Leu
   450 455 460
   Ala Thr Ile Ala Glu Phe Ser His Ile Pro Thr Thr Trp Asn Asn Thr
   465 470 475 480
   Ser His Leu Thr Arg Arg Leu Ala Phe Leu Leu Val Thr Leu Gly Leu
   485 490 495
   Thr Cys Gly Pro Thr Phe Tyr Val Ala Ile Ala Glu Ser Asn Gly Ser
   500 505 510
   Gly Gly Ser Leu Ala Leu Ile Leu Gly Ile Val Gln Phe Phe Ile Ser
   515 520 525
   Val Val Ala Thr Ala Leu Phe Thr Ile Met Pro Ser Gly Arg Met Phe
   530 535 540
   Gly Asp Arg Val Ala Gly Lys Ser Arg Lys Tyr Leu Ala Ser Gln Thr
   545 550 555 560
   Phe Thr Ala Ser Tyr Pro Ser Leu Pro Lys His Gln Arg Phe Ala Ser
   565 570 575
   Leu Leu Met Trp Phe Leu Ile Phe Gly Cys Lys Leu Thr Glu Ser Tyr
   580 585 590
   Phe Phe Leu Thr Leu Ser Phe Arg Asp Pro Ile Arg Val Met Val Gly
   595 600 605
   Met Lys Ile Gln Asn Cys Glu Asp Lys Ile Phe Gly Ser Gly Leu Cys
   610 615 620
   Arg Asn His Ala Ala Phe Thr Leu Thr Ile Met Tyr Ile Met Asp Leu
   625 630 635 640
   Val Leu Phe Phe Leu Asp Thr Phe Leu Trp Tyr Val Ile Trp Asn Ser
   645 650 655
   Val Phe Ser Ile Ala Arg Ser Phe Val Leu Gly Leu Ser Ile Trp Thr
   660 665 670
   Pro Trp Arg Asp Ile Phe Gln Arg Leu Pro Lys Arg Ile Tyr Ala Lys
           675                 680                 685
   Leu Leu Ala Thr Gly Asp Met Glu Val Lys Tyr Lys Pro Lys Val Leu
       690                 695                 700
   Val Ser Gln Ile Trp Asn Ala Ile Ile Ile Ser Met Tyr Arg Glu His
   705                 710                 715                 720
   Leu Leu Ser Ile Glu His Val Gln Lys Leu Leu Tyr His Gln Val Asp
                   725                 730                 735
   Thr Gly Glu Ala Gly Lys Arg Ser Leu Arg Ala Pro Pro Phe Phe Val
               740                 745                 750
   Ala Gln Gly Ser Ser Gly Gly Ser Gly Glu Phe Phe Pro Pro Gly Ser
           755                 760                 765
   Glu Ala Glu Arg Arg Ile Ser Phe Phe Ala Gln Ser Leu Ser Thr Glu
       770                 775                 780
   Ile Pro Gln Pro Ile Pro Val Asp Ala Met Pro Thr Phe Thr Val Leu
   785                 790                 795                 800
   Thr Pro His Tyr Ser Glu Lys Ile Leu Leu Ser Leu Arg Glu Ile Ile
                   805                 810                 815
   Arg Glu Glu Asp Gln Asn Thr Arg Val Thr Leu Leu Glu Tyr Leu Lys
               820                 825                 830
   Gln Leu His Pro Val Glu Trp Glu Asn Phe Val Lys Asp Thr Lys Ile
           835                 840                 845
   Leu Ala Glu Glu Ser Ala Met Phe Asn Gly Pro Ser Pro Phe Gly Asn
       850                 855                 860
   Asp Glu Lys Gly Gln Ser Lys Met Asp Asp Leu Pro Phe Tyr Cys Ile
   865                 870                 875                 880
   Gly Phe Lys Ser Ala Ala Pro Glu Tyr Thr Leu Arg Thr Arg Ile Trp
                   885                 890                 895
   Ala Ser Leu Arg Ala Gln Thr Leu Tyr Arg Thr Val Ser Gly Met Met
               900                 905                 910
   Asn Tyr Ala Lys Ala Ile Lys Leu Leu Tyr Arg Val Glu Asn Pro Glu
           915                 920                 925
   Val Val Gln Gln Phe Gly Gly Asn Thr Asp Lys Leu Glu Arg Glu Leu
       930                 935                 940
   Glu Arg Met Ala Arg Arg Lys Phe Lys Phe Leu Val Ser Met Gln Arg
   945                 950                 955                 960
   Tyr Ser Lys Phe Asn Lys Glu Glu His Glu Asn Ala Glu Phe Leu Leu
                   965                 970                 975
   Arg Ala Tyr Pro Asp Leu Gln Ile Ala Tyr Leu Glu Glu Glu Pro Pro
               980                 985                 990
   Arg Lys Glu Gly Gly Asp Pro Arg  Ile Phe Ser Ala Leu  Val Asp Gly
           995                 1000                 1005
   His Ser  Asp Ile Ile Pro Glu  Thr Gly Lys Arg Arg  Pro Lys Phe
       1010                 1015                 1020
   Arg Ile  Glu Leu Pro Gly Asn  Pro Ile Leu Gly Asp  Gly Lys Ser
       1025                 1030                 1035
   Asp Asn  Gln Asn His Ala Ile  Val Phe Tyr Arg Gly  Glu Tyr Leu
       1040                 1045                 1050
   Gln Leu  Ile Asp Ala Asn Gln  Asp Asn Tyr Leu Glu  Glu Cys Leu
       1055                 1060                 1065
   Lys Ile  Arg Asn Val Leu Ala  Glu Phe Glu Glu Tyr  Asp Val Ser
       1070                 1075                 1080
   Ser Gln  Ser Pro Tyr Ala Gln  Trp Ser Val Lys Glu  Phe Lys Arg
       1085                 1090                 1095
   Ser Pro  Val Ala Ile Val Gly  Ala Arg Glu Tyr Ile  Phe Ser Glu
       1100                 1105                 1110
   His Ile  Gly Ile Leu Gly Asp  Leu Ala Ala Gly Lys  Glu Gln Thr
       1115                 1120                 1125
   Phe Gly  Thr Leu Thr Ala Arg  Asn Asn Ala Phe Leu  Gly Gly Lys
       1130                 1135                 1140
   Leu His  Tyr Gly His Pro Asp  Phe Leu Asn Ala Leu  Tyr Met Asn
       1145                 1150                 1155
   Thr Arg  Gly Gly Val Ser Lys  Ala Gln Lys Gly Leu  His Leu Asn
       1160                 1165                 1170
   Glu Asp  Ile Tyr Ala Gly Met  Asn Ala Val Gly Arg  Gly Gly Arg
       1175                 1180                 1185
   Ile Lys  His Ser Glu Tyr Tyr  Gln Cys Gly Lys Gly  Arg Asp Leu
       1190                 1195                 1200
   Gly Phe  Gly Thr Ile Leu Asn  Phe Gln Thr Lys Ile  Gly Thr Gly
       1205                 1210                 1215
   Met Gly  Glu Gln Ile Leu Ser  Arg Glu Tyr Tyr Tyr  Leu Gly Thr
       1220                 1225                 1230
   Gln Leu  Pro Ile Asp Arg Phe  Leu Thr Phe Tyr Tyr  Ala His Pro
       1235                 1240                 1245
   Gly Phe  Gln Ile Asn Asn Met  Leu Val Ile Leu Ser  Val Gln Val
       1250                 1255                 1260
   Phe Ile  Val Thr Met Val Phe  Leu Gly Thr Leu Lys  Ser Ser Val
       1265                 1270                 1275
   Thr Ile  Cys Lys Tyr Thr Ser  Ser Gly Gln Tyr Ile  Gly Gly Gln
       1280                 1285                 1290
   Ser Gly  Cys Tyr Asn Leu Val  Pro Val Phe Gln Trp  Ile Glu Arg
       1295                 1300                 1305
   Cys Ile  Ile Ser Ile Phe Leu  Val Phe Met Ile Ala  Phe Met Pro
       1310                 1315                 1320
   Leu Phe  Leu Gln Glu Leu Val  Glu Arg Gly Thr Trp  Ser Ala Ile
       1325                 1330                 1335
   Trp Arg  Leu Leu Lys Gln Phe  Met Ser Leu Ser Pro  Val Phe Glu
       1340                 1345                 1350
   Val Phe  Ser Thr Gln Ile Gln  Thr His Ser Val Leu  Ser Asn Leu
       1355                 1360                 1365
   Thr Phe  Gly Gly Ala Arg Tyr  Ile Ala Thr Gly Arg  Gly Phe Ala
       1370                 1375                 1380
   Thr Ser  Arg Ile Ser Phe Ser  Ile Leu Phe Ser Arg  Phe Ala Gly
       1385                 1390                 1395
   Pro Ser  Ile Tyr Leu Gly Met  Arg Thr Leu Ile Met  Leu Leu Tyr
   1400 1405 1410
   Val Thr Leu Thr Ile Trp Thr Pro Trp Val Ile Tyr Phe Trp Val
   1415 1420 1425
   Ser Ile Leu Ser Leu Cys Ile Ala Pro Phe Leu Phe Asn Pro His
   1430 1435 1440
   Gln Phe Val Phe Ser Asp Phe Leu Ile Asp Tyr Arg Glu Tyr Leu
   1445 1450 1455
   Arg Trp Met Ser Arg Gly Asn Ser Arg Ser His Asn Asn Ser Trp
   1460 1465 1470
   Ile Gly Tyr Cys Arg Leu Ser Arg Thr Met Ile Thr Gly Tyr Lys
   1475 1480 1485
   Lys Lys Lys Leu Gly His Pro Ser Glu Lys Leu Ser Gly Asp Val
   1490 1495 1500
   Pro Arg Ala Gly Trp Arg Ala Val Leu Phe Ser Glu Ile Ile Phe
   1505 1510 1515
   Pro Ala Cys Met Ala Ile Leu Phe Ile Ile Ala Tyr Met Phe Val
   1520 1525 1530
   Lys Ser Phe Pro Leu Asp Gly Lys Gln Pro Pro Ser Gly Leu Val
   1535 1540 1545
   Arg Ile Ala Val Val Ser Ile Gly Pro Ile Val Trp Asn Ala Ala
   1550 1555 1560
   Ile Leu Leu Thr Leu Phe Leu Val Ser Leu Phe Leu Gly Pro Met
   1565 1570 1575
   Leu Asp Pro Val Phe Pro Leu Phe Gly Ser Val Met Ala Phe Ile
   1580 1585 1590
   Ala His Phe Leu Gly Thr Ile Gly Met Ile Gly Phe Phe Glu Phe
   1595 1600 1605
   Leu Trp Phe Leu Glu Ser Trp Glu Ala Ser His Ala Val Leu Gly
   1610 1615 1620
   Leu Ile Ala Val Ile Ser Ile Gln Arg Ala Ile His Lys Ile Leu
   1625 1630 1635
   Ile Ala Val Phe Leu Ser Arg Glu Phe Lys His Asp Glu Thr Asn
   1640 1645 1650
   Arg Ala Trp Trp Thr Gly Arg Trp Tyr Gly Arg Gly Leu Gly Thr
   1655 1660 1665
   His Ala Met Ser Gln Pro Ala Arg Glu Phe Val Val Lys Ile Ile
   1670 1675 1680
   Glu Leu Ser Leu Trp Ser Ser Asp Leu Ile Leu Gly His Ile Leu
   1685 1690 1695
   Leu Phe Met Leu Thr Pro Ala Val Leu Ile Pro Tyr Phe Asp Arg
   1700 1705 1710
   Leu His Ala Met Met Leu Phe Trp Leu Arg Pro Ser Lys Gln Ile
   1715 1720 1725
   Arg Ala Pro Leu Tyr Ser Ile Lys Gln Lys Arg Gln Arg Arg Trp
   1730 1735 1740
   Ile Ile Met Lys Tyr Gly Thr Val Tyr Val Thr Val Ile Ala Ile
   1745 1750 1755
   Phe Val Ala Leu Ile Ala Leu Pro Leu Val Phe Arg His Thr Leu
   1760 1765 1770
   Lys Val Glu Cys Ser Leu Cys Asp Ser Leu
   1775 1780
<210> 50
   <211> 1858
   <212> DNA
   <213> Schizophyllum commune
<400> 50
   atgtacgcca ccgcggcaca cgactacaag accaccggcc cccaggccgt catcgacggc      60
   atcgacaggc agttccagct ctccaaggat tcccttgtcg aaatcacaga agccttcctg     120
   cgcgagttca agcaagggct ctcgaagtat ggcgagccca tggccatgat gtgcgtgtta     180
   tcagtgaaac gggatcgatg ttcgatcagc tgaactgaga tcaatagacc gacattcgtg     240
   actggtgtcc ctgacggttc ggaaactggg tgcgtcgact gcctccttgc atcgccatgc     300
   agctgacgct tccccagtac attcctcgcc ctggaccttg gcggcacgaa cctgtaggcg     360
   aagcgactcc tttcccccgc cctctgctaa attcccgatc agccgcgtct gcgaggtcgt     420
   cttgaatggg gaccggacat tcgacctcac acaacagaag tacaaggttt ctgacgcctt     480
   aaagaccggc gaagctaccg acctcttcgg taggcaaaac ctccttaccc gctcctctat     540
   gactgacagc cgcgcggaaa agactacttg gcggattccg tcgacgcttt cctcaccacc      600
   cacgccaagt ctaccgcaag cgcgcacgta cccgggacag agtcgccata cccaacgaaa      660
   gccgactata acccggaaat cgtcaacctt ggcctcacct tctctttccc agtagagcaa      720
   actgcgctcg acgagggaaa gattttaacc tggacgaaag gcttttccgc gaagcatgcc      780
   gtcggaaacg atgtcgtaaa gctcctgcag gacgccttcg accgcaagca cttgcatgtg      840
   cgctgcgtcg cccttgtcaa cgacgtatgt gcactaggct cttgtccttt cccttgctaa      900
   ttccctatag actgttggcg cgcttctatc gcgagcgtac accggcggtg gctgcacact      960
   aggcgcgatc ttcggcacgg gcacgaatgg tgcctacgtc gagcaggttg ccaacatcac     1020
   caaactggcc aactcgcccg ccgctgccaa gggcggccaa atggtcatca acaccgaatg     1080
   gggtgcattc aacaacacgc gcacgcacct gcccaatacg ccctacgaca cctccctcga     1140
   ccgccggagt atcaacccgc gcttccaggc gttcgagaag ttcatttccg ggatgtatct     1200
   cggcgagatc acccgcaata tcctcgtatc ccttgtcgac gccgtgccga aaccgctgct     1260
   tttctccggc gcctcttcgg cgacgcttaa ccaccagtgg gggctcgaca cctcggtcat     1320
   gtccgaggtc gaggaggcct gggaagcagg gagggaggac acgtactcgg gcaagcccat     1380
   tgatgcgcca gacttcgagg cgctgtcgga cgagtccaag atcgacgaga aggtcaagac     1440
   gcgcctgcag aagatcaggg acattgtcgt acggcggctt cagctgcccg cgaacgacgt     1500
   ctccctgcgc gacgccgcca tcgtccggtg ggcatgcgcg ctcgtcgcgc ggcgcgcggg     1560
   gctgctcagc ggtaccgccg tcgccgctgt gctcatacag accgggcgcg gctcgctcct     1620
   gaagggggag tcaaatggca cgaacggcaa ggcggcggaa ccggaggaca agtttatcgt     1680
   tggtgtcgac ggtagcctga tccagttcta cccgaacttc gagccgacga tgcgcgaggc     1740
   gatccggatg ctcacgggcg agtctgtcga gaagagcgtc gagatcgggc tcgcgaagga     1800
   tgggagtggg gtcggtgctg cgctgtgcgc gctgcaggcg attaagcagc tggcataa       1858
<210> 51
   <211> 534
   <212> PRT
   <213> Schizophyllum commune
<400> 51
   Met Tyr Ala Thr Ala Ala His Asp Tyr Lys Thr Thr Gly Pro Gln Ala
   1               5                   10                  15
   Val Ile Asp Gly Ile Asp Arg Gln Phe Gln Leu Ser Lys Asp Ser Leu
               20                  25                  30
   Val Glu Ile Thr Glu Ala Phe Leu Arg Glu Phe Lys Gln Gly Leu Ser
           35                  40                  45
   Lys Tyr Gly Glu Pro Met Ala Met Ile Pro Thr Phe Val Thr Gly Val
       50                  55                  60
   Pro Asp Gly Ser Glu Thr Gly Thr Phe Leu Ala Leu Asp Leu Gly Gly
   65                  70                  75                  80
   Thr Asn Leu Arg Val Cys Glu Val Val Leu Asn Gly Asp Arg Thr Phe
                   85                  90                  95
   Asp Leu Thr Gln Gln Lys Tyr Lys Val Ser Asp Ala Leu Lys Thr Gly
               100                 105                 110
   Glu Ala Thr Asp Leu Phe Asp Tyr Leu Ala Asp Ser Val Asp Ala Phe
           115                 120                 125
   Leu Thr Thr His Ala Lys Ser Thr Ala Ser Ala His Val Pro Gly Thr
       130                 135                 140
   Glu Ser Pro Tyr Pro Thr Lys Ala Asp Tyr Asn Pro Glu Ile Val Asn
   145                 150                 155                 160
   Leu Gly Leu Thr Phe Ser Phe Pro Val Glu Gln Thr Ala Leu Asp Glu
                   165                 170                 175
   Gly Lys Ile Leu Thr Trp Thr Lys Gly Phe Ser Ala Lys His Ala Val
               180                 185                 190
   Gly Asn Asp Val Val Lys Leu Leu Gln Asp Ala Phe Asp Arg Lys His
           195                 200                 205
   Leu His Val Arg Cys Val Ala Leu Val Asn Asp Thr Val Gly Ala Leu
       210                 215                 220
   Leu Ser Arg Ala Tyr Thr Gly Gly Gly Cys Thr Leu Gly Ala Ile Phe
   225                 230                 235                 240
   Gly Thr Gly Thr Asn Gly Ala Tyr Val Glu Gln Val Ala Asn Ile Thr
                   245                 250                 255
   Lys Leu Ala Asn Ser Pro Ala Ala Ala Lys Gly Gly Gln Met Val Ile
               260                 265                 270
   Asn Thr Glu Trp Gly Ala Phe Asn Asn Thr Arg Thr His Leu Pro Asn
           275                 280                 285
   Thr Pro Tyr Asp Thr Ser Leu Asp Arg Arg Ser Ile Asn Pro Arg Phe
   290 295 300
   Gln Ala Phe Glu Lys Phe Ile Ser Gly Met Tyr Leu Gly Glu Ile Thr
   305 310 315 320
   Arg Asn Ile Leu Val Ser Leu Val Asp Ala Val Pro Lys Pro Leu Leu
   325 330 335
   Phe Ser Gly Ala Ser Ser Ala Thr Leu Asn His Gln Trp Gly Leu Asp
   340 345 350
   Thr Ser Val Met Ser Glu Val Glu Glu Ala Trp Glu Ala Gly Arg Glu
   355 360 365
   Asp Thr Tyr Ser Gly Lys Pro Ile Asp Ala Pro Asp Phe Glu Ala Leu
   370 375 380
   Ser Asp Glu Ser Lys Ile Asp Glu Lys Val Lys Thr Arg Leu Gln Lys
   385 390 395 400
   Ile Arg Asp Ile Val Val Arg Arg Leu Gln Leu Pro Ala Asn Asp Val
   405 410 415
   Ser Leu Arg Asp Ala Ala Ile Val Arg Trp Ala Cys Ala Leu Val Ala
   420 425 430
   Arg Arg Ala Gly Leu Leu Ser Gly Thr Ala Val Ala Ala Val Leu Ile
   435 440 445
   Gln Thr Gly Arg Gly Ser Leu Leu Lys Gly Glu Ser Asn Gly Thr Asn
   450 455 460
   Gly Lys Ala Ala Glu Pro Glu Asp Lys Phe Ile Val Gly Val Asp Gly
   465 470 475 480
   Ser Leu Ile Gln Phe Tyr Pro Asn Phe Glu Pro Thr Met Arg Glu Ala
   485 490 495
   Ile Arg Met Leu Thr Gly Glu Ser Val Glu Lys Ser Val Glu Ile Gly
   500 505 510
   Leu Ala Lys Asp Gly Ser Gly Val Gly Ala Ala Leu Cys Ala Leu Gln
   515 520 525
   Ala Ile Lys Gln Leu Ala
   530

## Description

The present invention relates to genetically modified microorganisms capable of producing beta-glucans herein also referred to as beta-glucans, and to a process for producing beta glucan polymers.

### Technical Background

Beta-glucans are known well-conserved components of cell walls in several microorganisms, particularly in fungi and yeast.

A large number of closely related beta-glucans exhibit a similar branching pattern such as schizophyllan, scleroglucan, pendulan, cinerian, laminarin, lentinan and pleuran, all of which exhibit a linear main chain of beta-D-(1-3)-glucopyranosyl units with a single beta-D-glucopyranosyl unit (1-6) linked to a beta-D-glucopyranosyl unit of the linear main chain with an average branching degree of about 0.3

PCT/EP2013/064024 relates to a genetically modified microorganism capable of producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, characterized in that said genetically modified microorganism overexpresses (i) a polynucleotide encoding a polypeptide having 1,3-beta-D-glucan synthase-activity, and/or (ii) a polypeptide having 1,3-beta-D-glucan synthase-activity, compared to a corresponding non-modified control microorganism of the same strain

### Description of the Invention

In a first embodiment the invention relates to genetically modified microorganism capable of producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, wherein the genetically modified microorganism - compared to a non-modified control microorganism of the same strain - has increased activity of at least two gene products selected from the group consisting of
(A) phosphoglucomutase with a SEQ ID NO:29 or a polypeptide sequence which is at least 95% identical to SEQ ID NO:29 and which have at least 80% of the enzymatic activity of the polypeptide according to SEQ ID NO:29,
(B) UTP-glucose-1-phosphate uridyltrasferase with a SEQ ID NO:41 or a polypeptide sequence which is at least 95% identical to SEQ ID NO:41 and which have at least 80% of the enzymatic activity of the polypeptide according to SEQ ID NO:41,
(C) branching enzyme with a SEQ ID NO:2 or a polypeptide sequence which is at least 95% identical to SEQ ID NO:2 and which have at least 80% of the enzymatic activity of the polypeptide according to SEQ ID NO:2;
wherein an increased activity of the respective gene product is achieved by introducing into the microorganism genes in multiple copies for the respective gene product, under conditions allowing said microorganism to produce said polymer; wherein said microorganism is Schizophyllum and wherein said polymer is schizophyllan.

The gene product phosphoglucomutase means an enzyme that transfers a phosphate group on an α-D-glucose monomer from the 1' to the 6' position in the forward direction or the 6' to the 1' position in the reverse direction.

The IUBMB Enzyme Nomenclature for hexokinase is EC 5.4.2.2.

A preferred polypeptide sequence for a hexokinase is disclosed in polypeptide sequences which are at least 96%, 97%, 98%, and 99% identical to SEQ ID NO: 29 and which have at least 80%, preferred 90%, 100% and most preferred more than 100%, more than 120%, more than 150% of the enzymatic activity of the polypeptide according to SEQ ID NO: 29.

The gene product UTP-glucose-1-phosphate uridyltrasferase, also known as glucose-1-phosphate uridylyltransferase (or UDP-glucose pyrophosphorylase) is an enzyme associated with glycogenesis. It synthesizes UDP-glucose from glucose-1-phosphate and UTP; The reaction catalyzed by it is:

glucose-1-phosphate + UTP -> UDP-glucose + pyrophosphate

The IUBMB Enzyme Nomenclature for a UTP-glucose-1-phosphate uridyltrasferase is EC 2.7.7.9.

Preferred polypeptide sequences for a UTP-glucose-1-phosphate uridyltrasferase are disclosed in polypeptide sequences which are at least 96%, 97%, 98%, and 99% identical to SEQ ID NO: 41 and which have at least 80%, preferred 90%, 100% and most preferred more than 100%, more than 120% , more than 150% of the enzymatic activity of the polypeptide according to SEQ ID NO: 41.

The gene product branching enzyme means Glycosyltransferases, Branching Enzymes, Glucosidases, Transglucosidases which catalyse the formation of a beta-D-(1-3)-glucopyranosyl units having a single beta-D-glucopyranosyl unit (1-6) linked to a beta-D-glucopyranosyl unit of the linear main chain with an average branching degree of about 0.3, that is a major component of the fungal cell wall. The reaction catalysed is:

UDP-glucose + {(1,3)-beta-D-glucosyl}(N) = UDP + {(1,3:1,6)-beta-D-glucosyl}(N+1)

The IUBMB Enzyme Nomenclature for a branching enzyme is EC 2.4.1.-.

Preferred polypeptide sequences for a branching enzyme are disclosed in polypeptide sequences which are at least 96%, 97%, 98%, and 99% identical to SEQ ID NO: 2, and which have at least 80%, preferred 90%, 100% and most preferred more than 100%, more than 120% , more than 150% of the enzymatic activity of the polypeptide according to SEQ ID NO: 2.

Suitable microorganisms useful as starting organisms for the genetic modification according to the invention are microorgansims of the genus *Schizophyllum* especially *Schizophyllum commune.*

Preferred microorganisms are *Schizophyllum commune* which are availabe from public deposits, e.g.:
DSM-1024, DSM-1025, DSM-1026, DSM-11223
ATCC® Number: 204191, ATCC® Number: MYA-2104, ATCC® Number: 26890, ATCC® Number: 26892, ATCC® Number: 62873, ATCC® Number: 38229,
ATCC® Number: 32746, ATCC® Number: MYA-4819, ATCC® Number: 52396,
ATCC® Number: MYA-1128, ATCC® Number: 42093, ATCC® Number: 18246,
ATCC® Number: MYA-1124, ATCC® Number: 38230, ATCC® Number: 26889,
ATCC® Number: 26262, ATCC® Number: 52398, ATCC® Number: MYA-1123.

In another embodiment the invention relates to a process for producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, said process comprising the steps of:
(i) culturing in a medium a genetically modified microorganism capable of producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, wherein the genetically modified microorganism - compared to a non-modified control microorganism of the same strain - has increased activity of at least two gene products selected from the group consisting of
   (A) phosphoglucomutase with a SEQ ID NO:29 or a polypeptide sequence which is at least 95% identical to SEQ ID NO:29 and which have at least 80% of the enzymatic activity of the polypeptide according to SEQ ID NO:29,
   (B) UTP-glucose-1-phosphate uridyltrasferase with a SEQ ID NO:41 or a polypeptide sequence which is at least 95% identical to SEQ ID NO:41 and which have at least 80% of the enzymatic activity of the polypeptide according to SEQ ID NO:41,
   (C) branching enzyme with a SEQ ID NO:2 or a polypeptide sequence which is at least 95% identical to SEQ ID NO:2 and which have at least 80% of the enzymatic activity of the polypeptide according to SEQ ID NO:2;
      under conditions allowing said microorganism to produce said polymer;
   wherein an increased activity of the respective gene product is achieved by introducing into the microorganism genes in multiple copies for the respective gene product,
(ii) optionally recovering said polymer from the medium,
wherein said microorganism is Schizophyllum and wherein said polymer is schizophyllan.

The genetically modified microorganisms according to the invention can be produced by known techniques of recombinant DNA technology such as described e.g. in Sambrook et al, Molecular Cloning - A Laboratory Manual, Cold Spring Harbor Laboratory, Cold Spring Harbor, New York (1989) or Current Protocols in Molecular Biology Volumes 1-3, John Wiley & Sons, Inc. (1994-1998). Further examples for the construction of genetically modified microorganisms are disclosed in the Experimental Part.

Methods for culturing microorganisms such as fermentation processes are known in the art and also described and exemplified herein (Kumari, Bioresource Technol (2008), 99: 1036-1043; Reyes, J Natural Studies (2009), 7(2), January-June). In context with the present invention, such methods allow the respective microorganism to grow and to produce the desired beta-glucan as described and exemplified herein. Suitable media may comprise, e.g., coconut water as described in Reyes, *loc cit.* Furthermore, as known in the art, there are several media particularly suitable for particular microorganisms.

For example, also in context with the present invention, suitable media for culturing S. *commune* comprise CYM medium (25 g agar (Difco), 20 g glucose (Sigma), 2 g trypticase peptone (Roth), 2 g yeast extract (Difco), 0.5 g MgSO₄ x 7 H₂O (Roth), 0.5 g KH₂PO₄ and 1 g K₂HPO₄ (both from Riedel-de Haën) per liter H₂O) (particularly useful for cultivation on solid support) or a medium comprising 30 g glucose (Sigma), 3 g yeast extract (Difco), 1 g KH₂PO₄ (Riedel-de Haën), 0.5 g MgSO₄ x 7 H₂O (Roth) per liter H₂O (particularly useful for liquid cultures) as also described and exemplified herein.

In context with the present invention, the term "average branching degree about 0,3" may mean that in average about 3 of 10 beta-D-(1-3)-glucopyranosyl units are (1-6) linked to a single beta-D-glucopyranosyl unit. In this context, the term "about" may mean that the average branching degree may be within the range from 0.1 to 0.5, preferably from 0.2 to 0.4, more preferably from 0.25 to 0.35, more preferably from 0.25 to 0.33, more preferably from 0.27 to 0.33, and most preferably from 0.3 to 0.33. It may also be 0.3 or 0.33. Schizophyllan, scleroglucan, pendulan, cinerian, laminarin, lentinan and pleuran all have an average branching degree between 0.25 and 0.33; for example, scleroglucan and schizophyllan have an average branching degree of 0.3 to 0.33 (Survase, *loc cit,* Novak, *loc cit*)*.* The average branching degree of a beta-glucan can be determined by methods known in the art, e.g., by periodic oxidation analysis, methylated sugar analysis and NMR (Brigand, Industrial Gums, Academic Press, New York/USA (1993), 461-472).

The recovering of the polymer from the fermentation product can be performed by a number of routine techniques known in biotechnology such as precipitation and centrifugation.

Many processes for the preparation of beta-glucans comprise the cultivation and fermentation of microorganisms capable of synthesizing such biopolymers. For example, EP 271 907 A2, EP 504 673 A1 and DE 40 12 238 A1 disclose processes for the preparation, *i.e.* the preparation is effected by batchwise fermentation of the fungus *Schizophy*//*um commune* with stirring and aeration. The culture medium substantially comprises glucose, yeast extract, potassium dihydrogen phosphate, magnesium sulfate and water. EP 271 907 A2 describes a method for isolating the polysaccharide, in which the culture suspension is first centrifuged and the polysaccharide is precipitated from the supernatant with isopropanol. A second method comprises a pressure filtration followed by an ultrafiltration of the solution obtained, without details of the method having been disclosed. "Udo Rau, "Biosynthese, Produktion und Eigenschaften von extrazellulären Pilz-Glucanen", Habilitationsschrift, Technical University of Brunswick, 1997, pages 70 to 95" and "Udo Rau, Biopolymers, Editor A. Steinbüchel, Volume 6, pages 63 to 79, WILEY-VCH Publishers, New York, 2002" describe the preparation of schizophyllan by continuous or batchwise fermentation. "GIT Fachzeitung Labor 12/92, pages 1233 - 1238" describes a continuous preparation of branched beta-1,3-glucans with cell recycling. WO 03/016545 A2 discloses a continuous process for the preparation of scleroglucans using *Sclerotium rolfsii.*

Furthermore, for economic reasons, the concentration of aqueous beta-glucan solutions should be as high as possible in order to ensure as little transport effort as possible for transporting the aqueous glucan solutions from the production site to the place of use. For this purpose, beta-glucan solutions are usually concentrated by drying, lyophilization and/or precipitation before being transported in order to reduce their weight.

The genetically modified microorganism according to the invention is able to produce at least 1.5 times, more preferably at least 1.8 times more, more preferably at least 2.0 times more, and most preferably at least 2.2 times more beta-glucan polymer compared to the corresponding non-modified control microorganism. In this context, production of, e.g., 1.5 times "more" beta-glucan polymer may mean that a genetically modified microorganism produces an amount of beta-glucan polymer which is 1.5 times higher compared to the amount of beta-glucan polymer produced in the same time under the same conditions by a corresponding non-modified control microorganism. Alternatively, production of, e.g., 1.5 times "more" beta-glucan polymer may mean that a genetically modified microorganism produces the same amount of beta-glucan polymer as a corresponding non-modified control organism under the same conditions, however, 1.5 times faster. The amount of produced beta-glucan polymer may be measured by methods known in the art and as also described herein.

### Experimental Part

### Example 1a

### Cloning of the Glucosidase 2 expression plasmid (pBE_2.1) and transformation into S. commune

In the genome of *Schizophyllum commune,* two genes encoding for a putative Glucosidase were identified by using BLAST analysis. In context of the present invention, it was proven that the overexpression one of the Glucosidases (Glucosidase 2) in S. *commune* results in increased yields of Schizophyllan.
Two expression plasmids (pBE_2.1)] and (pBE_2.2) (having pBluescript II as backbone) were generated carrying a bacterial selection marker cassette (*amp^{R}*), strong constitutive promoter (Tef1 promoter), the Glucosidase 2 gene sequence and terminator sequence (Tef1 terminator). pBE_2.1 is carrying the fungal selection marker *ura1,* pBE_2.2 is carrying the fungal selection marker *clonnat.*
All polynucleotide sequences described herein originate from *Schizopyllum commune,* isolated using PCR technology prepared by established microbiologic protocols (Sambrook, loc cit, Current Protocols in Molecular Biology, Update May 9, 2012, Print ISSN: 1934-3639, Online ISSN: 1934-3647).
All plasmid isolations were conducted according to manufacturer's instructions using HiSpeed Maxi Kit (Quiagen/Germany). For this purpose, *Escherichia coli* XL10 cells (Stratagene) containing the final expression plasmid or one of the interim plasmids were cultivated in Luria-Bertoni (LB) medium (Sigma-Aldrich) containing 50 mg/ml Ampicillin (Sigma-Aldrich).
For amplification of Glucosidase 2 gene (SEQ ID NO: 1), 50 µl PCR reaction contained 25 µl Pfu Mastermix (Stratagene) 22 µl H₂O, 1 µl of forward primer BE2_forw (Sto 378, SEQ ID NO: 3) and 1 µl of reverse primer BE2_rev (Sto380, SEQ ID NO: 4), 1 µl template (genomic DNA of *Schizophyllum commune* strain Lu15523). The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes. For amplification of the Tef terminator (SEQ ID NO: 5), 50 µl PCR reaction contained 25 µl Pfu Mastermix (Stratagene) 22 µl H₂O, 1 µl of forward primer (Sto 379, SEQ ID NO: 6) and 1 µl of reverse primer Sto229, SEQ ID NO: 7), 1 µl template (genomic DNA of *Schizophyllum commune* strain Lu15523). The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes.

In the next PCR reaction step, fusion of the two PCR products (*tef1* terminator (SEQ ID NO: 5) with Glucosidase 2 gene was carried out in a 50 µl PCR reaction containing 25 µl Pfu Mastermix, 21 µl of H₂O, 1 µl of each primer: Sto229, SEQ ID NO: 7) Sto 378 (SEQ ID NO: 3) and 1 µl of both templates. The reaction was carried out in Gene Amp^{®} PCR System 9700 Thermal Cycler from PE Applied Biosystems. The following program was used for the fusion of both sequences: an initial heating step up to 95 °C for 3 minutes was followed by 30 cycles of 30 seconds denaturing at 95 °C, 30 seconds of annealing step at 58 °C, 2.5 minutes elongation step at 72 °C, followed by one cycle at 72 °C for 10 minutes.

The product of the fusion PCR was treated with *EcoR*I and *Xho*I restriction enzymes (Roche) according to manufacturer's instructions and the vector (pBluescript 2KSP, Stratagene Cloning Systems, already carrying the Tef Promoter and a a plasmid selection marker was linearized using the same restriction enzymes and subsequently treated with alkaline phosphatase (Roche) according to manufacturer's instructions. Both, the digested PCR product and the linearized vector, were ligated using T4 DNA Ligase (New England Biolabs, Inc., Beverly, MA/USA) and transformed into *Escherichia coli* XL10 cells (Stratagene) according to manufacturer's instructions.

For the transformation of *Schizophyllum commune* with the Glucosidase 2 expression plasmid (pBE_2.1), plasmid preparation was carried out as follows. *Escherichia coli* XL10 cells containing the Glucosidase 2 expression plasmid were cultivated in Luria-Bertoni (LB) medium (Sigma-Aldrich) containing 50 mg/ml Ampicillin (Sigma-Aldrich) and the plasmid isolation was conducted according to manufacturer's instructions using HiSpeed Maxi Kit (Quiagen).

For preparation of *S. commune* protoplasts, fresh culture of the strain Lu 15527 was inoculated on a plate containing complex medium (CYM). For incubation at 26 °C for 2-3 days, plates were sealed with parafilm.

For inoculation of liquid preculture (50 ml working volume), the biomass from the plate was macerated for 1 minute at 13500 rpm using T 25 digital ULTRA-TURRAX^{®} (IKA), inoculated in shaking flask containing liquid CYM medium and incubated at 30 °C, 220 rpm for further 3 days. Main culture was inoculated with 15 ml of the preculture in 200 ml CYM medium and incubated further 3 days at 30 °C at 220 rpm. After finishing the culture growth, the main culture was divided in four 50 ml samples and centrifuged (4000 rpm, 15 min). Obtained pellet was washed twice with 1 M MgSO₄ (50ml) (Roth). After washing, four samples were united and dissolved 50 ml 1M MgSO₄.

To enable cell wall lysis, 100 mg Caylase (Cayla, Toulouse, France) were dissolved in 1 mL 1 M MgSO₄ and added to the pellet suspension. The sample was incubated over night at 30 °C under slight shaking (70 rpm). Subsequently distilled water was added to the sample (in 1:1 ratio), which was then incubated under slight shaking (70 rpm) for further 5 min. After this step, cells were incubated without shaking for 10 min and subsequently centrifuged (1100 rpm, 20 min, 4 °C). After the supernatant was filtrated using Miracloth-Membrane, one volume of cold 1 M sorbitol was added and the sample was allowed to equilibrate for 10 min. Subsequently, the sample was centrifuged (2000 rpm, 20 min, 2 °C). Pellet was washed by re-suspending carefully in 1 M sorbitol and centrifugation step was repeated. Finally the protoplasts were re-suspended in 1 M sorbitol and 50 mM CaCl2 at a concentration of 10⁸ protoplasts per ml.
DNA used for transformation was a circular plasmid and the integration in the genome of *S. commune* was ectopic. To transform the protoplasts with the DNA, 100 µl protoplasts and 10µl DNA (5-10 µg) were gently mixed and incubated for 60 min on ice. Subsequently, one volume of PEG 4000 (40 %) was added and the sample was incubated for 5 to 10 min on ice. After adding 2.5 ml regeneration medium (complete medium containing 0,1 µg/ml Phleomycin and 0.5 M MgSO₄), the sample was incubated at 30 °C, 70 rpm overnight.

After PEG mediated transformation, regenerated protoplasts were spread on petri dishes containing 40 ml solidified minimal medium: 2 g aspartic acid (Roth), 20 g glucose (Sigma), 0.5 g MgSO₄ (Roth), 0.5 g KH₂PO₄ , 1 g K₂HPO₄ (both from Riedel-de Haën), 120 µg thiaminhydrochlorid (Roth) per liter, pH 6,3 containing 1 % low melting agarose (Sigma). Selection plates were incubated 5 days at 30 °C.

### Example 1b (104)

### Cloning of the Glucosidase 2 expression plasmid (pBE-2.2) and transformation into S. commune

(pBE_2.2) (having pBluescript II as backbone) was generated carrying a bacterial selection marker cassette (*amp^{R}*), strong constitutive promoter (Tef1 promoter), the glucosidase 2 gene sequence (genomic seqence) and terminator sequence (Tef1 terminator) and the fungal selection marker *clonnat.*

The expression plasmid for Glucosidase 2 (SEQ ID NO:10) (pBE_2.2) was prepared based on pBE_2.1 from Example 1a.

The Ura marker and the Tef Promoter were cut out of the plasmid with EcoRI and SpeI, the linearized backbone was dephosphorylated with alkaline phosphatase.
As a source of the promoter sequence *tef1* (SEQ ID NO: 17); the same PCR product as in Example 1 was used.
For amplification of the Tef terminator (SEQ ID NO: 5), 50 µl PCR reaction contained 25 µl Pfu Mastermix (Stratagene) 22 µl H₂O, 1 µl of forward primer BE2_forw (Sto 658, SEQ ID NO: 12) and 1 µl of reverse primer BE2_rev (Sto659, SEQ ID NO: 13), 1 µl template (genomic DNA of *Schizophyllum commune* strain Lu 15523). The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes. The PCR fragment was cut wit EcoRI and SpeI and ligated with the linearized plasmid backbone , using Rapid Ligation Kit (Roche) and transformed into *Escherichia coli* XL10 cells (Stratagene) according to manufacturer's instructions.
The resulting plasmid served as an intermediate cloning construct. It was isolated from E.coli as described above and linearized with NotI and SpeI.
For amplification of the clonnat resistance cassette (SEQ ID NO: 14), 50 µl PCR reaction contained 25 µl Pwo Mastermix (Stratagene) 22 µl H₂O, 1 µl of forward primer (Sto 656, SEQ ID NO: 15) and 1 µl of reverse primer (Sto671, SEQ ID NO: 16), 1 µl template DNA. The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes. The resulting PCR fragment was cut with Noti and Spel and ligated with the linearized plasmid.
Transformation and isolation from E.coli were performed as described above.

For preparation of *S. commune* protoplasts, a fresh culture was inoculated on a plate containing complex medium (CYM). For incubation at 26 °C for 2-3 days, plates were sealed with parafilm.

For inoculation of liquid preculture (50 ml working volume), the biomass from the plate was macerated for 1 minute at 13500 rpm using T 25 digital ULTRA-TURRAX^{®} (IKA), inoculated in shaking flask containing liquid CYM medium and incubated at 30 °C, 220 rpm for further 3 days. Main culture was inoculated with 15 ml of the preculture in 200 ml CYM medium and incubated further 4 days at 30 °C at 220 rpm. After finishing the culture growth, the main culture was pre-treated with 2.5% Glucanase (Erbslöh Geisenheim AG) for 2h at 40 °C, then divided in four 50 ml samples and centrifuged (4000 rpm, 15 min). Obtained pellet was washed twice with 0.9M NaCl and resuspended in 0.9M NaCl (50ml).

To enable cell wall lysis, 100 mg Caylase (Cayla, Toulouse, France) was added to the pellet suspension. The sample was incubated over night at 30 °C under slight shaking (70 rpm) and then filtrated using Miracloth-Membrane. Subsequently, the sample was centrifuged (2500 rpm, 15 min). Pellet was washed by re-suspending carefully in 10 ml 0.9M NaCl, centrifuged again(2500 rpm, 15 min) and resuspended in 2ml 1M Sorbitol + 50 mM CaCl2.

DNA was transformed as a circular plasmid for ectopic integration in the genome of *S. commune.* To transform the protoplasts with the DNA, 200 µl protoplasts and 10µl DNA (5-20 µg) were gently mixed and incubated for 60 min on ice. Subsequently, one volume of PEG 4000 (40 %) was added and the sample was incubated for 5 to 10 min on ice. After adding 2.5 ml regeneration medium (complete medium containing 0,1 µg/ml Phleomycin and 0.8M sorbitol), the sample was incubated at 30 °C, 70 rpm overnight.

After PEG mediated transformation, regenerated protoplasts were spread on petri dishes containing 40 ml solidified minimal medium: 2 g aspartic acid (Roth), 20 g glucose (Sigma), 0.5 g MgSO₄ (Roth), 0.5 g KH₂PO₄ , 1 g K₂HPO₄ (both from Riedel-de Haën), 120 µg thiaminhydrochlorid (Roth) per liter, pH 6,3 containing 1 % low melting agarose (Sigma) for Uracil selection. For selction with Clonnat the following plates were used: 2g Tryptic Peptone, 2g Yeast Extract, 20 g glucose (Sigma), 0.5 g MgSO₄ (Roth), 0.5 g KH₂PO₄ , 1 g K₂HPO₄ (both from Riedel-de Haën), 100mg/ml Clonnat. Selection plates were incubated 5 days at 30 °C.

### Example 1 c)

### Cloning of the Glucosidase1 expression plasmid (pBE_1) and transformation into S. commune

In the genome of *Schizophyllum commune,* two genes encoding for a putative Glucosidase were identified by using BLAST analysis.

One expression plasmid (pBE_1) (having pBluescript II as backbone) was generated carrying a bacterial selection marker cassette (*amp^{R}*), strong constitutive promoter (Tef1 promoter), the Glucosidase 1 gene sequence (genomic sequence, SEQ ID NO: 17) and terminator sequence (Tef1 terminator) and the fungal selection marker *ura1.*

The individual elements (Tef1 promoter, Tef1 terminator and *ura1*) were isolated from the genomic DNA of *Schizophyllum commune* using PCR technology prepared by established microbiologic protocols (Sambrook, loc cit, Current Protocols in Molecular Biology, Update May 9, 2012, Print ISSN: 1934-3639, Online ISSN: 1934-3647).

The polynucleotides represented by SEQ ID NO 17 (genes Glucosidase 1 of Lu15523) were isolated from the genomic DNA of *Schizophyllum commune* strain Lu15523, using PCR technology prepared as described above.

All plasmid isolations were conducted according to manufacturer's instructions using HiSpeed Maxi Kit (Quiagen/Germany). For this purpose, *Escherichia coli* XL10 cells (Stratagene) containing the final expression plasmid or one of the interim plasmids were cultivated in Luria-Bertoni (LB) medium (Sigma-Aldrich) containing 50 mg/ml Ampicillin (Sigma-Aldrich).

For amplification of Glucosidase 1 gene 50 µl PCR reaction contained 25 µl Herculase Mastermix (Stratagene) 22 µl H₂O, 1 µl of forward primer (Sto 381, SEQ ID NO: 19) and 1 µl of reverse primer (Sto348, SEQ ID NO: 20), 1 µl template (genomic DNA of *Schizophyllum commune* strain Lu15523). The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes.

For amplification of the Tef terminator, 50 µl PCR reaction contained 25 µl Pfu Mastermix (Stratagene) 22 µl H₂O, 1 µl of forward primer (Sto 393, SEQ ID NO: 21) and 1 µl of reverse primer (Sto282, SEQ ID NO: 22), 1 µl template (genomic DNA of *Schizophyllum commune* strain Lu 15523). The reaction was carried out in Gene Amp^{®} PCR System 9700 Thermal Cycler from PE Applied Biosystems. The following program was used for the amplification: an initial heating step up to 95 °C for 3 minutes was followed by 30 cycles of 30 seconds denaturing at 95 °C, 30 seconds of annealing step at 58 °C, 2 minutes elongation step at 72 °C, followed by one cycle at 72 °C for 10 minutes.

In the next PCR reaction step, fusion of the two PCR products *tef1* terminator with Glucosidase1 gene was carried out in a 50 µl PCR reaction containing 25 µl Herculase Mastermix, 21 µl of H₂O, 1 µl of each primer: Sto381, (SEQ ID NO: 23), and Sto 382 (SEQ ID NO: 22) and 1 µl of both templates. The reaction was carried out in Gene Amp^{®} PCR System 9700 Thermal Cycler from PE Applied Biosystems. The following program was used for the fusion of both sequences: an initial heating step up to 95 °C for 3 minutes was followed by 30 cycles of 30 seconds denaturing at 95 °C, 30 seconds of annealing step at 58 °C, 2.5 minutes elongation step at 72 °C, followed by one cycle at 72 °C for 10 minutes.

The product of the fusion PCR was treated with *EcoR*I and *Kpn*I restriction enzymes (Roche) according to manufacturer's instructions and the vector (pBluescript 2KSP, Stratagene Cloning Systems, already carrying the Tef Promoter and a a plasmid selection marker was linearized using the same restriction enzymes and subsequently treated with alkaline phosphatase (Roche) according to manufacturer's instructions. Both, the digested PCR product and the linearized vector, were ligated using T4 DNA Ligase (New England Biolabs, Inc., Beverly, MA/USA) and transformed into *Escherichia coli* XL10 cells (Stratagene) according to manufacturer's instructions.

For preparation of *S. commune* protoplasts, fresh culture of the strain Lu 15527 was inoculated on a plate containing complex medium (CYM). For incubation at 26 °C for 2-3 days, plates were sealed with parafilm.

For inoculation of liquid preculture (50 ml working volume), the biomass from the plate was macerated for 1 minute at 13500 rpm using T 25 digital ULTRA-TURRAX^{®} (IKA), inoculated in shaking flask containing liquid CYM medium and incubated at 30 °C, 220 rpm for further 3 days. Main culture was inoculated with 15 ml of the preculture in 200 ml CYM medium and incubated further 3 days at 30 °C at 220 rpm. After finishing the culture growth, the main culture was divided in four 50 ml samples and centrifuged (4000 rpm, 15 min). Obtained pellet was washed twice with 1 M MgSO₄ (50ml) (Roth). After washing, four samples were united and dissolved 50 ml 1M MgSO₄.

To enable cell wall lysis, 100 mg Caylase (Cayla, Toulouse, France) were dissolved in 1 mL 1 M MgSO₄ and added to the pellet suspension. The sample was incubated over night at 30 °C under slight shaking (70 rpm). Subsequently distilled water was added to the sample (in 1:1 ratio), which was then incubated under slight shaking (70 rpm) for further 5 min. After this step, cells were incubated without shaking for 10 min and subsequently centrifuged (1100 rpm, 20 min, 4 °C). After the supernatant was filtrated using Miracloth-Membrane, one volume of cold 1 M sorbitol was added and the sample was allowed to equilibrate for 10 min. Subsequently, the sample was centrifuged (2000 rpm, 20 min, 2 °C). Pellet was washed by re-suspending carefully in 1 M sorbitol and centrifugation step was repeated. Finally the protoplasts were re-suspended in 1 M sorbitol and 50 mM CaCl2 at a concentration of 10⁸ protoplasts per ml.

DNA used for transformation was a circular plasmid (pGS_1) and the integration in the genome of *S. commune* was ectopic. To transform the protoplasts with the DNA, 100 µl protoplasts and 10µl DNA (5-10 µg) were gently mixed and incubated for 60 min on ice. Subsequently, one volume of PEG 4000 (40 %) was added and the sample was incubated for 5 to 10 min on ice. After adding 2.5 ml regeneration medium (complete medium containing 0,1 µg/ml Phleomycin and 0.5 M MgSO₄), the sample was incubated at 30 °C, 70 rpm overnight.

After PEG mediated transformation, regenerated protoplasts were spread on petri dishes containing 40 ml solidified minimal medium: 2 g aspartic acid (Roth), 20 g glucose (Sigma), 0.5 g MgSO₄ (Roth), 0.5 g KH₂PO₄ , 1 g K₂HPO₄ (both from Riedel-de Haën), 120 µg thiaminhydrochlorid (Roth) per liter, pH 6,3 containing 1 % low melting agarose (Sigma). Selection plates were incubated 5 days at 30 °C.

### Example 1 d)

### Verification of the functionality of the engineered S. commune strains

Genetically modified *S. commune* strains generated as described above were tested in shaking flasks for increased Schizophyllan production. To assure the reproducibility of the results, a three-step cultivation was applied, consisting of two pre-cultures and one main culture as further described herein below.

For the cultivation of the genetically modified *Schizophyllum commune* strains, two different media were used. For cultivation on solid media, CYM medium (25 g agar (Difco), 20 g glucose (Sigma), 2 g trypticase peptone (Roth), 2 g yeast extract (Difco), 0.5 g MgSO₄ x 7 H₂O (Roth), 0.5 g KH₂PO₄ and 1 g K₂HPO₄ (both from Riedel-de Haën) per liter H₂O) was used. Strains were inoculated on agar plates containing CYM medium covered with cellophane (to avoid mycelium growth into the agar) and incubated for three to four days at 26 °C.

For the liquid cultures, the following medium was used (hereinafter referred to as "Standard Medium"): 30 g glucose (Sigma), 3 g yeast extract (Difco), 1 g KH₂PO₄ (Riedel-de Haën), 0.5 g MgSO₄ x 7 H₂O (Roth) per liter H₂O.

For both pre-cultures and for main culture, 250 ml shaking flasks filled with 30 ml Standard Medium were used. The cultivation was carried out at 27 °C and 225 rpm. Before each inoculation, the biomass was homogenized for 1 minute at 13500 rpm using T 25 digital ULTRA-TURRAX^{®} (IKA).

The first pre-culture was inoculated with 50 mg of wet biomass. The cultures were incubated for 72 hours. After 72 hours, the second pre-culture was started. The concentration of the homogenized wet biomass from the first pre-culture used for inoculation was 250 mg. Cultivation time was 45 hours. After 45 hours, the main culture was inoculated with 500 mg of homogenized wet biomass from the second pre-culture and cultivated for another 45 hours.

After the cultivation was finished, standard analytical methods as described herein below were applied to define the biomass concentration, Schizophyllan concentration, ethanol concentration and residual glucose in medium. 50 ml aliquots of the cultures were stabilized with 3 g/l Acticide BW20 (Thor).

Ethanol and glucose concentration was estimated using HPLC method. For this purpose 14 ml of the culture were centrifuged (30 min, 8500 rpm). The supernatant was sterile-filtrated and 1 ml of the filtrate was injected for the HPLC analysis (HPLC cation exchanger: Aminex HPX-87-H, BIO-RAD with 0.5 M H₂SO₄, Roth, as eluent and 0.5 ml/min flow rate at 30 °C).

Due to the fact that Schizophyllan consists only of glucose molecules, the quantification of this polymer can be done using standard analytical methods for glucose. 10 ml of the culture, 20 ml H₂O and 90 µl Acticide BW20 were mixed. The sample was digested for 24 h at 40 °C with beta-glucanase (0.3 ml) (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄ (Roth) as eluent and 0.5 ml/min flow rate at 30°C.

For the biomass determination, the remaining biomass in form of pellet (after beta-glucanase digestion sample was centrifuged) was washed twice with 50 ml H₂O, filtrated using Whatman-Filter (with determination of filter's weight before filtration), washed twice with H₂O and dried in HB43S drying scale from Mettler Toledo. Drying of the filter was carried out for 5 to 10 minutes at 180 °C. Subsequently, weight of the dry filter was determined.

The evaluation of the results obtained in shaking flasks showed clear effect of the overexpression of Glucosidase 2 on the schizophyllan production. Because of the fact that in the expression plasmid was ectopically integrated into genome and the integration locus has an explicit effect on the expression of the target gene, 20 clones carrying the plasmid (pBE_2.1) , 20 clones carrying the plasmid (pBE_2.2) and 10 clones carrying the plasmid (pBE_1) were tested in shaking flask experiments. The increase of schizophyllan production in the genetically modified strains is shown in Table 1 in comparison to the non-modified *Schizophyllum commune* control strain. The results shown in the Table 1 refer to the best strain tested. For classification of the strains, the amount of schizophyllan in the sample was decisive. 10 ml of the culture, 20 ml H₂O and 90 µl Acticide BW20 were mixed. The sample was digested for 24 h at 40 °C with 0.3 ml beta-glucanase (Erbslöh). After the incubation, the sample was centrifuged (30 minutes at 3400 g) and the supernatant was analyzed for glucose and ethanol content using HPLC cation exchanger (Aminex HPX-87-H, BIO-RAD) with 0.5 M H₂SO₄ (Roth) as eluent and 0.5 ml/min flow rate at 30 °C.

**Table 1: Comparison of Schizophyllum commune control strain with two genetically modified S. commune strains carrying Glucosidase expression plasmids (pGS_2.1) or (pGS_2.2).**

| | Schizophyllan (%) | EtOH (%) |
|---|---|---|
| S. commune control strain | 100 | 100 |
| S. commune pBE2.1 | 463 | 52 |
| S. commune control strain | 100 | 100 |
| S. commune pBE2.2 | 118 | 27 |

In addition to increased yields of schizophyllan production in the genetically modified S. *commune* strains, a clear decrease in the synthesis of the by-product ethanol was observed. This can be an indication that the excess rate of glucose by up-regulated Glucosidase activity is metabolized more directly in the schizophyllan pathway instead of partly being used for ethanol synthesis.

For the analysis of the effect of Glucosidase1 overexpression, 10 clones carrying the plasmid (pBE_1.1) were tested in shaking flask experiments, because of the fact that in the expression plasmid was ectopically integrated into genome and the integration locus has an explicit effect on the expression of the target gene. The experiment was performed as described above.

The evaluation of the results obtained in shaking flasks showed no effect of the overexpression of Glucosidase 1 on the schizophyllan production.

### Structure and conformation analysis of the product

To assure that the polymer synthesized through genetically modified *S. commune* strains is schizophyllan, XRD and NMR methods were applied to confirm the structure of the molecule as follows.

Powder X-ray diffraction (XRD) allows rapid, non-destructive analysis of materials consisting of multiple components. Moreover, the sample preparation is straightforward. The data from the measurement is presented as a diffractogram in which the diffracted intensity (I) is shown as a function of scattering angle 2θ. The crystallinity of the given material can be determined by this measurement. In general, crystalline materials have reflection patterns of a series of sharp peaks whereas amorphous materials give broad signals. Many polymers exhibit semicrystalline behaviour which can also be detected by XRD (Hammond, The basics of chrystallography and diffraction, 3rd Ed., Oxford University Press 2009).

### Sample preparation from aqueous solution

Aqueous solution containing schizophyllan was poured in ethanol to precipitate schizophyllan. The precipitation was filtered and dried in a vacuum oven. The dried sample was measured by XRD.

### Sample measurement and results by XRD

Schizophyllan exhibits a triple helical structure. This was evident from the diffractogram of the precipitated and dried schizophyllan sample (Figure 2). The triple helix could be seen as an intensive diffraction at 5 ° 2θ and the amorphous region of the material gives broad diffraction in the range of 20-25 ° 2θ (Hisamatsu, Carbohydr Res (1997), 298: 117).

### Sample measurement and results by NMR

The NMR spectra were recorded on a Varian VNMRS 600 MHz system equipped with a ¹³C-enhanced cryo-probe (inverse configuration) at ambient temperatures or at 50 °C using standard pulse sequences for ¹H and ¹³C.

It is known that schizophyllan has a triple helical structure formed by three β(1-3)-D-glucan chains held together by hydrogen bonds in water. This structure is shielded in the magnetic field due to the rigid, ordered conformation. This means that in NMR spectrum chemical shifts for schizophyllan are not obtained (Rinaudo, Carbohydr Polym (1982), 2: 135; Vlachou, Carbohydr Polym (2001), 46: 349) (2D NMR). In order to investigate the molecular structure of schizophyllan and not the macromolecular structure consisting of triple helices and further to record the successful NMR spectra with a good signal-to-noise ratio, the conformation of the triple helix has to be changed. It is also known that the triple helix of schizophyllan can be altered to form a random coil structure by addition of DMSO. When the DMSO concentration exceeds a certain threshold values (i.e. 87%), the conformation change takes place; therefore deuterated [D₆]-DMSO was used as a solvent for the measurements. This conformation matter is important to take into consideration when conducting NMR experiments for schizophyllan. Hence, the sample was measured in [D₆]-DMSO, the well-resolved spectra can be obtained (Figure X).

The chemical structures of the materials from *S. commune* (pBE2.1) and *S. commune* (pBE2_2) strain was identified to be the correct for that of schizophyllan. In addition, the materials exhibit the triple helix conformations.

XRD data of the material from *S. commune* (pBE1.1) shows that this sample does not contain the characteristic triple helix of Schizophyllan.

### Example 2

### Cloning of additional Glucosidase2 variants, transformation into S. commune and analysis of the effect on Schizophyllan production upon overexpression.

### Of the Glucosidase 2 gene, additional gene variants with amino acid exchanges were generated with the use of the Quickchange Site-Directed Mutagenesis Kit (Stratagene) This resulted in plasmids pBE2_3 and pBE2_4. The Glucosidase gene sequence of pBE2_3 ((SEQ ID NO: 25) is derived from the genomic sequence of strain Lu 15634, the Glucosidase gene sequence of pBE2_4 contains an additional Asparagin as second amino acid ((SEQ ID NO: 27).

These plasmids are going to be transformed into S. commune strains and the resulting transformants will be tested in shaking flask cultures regarding their Schizophyllan productivity.

### Example 3

### Cloning of a Phosphoglucomutase gene, transformation into S. commune and analysis of the effect on Schizophyllan production upon overexpression.

In the genome of *Schizophyllum commune,* a gene encoding a putative Phosphoglucomutase was identified by using BLAST analysis. In context of the present invention, it was proven that the overexpression in *S. commune* results in increased yields of Schizophyllan.

An expression plasmid (pPGM_1) (having pBluescript II as backbone) was generated carrying a bacterial selection marker cassette (*amp^{R}*), strong constitutive promoter (GFD (glycerol-3-phosphate dehydrogenase) promoter), the Phosphoglucomutase gene sequence and terminator sequence (Tef1 terminator) and the fungal selection marker *clonnat.*

All polynucleotide sequences described herein originate from *Schizopyllum commune,* isolated using PCR technology prepared by established microbiologic protocols (Sambrook, loc cit, Current Protocols in Molecular Biology, Update May 9, 2012, Print ISSN: 1934-3639, Online ISSN: 1934-3647).
All plasmid isolations were conducted according to manufacturer's instructions using HiSpeed Maxi Kit (Quiagen/Germany). For this purpose, *Escherichia coli* XL10 cells (Stratagene) containing the final expression plasmid or one of the interim plasmids were cultivated in Luria-Bertoni (LB) medium (Sigma-Aldrich) containing 50 mg/ml Ampicillin (Sigma-Aldrich).

For amplification of the Phosphoglucomutase gene sequence (SEQ ID NO: 28), 50 µl PCR reaction contained 25 µl PWO Mastermix (Roche), 22 µl H₂O, 1 µl of forward primer PGM_forw (Sto 687, SEQ ID NO: 30) and 1 µl of reverse primer PGM_rev (Sto688, SEQ ID NO: 31), 1 µl template (genomic DNA of *Schizophyllum commune* strain Lu 15634). The reaction was carried out in Gene Amp^{®} PCR System 9700 Thermal Cycler from PE Applied Biosystems. The following program was used for the amplification: an initial heating step up to 95 °C for 5 minutes was followed by 30 cycles of 30 seconds denaturing at 95 °C, 30 seconds of annealing step at 54 °C, 2 minutes elongation step at 72 °C, followed by one cycle at 72 °C for 5 minutes.
The genomic sequence of PGM was cloned into the final expression plasmid pPGM_1 analogous to the cloning of the expression plasmid pBE_2.2, as described in example 1b)

### Verification of the functionality of the engineered S. commune strains

Transformation of the plasmid pPGM_1 into strain Lu15523 was performed as described above (Example 1b). Genetically modified *S. commune* strains were tested in shaking flasks for increased Schizophyllan production, as described (see Example 1 d).

The evaluation of the results obtained in shaking flasks showed clear effect of the overexpression of PGM on Schizophyllan production. Because of the fact that in the expression plasmid was ectopically integrated into genome and the integration locus has an explicit effect on the expression of the target gene, a minimum of 20 clones was tested in shaking flask experiments. The increase of schizophyllan productionof the best modified strain tested is shown in comparison to the non-modified *Schizophyllum commune* control strain.

**Table 2: Comparison of Schizophyllum commune control strain with a genetically modified S. commune strains carrying a Phosphoglucomutase expression plasmid.**

| | Schizophyllan (%) | EtOH (%) |
|---|---|---|
| S. commune control strain | 100 | 100 |
| S. commune pPGM | 188 | 129 |

### Example 4

### Cloning of UDP-glucose-1-phosphate uridylyltransferase gene variants, transformation into S. commune and analysis of the effect on Schizophyllan production upon overexpression.

In the genome of *Schizophyllum commune,* three genes encoding a putative UDP-glucose-1-phosphate uridylyltransferase (*utp1, utp2* and *utp3*) were identified by using BLAST analysis. In context of the present invention, it was proven that the overexpression in *S. commune* results in increased yields of Schizophyllan.

Expression plasmids (pUTP_1, pUTP_2, pUTP_3) (having pBluescript II as backbone) were generated carrying a bacterial selection marker cassette (*amp^{R}*), strong constitutive promoter (GFD (glycerol-3-phosphate dehydrogenase) promoter), the respective *utp* gene sequence and terminator sequence (Tef1 terminator) and the fungal selection marker *clonnat.*

All polynucleotide sequences described herein originate from *Schizopyllum commune,* isolated using PCR technology prepared by established microbiologic protocols (Sambrook, loc cit, Current Protocols in Molecular Biology, Update May 9, 2012, Print ISSN: 1934-3639, Online ISSN: 1934-3647).

All plasmid isolations were conducted according to manufacturer's instructions using HiSpeed Maxi Kit (Quiagen/Germany). For this purpose, *Escherichia coli* XL10 cells (Stratagene) containing the final expression plasmid or one of the interim plasmids were cultivated in Luria-Bertoni (LB) medium (Sigma-Aldrich) containing 50 mg/ml Ampicillin (Sigma-Aldrich).

For amplification of the UTP1 gene sequence (SEQ ID NO: 32), 50 µl PCR reaction contained 25 µl PWO Mastermix (Roche), 22 µl H₂O, 1 µl of forward primer (Sto 126, SEQ ID NO: 34) and 1 µl of reverse primer (Sto334, SEQ ID NO: 35), 1 µl template (genomic DNA of *Schizophyllum commune* strain Lu15634). The reaction was carried out in Gene Amp^{®} PCR System 9700 Thermal Cycler from PE Applied Biosystems. The following program was used for the amplification: an initial heating step up to 95 °C for 5 minutes was followed by 30 cycles of 30 seconds denaturing at 95 °C, 30 seconds of annealing step at 47 °C, 2 minutes elongation step at 72 °C, followed by one cycle at 72 °C for 10 minutes. The genomic sequence of UTP1 was cloned into the final expression plasmid pUTP_1 analogous to the cloning of the expression plasmid pBE_2.2, as described in example 1b).

For amplification of the UTP2 gene sequence (SEQ ID NO: 36), 50 µl PCR reaction contained 25 µl PWO Mastermix (Roche), 22 µl H₂O, 1 µl of forward primer (Sto 124, SEQ ID NO: 38) and 1 µl of reverse primer (Sto201, SEQ ID NO: 39), 1 µl template (genomic DNA of *Schizophyllum commune* strain Lu15634). The reaction was carried out in Gene Amp^{®} PCR System 9700 Thermal Cycler from PE Applied Biosystems. The following program was used for the amplification: an initial heating step up to 95 °C for 5 minutes was followed by 30 cycles of 30 seconds denaturing at 95 °C, 30 seconds of annealing step at 65 °C, 2 minutes elongation step at 72 °C, followed by one cycle at 72 °C for 5 minutes. The genomic sequence of UTP2 was cloned into the final expression plasmid pUTP_2 analogous to the cloning of the expression plasmid pBE_2.2, as described in example 1b).

For amplification of the UTP3 gene sequence (SEQ ID NO: 40), 50 µl PCR reaction contained 25 µl PWO Mastermix (Roche), 22 µl H₂O, 1 µl of forward primer (Sto 781, SEQ ID NO: 42) and 1 µl of reverse primer (Sto782, SEQ ID NO: 43), 1 µl template (genomic DNA of *Schizophyllum commune* strain Lu15634). The reaction was carried out in Gene Amp^{®} PCR System 9700 Thermal Cycler from PE Applied Biosystems. The following program was used for the amplification: an initial heating step up to 95 °C for 5 minutes was followed by 30 cycles of 30 seconds denaturing at 95 °C, 30 seconds of annealing step at 65 °C, 2 minutes elongation step at 72 °C, followed by one cycle at 72 °C for 5 minutes. The genomic sequence of UTP3 was cloned into the final expression plasmid pUTP_3 analogous to the cloning of the expression plasmid pBE_2.2, as described in example 1b).

Of the UTP3 gene, additional gene variants with amino acid exchanges were generated with the use of the Quickchange Site-Directed Mutagenesis Kit (Stratagene)
This resulted in plasmids pUTP_3.2 (UTP3 (D2G, E313K)), pUTP_3.3 UTP3 (D2G, E313K, D212K) and pUTP_3.4 (UTP3 (D2G, E313K, D212K, E179K).

### Verification of the functionality of the engineered S. commune strains

Transformation of the plasmids pUTP_1, pUTP_2, pUTP_3.1, pUTP_3.2, pUTP_3.3 and pUTP_3.4 into strain Lu 15523 was performed as described above (Example 1b). Genetically modified *S. commune* strains were tested in shaking flasks for increased Schizophyllan production, as described (see Example 1 d).
The evaluation of the results obtained in shaking flasks showed clear effect of the overexpression of different UTP gene variants on Schizophyllan production. Because of the fact that in the expression plasmid was ectopically integrated into genome and the integration locus has an explicit effect on the expression of the target gene, several clones were tested in shaking flask experiments. The increase of schizophyllan production of the best modified strains tested is shown in comparison to the non-modified *Schizophyllum commune* control strain.

**Table 3: Comparison of Schizophyllum commune control strain with genetically modified S. commune strains carrying a UDP-glucose-1-phosphate uridylyltransferase expression plasmid.**

| | Schizophyllan (%) |
|---|---|
| S. commune control strain | 100 |
| S. commune pUTP_3.1 | 118 |
| S. commune pUTP_3.2 | 133 |

## Claims

1. Process for producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, said process comprising the steps of:
(i) culturing in a medium a genetically modified microorganism capable of producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, wherein the genetically modified microorganism - compared to a non-modified control microorganism of the same strain - has increased activity of at least two gene products selected from the group consisting of
(A) phosphoglucomutase with a SEQ ID NO:29 or a polypeptide sequence which is at least 95% identical to SEQ ID NO:29 and which have at least 80% of the enzymatic activity of the polypeptide according to SEQ ID NO:29,
(B) UTP-glucose-1-phosphate uridyltrasferase with a SEQ ID NO:41 or a polypeptide sequence which is at least 95% identical to SEQ ID NO:41 and which have at least 80% of the enzymatic activity of the polypeptide according to SEQ ID NO:41,
(C) branching enzyme with a SEQ ID NO:2 or a polypeptide sequence which is at least 95% identical to SEQ ID NO:2 and which have at least 80% of the enzymatic activity of the polypeptide according to SEQ ID NO:2;
under conditions allowing said microorganism to produce said polymer; wherein an increased activity of the respective gene product is achieved by introducing into the microorganism genes in multiple copies for the respective gene product,
(ii) optionally recovering said polymer from the medium, wherein said microorganism is Schizophyllum and wherein said polymer is schizophyllan.

2. Genetically modified microorganism capable of producing a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3, wherein the genetically modified microorganism - compared to a non-modified control microorganism of the same strain - has increased activity of at least two gene products selected from the group consisting of
(A) phosphoglucomutase with a SEQ ID NO:29 or a polypeptide sequence which is at least 95% identical to SEQ ID NO:29 and which have at least 80% of the enzymatic activity of the polypeptide according to SEQ ID NO:29,
(B) UTP-glucose-1-phosphate uridyltrasferase with a SEQ ID NO:41 or a polypeptide sequence which is at least 95% identical to SEQ ID NO:41 and which have at least 80% of the enzymatic activity of the polypeptide according to SEQ ID NO:41,
(C) branching enzyme with a SEQ ID NO:2 or a polypeptide sequence which is at least 95% identical to SEQ ID NO:2 and which have at least 80% of the enzymatic activity of the polypeptide according to SEQ ID NO:2;
wherein an increased activity of the respective gene product is achieved by introducing into the microorganism genes in multiple copies for the respective gene product, under conditions allowing said microorganism to produce said polymer;
wherein said microorganism is Schizophyllum and wherein said polymer is schizophyllan.

## Patentansprüche

1. Verfahren zur Herstellung eines Polymers, das aus einer geradlinigen Hauptkette von Beta-D-(1-3)-Glucopyranosyl-Einheiten mit einer einzelnen Beta-D-Glucopyranosyl-Einheit, die mit einer Beta-D-Glucopyranosyl-Einheit der geradlinigen Hauptkette (1-6)-verknüpft ist, bei einem durchschnittlichen Verzweigungsgrad von etwa 0,3, besteht, wobei das Verfahren die folgenden Schritte umfasst:
(i) Kultivieren eines gentechnisch veränderten Mikroorganismus mit der Fähigkeit zur Herstellung eines Polymers, das aus einer geradlinigen Hauptkette von Beta-D-(1-3)-Glucopyranosyl-Einheiten mit einer einzelnen Beta-D-Glucopyranosyl-Einheit, die mit einer Beta-D-Glucopyranosyl-Einheit der geradlinigen Hauptkette (1-6)-verknüpft ist, bei einem durchschnittlichen Verzweigungsgrad von etwa 0,3, besteht, in einem Medium, wobei der gentechnisch veränderte Mikroorganismus - verglichen mit einem nicht veränderten Kontrollmikroorganismus des gleichen Stamms - eine erhöhte Aktivität von wenigstens zwei Genprodukten aufweist, die ausgewählt sind aus der Gruppe bestehend aus
(A) Phosphoglucomutase mit einer SEQ ID NO:29 oder einer Polypeptidsequenz, die zu wenigstens 95% mit SEQ ID NO:29 identisch ist und die wenigstens 80% der enzymatischen Aktivität des Polypeptids gemäß SEQ ID NO:29 aufweist,
(B) UTP-Glucose-1-phosphat-Uridyltransferase mit einer SEQ ID NO:41 oder einer Polypeptidsequenz, die zu wenigstens 95% mit SEQ ID NO:41 identisch ist und die wenigstens 80% der enzymatischen Aktivität des Polypeptids gemäß SEQ ID NO:41 aufweist,
(C) Verzweigungsenzym mit einer SEQ ID NO:2 oder einer Polypeptidsequenz, die zu wenigstens 95% mit SEQ ID NO:2 identisch ist und die wenigstens 80% der enzymatischen Aktivität des Polypeptids gemäß SEQ ID NO:2 aufweist;
unter Bedingungen, die dem Mikroorganismus gestatten, das Polymer zu produzieren;
wobei eine erhöhte Aktivität des jeweiligen Genprodukts durch Einführen von mehreren Kopien von Genen für das jeweilige Genprodukt in den Mikroorganismus erreicht wird,
(ii) gegebenenfalls Gewinnen des Polymers aus dem Medium,
wobei es sich bei dem Mikroorganismus um Schizophyllum handelt und wobei es sich bei dem Polymer um Schizophyllan handelt.

2. Gentechnisch veränderter Mikroorganismus mit der Fähigkeit zur Herstellung eines Polymers, das aus einer geradlinigen Hauptkette von Beta-D-(1-3)-Glucopyranosyl-Einheiten mit einer einzelnen Beta-D-Glucopyranosyl-Einheit, die mit einer Beta-D-Glucopyranosyl-Einheit der geradlinigen Hauptkette (1-6)-verknüpft ist, bei einem durchschnittlichen Verzweigungsgrad von etwa 0,3, besteht, in einem Medium, wobei der gentechnisch veränderte Mikroorganismus - verglichen mit einem nicht veränderten Kontrollmikroorganismus des gleichen Stamms - eine erhöhte Aktivität von wenigstens zwei Genprodukten aufweist, die ausgewählt sind aus der Gruppe bestehend aus
(A) Phosphoglucomutase mit einer SEQ ID NO:29 oder einer Polypeptidsequenz, die zu wenigstens 95% mit SEQ ID NO:29 identisch ist und die wenigstens 80% der enzymatischen Aktivität des Polypeptids gemäß SEQ ID NO:29 aufweist,
(B) UTP-Glucose-1-phosphat-Uridyltransferase mit einer SEQ ID NO:41 oder einer Polypeptidsequenz, die zu wenigstens 95% mit SEQ ID NO:41 identisch ist und die wenigstens 80% der enzymatischen Aktivität des Polypeptids gemäß SEQ ID NO:41 aufweist,
(C) Verzweigungsenzym mit einer SEQ ID NO:2 oder einer Polypeptidsequenz, die zu wenigstens 95% mit SEQ ID NO:2 identisch ist und die wenigstens 80% der enzymatischen Aktivität des Polypeptids gemäß SEQ ID NO:2 aufweist;
wobei eine erhöhte Aktivität des jeweiligen Genprodukts durch Einführen mehrerer Kopien von Genen für das jeweilige Genprodukt in den Mikroorganismus erreicht wird,
unter Bedingungen, die dem Mikroorganismus gestatten, das Polymer zu produzieren;
wobei es sich bei dem Mikroorganismus um Schizophyllum handelt und wobei es sich bei dem Polymer um Schizophyllan handelt.

## Revendications

1. Processus destiné à produire un polymère constitué par une chaîne principale linéaire d'unités de bêta-D-(1,3)-glucopyranosyle ayant une seule unité de bêta-D-glucopyranosyle liée en (1,6) à une unité de bêta-D-glucopyranosyle de la chaîne principale linéaire avec un degré moyen de ramification de 0,3 environ, ledit processus comprenant les étapes de :
(i) culture dans un milieu d'un microorganisme génétiquement modifié capable de produire un polymère constitué par une chaîne principale linéaire d'unités de bêta-D-(1,3)-glucopyranosyle ayant une seule unité de bêta-D-glucopyranosyle liée en (1,6) à une unité de bêta-D-glucopyranosyle de la chaîne principale linéaire avec un degré moyen de ramification de 0,3 environ, dans lequel le microorganisme génétiquement modifié - en comparaison avec un microorganisme témoin non modifié de la même souche - a une activité accrue d'au moins deux produits géniques choisis dans le groupe constitué par
(A) une phosphoglucomutase avec une SEQ ID n° : 29 ou une séquence polypeptidique qui est identique au moins à 95% à la SEQ ID n° : 29 et qui a au moins 80% de l'activité enzymatique du polypeptide selon la SEQ ID n° : 29,
(B) une UTP-glucose-1-phosphate uridyltransférase avec une SEQ ID n° : 41 ou une séquence polypeptidique qui est identique au moins à 95% à la SEQ ID n° : 41 et qui a au moins 80% de l'activité enzymatique du polypeptide selon la SEQ ID n° : 41,
(C) une enzyme de ramification avec une SEQ ID n° : 2 ou une séquence polypeptidique qui est identique au moins à 95% à la SEQ ID n° : 2 et qui a au moins 80% de l'activité enzymatique du polypeptide selon la SEQ ID n° : 2 ;
dans des conditions permettant au dit microorganisme de produire ledit polymère ;
dans lequel une activité accrue du produit génique respectif est obtenue par une introduction dans le microorganisme de gènes en copies multiples pour le produit génique respectif,
(ii) en option, récupérer ledit polymère à partir du milieu,
dans lequel ledit microorganisme est Schizophyllum et dans lequel ledit polymère est le schizophyllane.

2. Microorganisme génétiquement modifié capable de produire un polymère constitué par une chaîne principale linéaire d'unités de bêta-D-(1,3)-glucopyranosyle ayant une seule unité de bêta-D-glucopyranosyle liée en (1,6) à une unité de bêta-D-glucopyranosyle de la chaîne principale linéaire avec un degré moyen de ramification de 0,3 environ, le microorganisme génétiquement modifié - en comparaison avec un microorganisme témoin non modifié de la même souche - ayant une activité accrue d'au moins deux produits géniques choisis dans le groupe constitué par
(A) une phosphoglucomutase avec une SEQ ID n° : 29 ou une séquence polypeptidique qui est identique au moins à 95% à la SEQ ID n° : 29 et qui a au moins 80% de l'activité enzymatique du polypeptide selon la SEQ ID n° : 29,
(B) une UTP-glucose-1-phosphate uridyltransférase avec une SEQ ID n° : 41 ou une séquence polypeptidique qui est identique au moins à 95% à la SEQ ID n° : 41 et qui a au moins 80% de l'activité enzymatique du polypeptide selon la SEQ ID n° : 41,
(C) une enzyme de ramification avec une SEQ ID n° : 2 ou une séquence polypeptidique qui est identique au moins à 95% à la SEQ ID n° : 2 et qui a au moins 80% de l'activité enzymatique du polypeptide selon la SEQ ID n° : 2 ;
dans lequel une activité accrue du produit génique respectif est obtenue par une introduction dans le microorganisme de gènes en copies multiples pour le produit génique respectif,
dans des conditions permettant au dit microorganisme de produire ledit polymère ;
dans lequel ledit microorganisme est Schizophyllum et dans lequel ledit polymère est le schizophyllane.
